# EUROPEAN PATENT SPECIFICATION

(11) **EP 3 807 273 B1**
(45) Date of publication and mention of the grant of the patent: **03.08.2022**
(21) Application number: 19735488.9
(22) Date of filing: 11.06.2019
(51) Int. Cl.: C07D 471/04, C07D 487/04, A61P 11/06, A61K 31/519

(54) **AZAINDOLE DERIVATIVES AS RHO-KINASE INHIBITORS**
AZAINDOL-DERIVATE ALS RHO-KINASE-HEMMER
DÉRIVÉS D'AZAINDOLE COMME INHIBITEURS DE LA KINASE RHO

(30) Priority: 13.06.2018 EP 18177556
(43) Date of publication of application: 21.04.2021
(73) Proprietor: Chiesi Farmaceutici S.p.A., 43122 Parma (IT)
(72) Inventor: ACCETTA, Alessandro, 43122 PARMA (IT); RANCATI, Fabio, 43122 PARMA (IT); CLARK, David Edward, 43122 PARMA (IT); EDWARDS, Christine, 43122 PARMA (IT)
(74) Representative: Bianchetti & Minoja SRL
(86) International application number: PCT/EP2019/065119
(87) International publication number: WO 2019/238628

(56) References cited:
- US-A- 5 795 977
- US-A1- 2008 139 595
- US-A1- 2018 215 758
- DEFERT; BOLAND: "Rho kinase inhibitors: a patent review (2014 - 2016)", EXPERT OPIN. THER. PATENTS, vol. 27, no. 4, 16 January 2017 (2017-01-16), pages 507-515, XP055400492, ISSN: 1354-3776, DOI: 10.1080/13543776.2017.1272579

## Description

### FIELD OF THE INVENTION

The present invention relates to compounds inhibiting Rho Kinase (hereinafter ROCK Inhibitors); particularly the invention relates to compounds that are azaindole derivatives, specifically substituted aryloxy derivatives of 7-azaindole and 5,7-diazaindole, methods of preparing such compounds, pharmaceutical compositions containing them and therapeutic use thereof.

The compounds of the invention are inhibitors of the activity or function of the ROCK-I and/or ROCK-II isoforms of the Rho-associated coiled-coil forming protein kinase (ROCK).

Therefore, the compounds of the invention may be useful in the treatment of many disorders associated with ROCK enzymes mechanisms, such as pulmonary diseases including asthma, chronic obstructive pulmonary disease (COPD), idiopathic pulmonary fibrosis (IPF) and pulmonary arterial hypertension (PAH).

### BACKGROUND OF THE INVENTION

Rho-associated coiled-coil forming protein kinase (ROCK) belongs to the AGC (PKA/PKG/PKC) family of serine-threonine kinases. Two human isoforms of ROCK have been described, ROCK-I (also referred to as p160 ROCK or ROKβ) and ROCK-II (ROKα) are approximately 160 kDa proteins containing an N-terminal Ser/Thr kinase domain, followed by a coiled-coil structure, a pleckstrin homology domain, and a cysteine-rich region at the C-terminus (Riento, K.; Ridley, A. J. Rocks: multifunctional kinases in cell behaviour. Nat. Rev. Mol. Cell Biol. 2003, 4, 446-456).

Both ROCK-II and ROCK-I are expressed in many human and rodent tissues including the heart, pancreas, lung, liver, skeletal muscle, kidney and brain (Riento and Ridley, 2003). In patients with pulmonary hypertension, ROCK activity is significantly higher in both lung tissues and circulating neutrophils as compared with controls (Duong-Quy S, Bei Y, Liu Z, Dinh-Xuan AT. Role of Rho-kinase and its inhibitors in pulmonary hypertension. Pharmacol Ther. 2013;137(3):352-64). A significant correlation was established between neutrophil ROCK activity and the severity and duration of pulmonary hypertension (Duong-Quy et al., 2013).

There is now substantial evidence that ROCK is involved in many of the pathways that contribute to the pathologies associated with several acute and chronic pulmonary diseases, including asthma, COPD, bronchiectasis and ARDS/ALI. Given the biological effect of ROCK, selective inhibitors have the potential to treat a number of pathological mechanisms in respiratory diseases, such as smooth muscle hyper-reactivity, bronchoconstriction, airway inflammation and airway remodeling, neuromodulation and exacerbations due to respiratory tract viral infection (Fernandes LB, Henry PJ, Goldie RG. Rho kinase as a therapeutic target in the treatment of asthma and chronic obstructive pulmonary disease. Ther Adv Respir Dis. 2007 Oct;1(1):25-33). Indeed the Rho kinase inhibitor Y-27632 causes bronchodilatation and reduces pulmonary eosinophilia trafficking and airways hyperresponsiveness (Gosens, R.; Schaafsma, D.; Nelemans, S. A.; Halayko, A. J. Rhokinase as a drug target for the treatment of airway hyperresponsiveness in asthma. Mini-Rev. Med. Chem. 2006, 6, 339-348). Pulmonary ROCK activation has been demonstrated in humans with idiopathic pulmonary fibrosis (IPF) and in animal models of this disease. ROCK inhibitors can prevent fibrosis in these models, and more importantly, induce the regression of already established fibrosis, thus indicating ROCK inhibitors as potential powerful pharmacological agents to halt progression of pulmonary fibrosis (Jiang, C.; Huang, H.; Liu, J.; Wang, Y.; Lu, Z.; Xu, Z. Fasudil, a rho-kinase inhibitor, attenuates bleomycin-induced pulmonary fibrosis in mice. Int. J. Mol. Sci. 2012, 13, 8293-8307).

Various compounds have been described in the literature as Rho Kinase Inhibitors. See e.g. WO2004/039796; WO2006/009889; WO2010/032875; WO2009/079008; WO2014/118133. US2008/139595A1 discloses Azaindole derivatives as Rho kinase inhibitors from which the present invention differs at least for the substituents to the azaindole.

There remains a potential for developing novel and pharmacologically improved ROCK inhibitors in many therapeutic areas such as: cardiovascular and respiratory diseases, erectile dysfunction, fibrotic diseases, insulin resistance, kidney failure, central nervous system disorders, auto-immune diseases and oncology.

In view of the number of pathological responses which are mediated by ROCK enzymes, there is a continuing need for inhibitors of such enzymes which can be useful in the treatment of many disorders. The present invention relates to novel compounds which are inhibitors of ROCK-I and ROCK-II isoforms of the Rho-associated coiled-coil forming protein kinase (ROCK), as demonstrated by the pharmacological activity data reported. Furthermore the compounds of the invention have therapeutically desirable characteristics, that makes them particularly suitable to be administered also by inhalation for the treatment of respiratory disease. The compounds of the invention are particularly promising for some pulmonary diseases including asthma, chronic obstructive pulmonary disease (COPD), idiopathic pulmonary fibrosis (IPF) and pulmonary hypertension (PH) and specifically pulmonary arterial hypertension (PAH).

### SUMMARY OF THE INVENTION

The present invention is directed to compounds of formula (I) 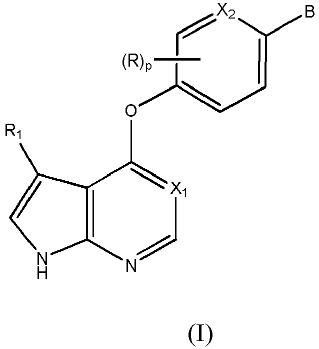 wherein X₁, X₂, R, R₁, B and p are as reported below in the detailed description of the invention, acting as ROCK inhibitors, to processes for the preparation thereof, pharmaceutical compositions comprising them either alone or in combination with one or more active ingredient, in admixture with one or more pharmaceutically acceptable carrier.

In one aspect the present invention provides the use of a compound of the invention for the manufacture of a medicament.

In a further aspect the present invention provides the use of a compound of the invention for the preparation of a medicament for the treatment of any disease characterized by ROCK enzyme aberrant activity and/or wherein an inhibition of activity is desirable and in particular through the selective inhibition of the ROCK enzyme isoforms over other Kinases.

Moreover the present invention provides a method for prevention and/or treatment of any disease wherein a ROCK enzyme inhibition is desirable, said method comprises administering to a patient in need of such treatment a therapeutically effective amount of a compound of the invention.

In particular the compounds of the invention alone or combined with other active ingredients may be administered for the prevention and/or treatment of a pulmonary disease including asthma, chronic obstructive pulmonary disease (COPD), idiopathic pulmonary fibrosis (IPF) and pulmonary hypertension (PH) and specifically pulmonary arterial hypertension (PAH).

### DETAILED DESCRIPTION OF THE INVENTION

The invention is directed to a class of compounds acting as inhibitors of the Rho Kinase (ROCK).

Said class of compounds inhibits the activity or function of the ROCK enzyme and more specifically, they are inhibitors of ROCK-I and ROCK-II isoforms of the Rho-associated coiled-coil forming protein kinase (ROCK). The present invention relates to compounds of formula (I) 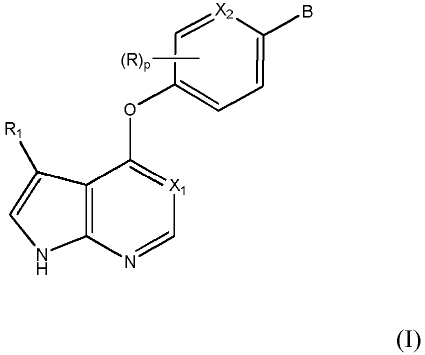 wherein
**X₁,** and **X₂** are in each occurrence independently CH or a nitrogen atom;
p is 0 or an integer from 1 to 3;
each **R**, when present, is at each occurrence independently a halogen; **R₁** is a group of formula **K** 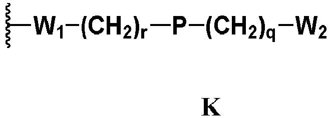 wherein **r** is 0 or an integer from 1 to 4; **q** is 0 or an integer from 1 to 4;
W₁ is an arylene or heteroarylene divalent group selected from A1-A11 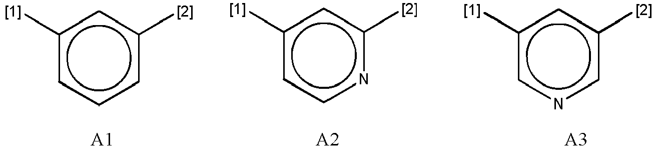 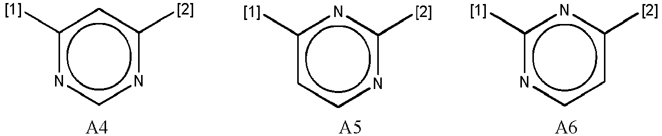 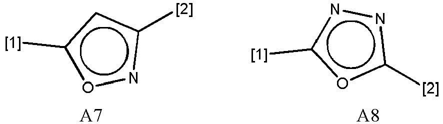 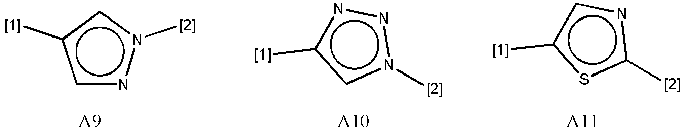
wherein [1] is the point of attachment of W1 to the rest of the molecule and [2] is the point of attachment to -(CH₂)r-;
   **P** is absent or is a divalent group selected from O, S, SO, SO₂, CO, NR₆, N(R₆)(CH₂)nSO₂, N(R₆)COO, N(R₆)(CH₂)ₙC(O), N(R₆)(CH₂)ₙO, SO₂N(R₆), OC(O)N(R₆) C(O)N(R₆), and N(R₆)C(O)N(R₆);
   **W₂** is H or selected from (C₁-C₆) alkyl, (C₃-C₈)cycloalkyl, (C₃-C₈)heterocycloalkyl, aryl, aryl(C₁-C₆)alkyl and heteroaryl, optionally substituted by one or more substituents selected independently from halogen atoms, -OH, oxo (=O), -SH, -NO₂, -CN, -CON(R₆)₂, -C(O)R₆, -NR₆C(O)CH₃, -NH₂, -NHCOR₆, -CO₂R₆, -SO₂N(R₆)₂, -NR₆ SO₂CH₃, (C₁-C₆)alkyl, (C₁-C₆)haloalkyl, (C₁-C₁₀)alkoxy, (C₃-C₈)cycloalkyl, and (C₃-C₆)cycloalkyl-carbonyl;
n is at each occurrence independently 0 or an integer from 1 to 3;
   **R₆** is at each occurrence independently H or selected from (C₁-C₆)alkyl, (C₁-C₆)haloalkyl, (C₂-C₆)alkynyl, (C₂-C₆)alkenyl, (C₃-C₈)cycloalkyl, heteroaryl and aryl optionally substituted by one or more substituents selected from halogen atoms, -OH, oxo (=O), -SH, -NO₂, -CN, -CONH₂, -COOH, , (C₁-C₁₀)alkyl and (C₁-C₁₀)alkoxy;
B is a group of formula R₂R₃N-(C₁-C₆) alkyl, optionally substituted by one or more group selected from hydroxyl, (C₁-C₆) alkyl and (C₁-C₆) hydroxyalkyl;
wherein
R₂ and R₃, the same or different, are H or selected from (C₁-C₆) alkyl, (C₁-C₆) hydroxyalkyl,
   or
B is a group of formula R₄R₃N-(C₁-C₆) alkyl, wherein
R₃ is H, or is selected from (C₁-C₆) alkyl, (C₁-C₆) hydroxyalkyl and R₄ is a divalent alkyl group -(CH₂)ₛ-, wherein s is an integer from 1 to 3, said divalent alkyl group being connected to the carbon atom in the ortho position on the adjacent ring to form a heterocyclic ring, preferably a 4 to 10 membered heterocyclic ring, fused with the adjacent ring forming a bicyclic group; said heterocyclic ring being in its turn further optionally substituted with one or more group selected from (C₁-C₆) alkyl, (C₁-C₆) hydroxyalkyl;
and pharmaceutically acceptable salt and solvates thereof.

### DEFINITIONS

The term "pharmaceutically acceptable salts" refers to derivatives of compounds of formula (I) wherein the parent compound is suitably modified by converting any of the free acid or basic group, if present, into the corresponding addition salt with any base or acid conventionally intended as being pharmaceutically acceptable.

Suitable examples of said salts may thus include mineral or organic acid addition salts of basic residues such as amino groups, as well as mineral or organic basic addition salts of acid residues such as carboxylic groups.

Cations of inorganic bases which can be suitably used to prepare salts within the invention comprise ions of alkali or alkaline earth metals such as potassium, sodium, calcium or magnesium.

Those obtained by reacting the main compound, functioning as a base, with an inorganic or organic acid to form a salt comprise, for example, salts of hydrochloric acid, hydrobromic acid, sulfuric acid, phosphoric acid, methane sulfonic acid, camphor sulfonic acid, acetic acid, oxalic acid, maleic acid, fumaric acid, succinic acid and citric acid.

The term "halogen" or "halogen atoms" includes fluorine, chlorine, bromine, and iodine atom, preferably chlorine or fluorine; meaning Fluoro, Chloro, Bromo, Iodo as substituent.

The term "(C₁-C₆) alkyl" refers to straight-chained or branched alkyl groups wherein the number of constituent carbon atoms is in the range 1 to 6. Particular alkyl groups are methyl, ethyl, n-propyl, isopropyl and t-butyl.

The expressions "(C₁-C₆) haloalkyl" refer to the above defined "(C₁-C₆)alkyl" groups wherein one or more hydrogen atoms are replaced by one or more halogen atoms, which can be the same or different from each other.

Examples of said (C₁-C₆) haloalkyl groups may thus include halogenated, poly-halogenated and fully halogenated alkyl groups wherein all of the hydrogen atoms are replaced by halogen atoms, e.g. trifluoromethyl or difluoro methyl groups.

By way of analogy, the terms "(C₁-C₆) hydroxyalkyl" or "(C₁-C₆) aminoalkyl" refer to the above defined "(C₁-C₆) alkyl" groups wherein one or more hydrogen atoms are replaced by one or more hydroxyl (i.e. hydroxy OH) or amino group respectively. Non limiting examples being respectively hydroxymethyl and aminomethyl and the like.

In the present description, unless otherwise provided, the definition of aminoalkyl encompasses alkyl groups (i.e. "(C₁-C₆) alkyl" groups) substituted by one or more amino group (NR₇R₈). Thus, an example of aminoalkyl is a mono-aminoalkyl group such as R₇R₈N-(C₁-C₆) alkyl.

With reference to the substituent R₇ and R₈ they are defined as R₆ above and, it is here further explained that R₇ and R₈ can be also taken together with the nitrogen atom they are linked to form a 4 to 6 membered heterocyclic radical, at least one further ring carbon atom in the said heterocyclic radical is optionally replaced by at least one heteroatom (e.g. N, S or O) and/or may bear -oxo (=O) substituent groups. It is understood that said heterocyclic radical might be further optionally substituted on any available points in the ring, namely on a carbon atom, or on any heteroatom available for substitution. Substitution on a carbon atom includes spiro disubstitution as well as substitution on two adjacent carbon atoms, in both cases thus form an additional 5 to 6 membered heterocyclic ring. Thus, examples of said heterocycle radicals are 1-pyrrolidinyl, 1-piperidinyl, 1-piperazinyl, 4-morpholinyl, piperazin-4-yl-2-one, 4-methylpiperazine-1-yl,

The term "(C₃-C₁₀) cycloalkyl" likewise "(C₃-C₈) cycloalkyl" and "(C₃-C₆) cycloalkyl" refer to saturated cyclic hydrocarbon groups containing the indicated number of ring carbon atoms. Examples include cyclopropyl, cyclobutyl, cyclopentyl, cyclohexyl and cycloheptyl, and polycyclic ring systems such as adamantan-yl.

The term "(C₂-C₆) alkenyl" refers to straight or branched carbon chains with one or more double bonds, conjugated or not conjugated, in cis or trans configuration, wherein the number atoms is in the range 2 to 6.

The term "(C₂-C₆) alkynyl" refers to straight or branched carbon chains with one or more triple bonds wherein the number atoms is in the range 2 to 6.

The expression "aryl" refers to mono, bi- or tri-cyclic carbon ring systems which have 6 to 20, preferably from 6 to 15 ring atoms, wherein at least one ring is aromatic. The expression "heteroaryl" refers to mono-, bi- or tri-cyclic ring systems with 5 to 20, preferably from 5 to 15 ring atoms, in which at least one ring is aromatic and in which at least one ring atom is a heteroatom (e.g. N, S or O).

Examples of suitable aryl or heteroaryl monocyclic ring systems include, for instance, phenyl, thienyl, pyrrolyl, pyrazolyl, imidazolyl, isoxazolyl, oxazolyl, isothiazolyl, thiazolyl, pyridinyl, pyrimidinyl, pyrazinyl, triazinyl, furanyl radicals and the like.

Examples of suitable aryl or heteroaryl bicyclic ring systems include naphthalenyl, biphenylenyl, purinyl, pteridinyl, pyrazolopyrimidinyl, benzotriazolyl, benzoimidazole-yl, quinolinyl, isoquinolinyl, indolyl, isoindolyl, benzothiopheneyl, benzodioxinyl, dihydrobenzodioxinyl, indenyl, dihydro-indenyl, dihydrobenzodioxepinyl, benzooxazinyl radicals and the like.

Examples of suitable aryl or heteroaryl tricyclic ring systems include fluorenyl radicals as well as benzocondensed derivatives of the aforementioned heteroaryl bicyclic ring systems.

In an analogous manner, the expressions "arylene" and "heteroarylene" refer to divalent groups, such a phenylene, biphenylene and thienylene. Such groups are also commonly named as "arenediyl" or "heteroarenediyl" groups. For example o-phenylene is also named benzene-1,2-diyl. Thienyl-ene is alternatively named thiophenediyl. Other examples of such arylene and heteroarylene groups are pyrimidine-diyl, pyridine-diyl, oxadiazole-diyl, oxazole-diyl, phenyl-diyl, pyrazole-diyl, thiazole-diyl, triazole-diyl.

The derived expressions "(C₃-C₈) heterocycloalkyl", "(C₃-C₆) heterocycloalkyl" refer to saturated or partially unsaturated monocyclic cycloalkyl groups of the indicated number of carbons in which at least one ring carbon atom is replaced by at least one heteroatom (e.g. N, S or O) or may bear an -oxo (=O) substituent group. The said heterocycloalkyl (i.e. heterocyclic radical or group) might be further optionally substituted on the available points in the ring, namely on a carbon atom, or on an heteroatom available for substitution. Substitution on a carbon atom includes spiro disubstitution as well as substitution on two adjacent carbon atoms, in both cases thus form additional condensed 5 to 6 membered heterocyclic ring. Examples of (C₃-C₆) heterocycloalkyl are represented by: pyrrolidinyl, imidazolidinyl, thiazolidinyl, piperazinyl, piperidinyl, morpholinyl, thiomorpholinyl, dihydro- or tetrahydro-pyridinyl, tetrahydropyranyl, pyranyl, 2H- or 4H-pyranyl, dihydro- or tetrahydrofuranyl, dihydroisoxazolyl, pyrrolidin-2-one-yl, dihydropyrrolyl radicals and the like.

Specific examples of said heterocycle radicals are 1-pyrrolidinyl, 1-methyl-2-pyrrolidinyl, 1-piperidinyl, 1-piperazinyl, 4-morpholinyl, piperazin-4-yl-2-one, 4-methylpiperazine-1-yl, 1-methylpiperidin-4-yl, 7-methyl-2,7-diazaspiro[3.5]nonan-2-yl, 2-methyl-2,9-diazaspiro[5.5]undecan-9-yl, 9-methyl-3,9-diazaspiro[5.5]undecan-3-yl, and (3aR,6aS)-5-methyl-octahydropyrrolo[3,4-c]pyrrol-2-yl.

The term "aryl(C₁-C₆)alkyl" refers to an aryl ring linked to a straight-chained or branched alkyl groups wherein the number of constituent carbon atoms is in the range from 1 to 6, e.g. phenylmethyl (i.e. benzyl), phenylethyl or phenylpropyl.

The term "(C₁-C₁₀) alkoxy" or "(C₁-C₁₀) alkoxyl", likewise "(C₁-C₆) alkoxy" or "(C₁-C₆) alkoxyl" etc., refers to a straight or branched hydrocarbon of the indicated number of carbons, attached to the rest of the molecule through an oxygen bridge.

Enchained substituents derive their definition from the composing groups, for example "(C₃-C₆) cycloalkyl-carbonyl", "(C₃-C₆) heterocycloalkyl-carbonyl", "heteroaryl-carbonyl" refer to the above defined groups "(C₃-C₆) cycloalkyl", "(C₃-C₆) heterocycloalkyl", "heteroaryl" attached to the rest of the molecule via a carbonyl group. Examples of such groups being cyclopropanecarbonyl, pyrrolidine-3-carbonyl, (pyridin-3-yl)carbonyl. Whenever basic amino or quaternary ammonium groups are present in the compounds of formula I, physiological acceptable anions, selected among chloride, bromide, iodide, trifluoroacetate, formate, sulfate, phosphate, methanesulfonate, nitrate, maleate, acetate, citrate, fumarate, tartrate, oxalate, succinate, benzoate, p-toluenesulfonate, pamoate and naphthalene disulfonate may be present. Likewise, in the presence of acidic groups such as COOH groups, corresponding physiological cation salts may be present as well, for instance including alkaline or alkaline earth metal ions.

It will be apparent to those skilled in the art that compounds of formula (I) when contain one or more stereogenic center, may exist as optical stereoisomers.

Where the compounds according to the invention have at least one stereogenic center, they may accordingly exist as enantiomers. Where the compounds according to the invention possess two or more stereogenic centers, they may additionally exist as diastereoisomers. It is to be understood that all such single enantiomers, diastereoisomers and mixtures thereof in any proportion are encompassed within the scope of the present invention. The absolute configuration (R) or (S) for carbon bearing a stereogenic center is assigned on the basis of Cahn-Ingold-Prelog nomenclature rules based on groups' priorities.

Atropisomers are resulting from hindered rotation about single bonds where the steric strain barrier to rotation is high enough to allow for the isolation of the conformers (Bringmann G et al, Angew. Chemie Int. Ed. 44 (34), 5384-5427, 2005. doi: 10.1002/anie.200462661).

Oki defined atropisomers as conformers that interconvert with a half-life of more than 1000 seconds at a given temperature (Oki M, Topics in Stereochemistry 14, 1-82, 1983).

Atropisomers differ from other chiral compounds in that in many cases they can be equilibrated thermally whereas in the other forms of chirality isomerization is usually only possible chemically.

Separation of atropisomers is possible by chiral resolution methods such as selective crystallization. In an atropo-enantioselective or atroposelective synthesis one atropisomer is formed at the expense of the other. Atroposelective synthesis may be carried out by use of chiral auxiliaries like a Corey Bakshi Shibata (CBS) catalyst, an asymmetric catalyst derived from proline, or by approaches based on thermodynamic equilibration when an isomerization reaction favors one atropisomer over the other.

Racemic forms of compounds of formula (I) as well as the individual atropisomers (substantially free of its corresponding enantiomer) and stereoisomer-enriched atropisomers mixtures are included in the scope of the present invention.

The invention further concerns the corresponding deuterated derivatives of compounds of formula (I). In the context of the present invention, the term deuterated derivatives refers to compounds in which at least one hydrogen atom is replaced with deuterium at a level above the natural abundance of deuterium. Suitably, the degree of deuteration is at least 50 %, preferably at least 75 %, more preferably ate least 90 %, even more preferably at least 95 %. The degree of deuteration may be 100%, less than 100 %, less than 99 %, or less than 95%. Suitably, the compound is deuterated at one or more exchangeable hydrogen atoms, such as a hydroxyl hydrogen atom, a carboxyl hydrogen atom, an amino hydrogen atom, an amide hydrogen atom, or a thiol hydrogen atom.

It is to be understood that all preferred embodiments described above and herebelow for compounds of formula I may be combined among each other and apply as well *mutatis mutandis.*

In a preferred embodiment, the invention is directed to compounds of formula (I) as above defined wherein each of X₁ and X₂ is a CH; represented by the formula IA: 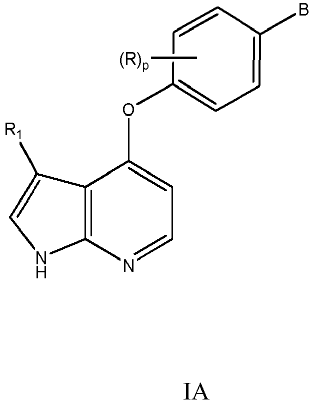 wherein B, R, R1 and p are as above defined.

In another preferred embodiment, the invention is directed to compounds of formula (I) wherein

B is a group of formula R₄R₃N-(C₁-C₆) alkyl wherein R₃ is H, R₄ is connected to the carbon atom in ortho position on the adjacent ring forming a bicyclic group selected from 11-16 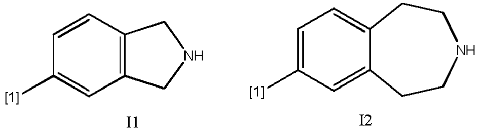 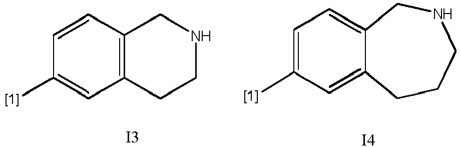 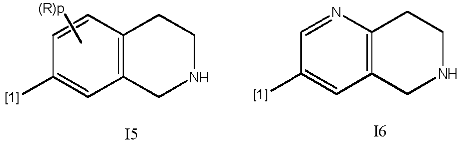
wherein [1] represent the point of attachment of the bicyclic group to the rest of the molecule via the ether bridge,
and pharmaceutically acceptable salts and solvates thereof.

In a further preferred embodiment, the invention is directed to compounds of formula (I) wherein B is connected to the carbon atom in ortho position on the adjacent ring forming a bicyclic group 15, represented by the formula IB 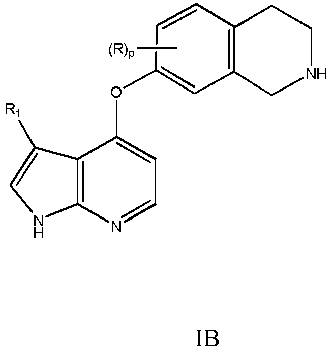 wherein R, R1 and p are as above defined,
and pharmaceutically acceptable salts and solvates thereof.

Particularly preferred in this embodiment, are compounds of formula (IB) wherein **R₁** is a group of formula **K** 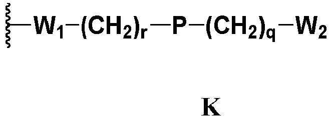
wherein **r** is 0 or an integer from 1 to 4; **q** is 0 or an integer from 1 to 4;
   W1 is an arylene or heteroarylene divalent group selected from A2, A4 or A9
**P** is absent or is a divalent group selected from O, S, SO, SO₂, CO, NR₆, N(R₆)(CH₂)nSO₂, N(R₆)COO, N(R₆)(CH₂)ₙC(O), N(R₆)(CH₂)ₙO, SO₂N(R₆), OC(O)N(R₆) and C(O)N(R₆), N(R₆)C(O)N(R₆);
**W₂** is H or selected from, (C₁-C₆) alkyl, (C₃-C₈)cycloalkyl, (C₃-C₈)heterocycloalkyl, aryl, aryl(C₁-C₆)alkyl and heteroaryl; optionally substituted by one or more substituents selected independently from halogen atoms, -OH, oxo (=O), -SH, -NO₂, -CN, -CON(R₆)₂, -C(O)R₆, -NR₆C(O)CH₃, -NH₂, -NHCOR₆, -CO₂R₆, -SO₂N(R₆)₂, -NR₆ SO₂CH₃, (C₁-C₆)alkyl, (C₁-C₆)haloalkyl (C₁-C₁₀)alkoxy, (C₃-C₈)cycloalkyl, (C₃-C₆)cycloalkyl-carbonyl;
   n is at each occurrence independently 0 or an integer from 1 to 3;
**R₆** is at each occurrence independently H or (C₁-C₆)alkyl;
   and pharmaceutically acceptable salts and solvates thereof.

In another preferred embodiment, the invention is directed to compounds of formula (I), wherein B is a group of formula R₂R₃N-(C₁-C₆) alkyl, R₂ and R₃ are H, and (C₁-C₆) alkyl is methyl, represented by the formula IC, 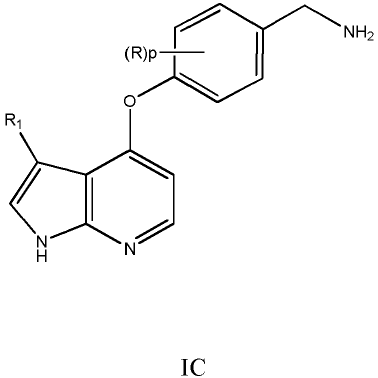
wherein R, R1 and p are as above defined,
and pharmaceutically acceptable salts and solvates thereof.

Particularly preferred in this embodiment, are compounds of formula (IC) wherein
each **R**, when present, is halogen;
**R₁** is a group of formula **K** 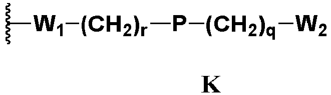 wherein **r** is 0; **q** is 0 or 1;
   W1 is an arylene or heteroarylene divalent group selected from A2, A4 or A9 **P** is absent or is selected from the divalent groups consisting of NR₆, N(R₆)(CH₂)ₙSO₂, and

      N(R₆)(CH₂)ₙC(O);

      **W₂** is selected from, (C₃-C₈)cycloalkyl, (C₃-C₈)heterocycloalkyl, aryl and heteroaryl,
   optionally substituted by one or more (C₁-C₆)alkyl;
   n is at each occurrence independently 0 or an integer from 1 to 3;
      **R₆** is at each occurrence independently H or (C₁-C₆)alkyl;
   and pharmaceutically acceptable salts and solvates thereof.

The invention also provides a pharmaceutical composition comprising a compound of formula I, or a pharmaceutically acceptable salt thereof in admixture with one or more pharmaceutically acceptable carrier or excipient, either alone or in combination with one or more further active ingredient.

In one aspect the invention provides a compound of formula (I) for use as a medicament.

In a further aspect the invention provides the use of a compound (I), or a pharmaceutically acceptable salt thereof, in the manufacture of a medicament for the treatment of disorders associated with ROCK enzymes mechanisms, particularly for the treatment of disorders such as pulmonary diseases.

In particular the invention provides compounds of formula (I) for use in the prevention and /or treatment of pulmonary disease selected from the group consisting of asthma, chronic obstructive pulmonary disease COPD, idiopathic pulmonary fibrosis (IPF), pulmonary hypertension (PH) and specifically Pulmonary Arterial Hypertension (PAH).

Moreover the invention provides a method for the prevention and/or treatment of disorders associated with ROCK enzymes mechanisms, said method comprising administering to a patient in need of such treatment a therapeutically effective amount of a compound of the invention.

In particular the invention provides methods for the prevention and/or treatment wherein the disorder is asthma, chronic obstructive pulmonary disease COPD idiopathic pulmonary fibrosis (IPF), Pulmonary hypertension (PH) and specifically Pulmonary Arterial Hypertension (PAH).

According to specific embodiments, the invention refers to at least one compound and pharmaceutical acceptable salts thereof, selected from the group indicated in Table 1 below.

| **Ex. N.** | **Chemical Name** |
|---|---|
| 1 | 7-((3-(pyridin-4-yl)-1H-pyrrolo[2,3-b]pyridin-4-yl)oxy)-1,2,3,4-tetrahydroisoquinoline |
| 2 | (4-((3-(5-(benzyloxy)pyridin-3-yl)-1H-pyrrolo[2,3-b]pyridin-4-yl)oxy)phenyl)methanamine |
| 3 | (4-((3-(3-(benzyloxy)phenyl)-1H-pyrrolo[2,3-b]pyridin-4-yl)oxy)phenyl)methanamine |
| 4 | 6-(4-(4-(aminomethyl)-2,6-difluorophenoxy)-1H-pyrrolo[2,3-b]pyridin-3-yl)-N-(2-tosylethyl)pyrimidin-4-amine |
| 5 | 4-(4-(4-(aminomethyl)-2,6-difluorophenoxy)-7H-pyrrolo[2,3-d]pyrimidin-5-yl)-N-benzylpyridin-2-amine |
| 6 | N-benzyl-4-(4-((6-fluoro-1,2,3,4-tetrahydroisoquinolin-7-yl)oxy)-7H-pyrrolo[2,3-d]pyrimidin-5-yl)pyridin-2-amine |
| 7 | 4-(4-(4-(aminomethyl)-2,6-difluorophenoxy)-1H-pyrrolo[2,3-b]pyridin-3-yl)-N-(pyridin-3-ylmethyl)pyridin-2-amine |
| 8 | 4-(4-(4-(aminomethyl)-2,6-difluorophenoxy)-1H-pyrrolo[2,3-b]pyridin-3-yl)-N-(cyclopropylmethyl)pyridin-2-amine |
| 9 | N-benzyl-5-(4-((1,2,3,4-tetrahydroisoquinolin-7-yl)oxy)-1H-pyrrolo[2,3-b]pyridin-3-yl)isoxazol-3-amine |
| 10 | N-cyclopropyl-1-(4-(4-((1,2,3,4-tetrahydroisoquinolin-7-yl)oxy)-1H-pyrrolo[2,3-b]pyridin-3-yl)pyridin-2-yl)pyrrolidine-3-carboxamide |
| 11 | N-(4-(4-(4-(aminomethyl)-2,6-difluorophenoxy)-1H-pyrrolo[2,3-b]pyridin-3-yl)pyridin-2-yl)cyclopropanecarboxamide |
| 12 | N-benzyl-2-(4-(4-((1,2,3,4-tetrahydroisoquinolin-7-yl)oxy)-1H-pyrrolo[2,3-b]pyridin-3-yl)-1H-1,2,3-triazol-1-yl)acetamide |
| 13 | N-benzyl-5-(4-((1,2,3,4-tetrahydroisoquinolin-7-yl)oxy)-1H-pyrrolo[2,3-b]pyridin-3-yl)isoxazole-3-carboxamide |
| 14 | 2-phenyl-N-(5-(4-((1,2,3,4-tetrahydroisoquinolin-7-yl)oxy)-1H-pyrrolo[2,3-b]pyridin-3-yl)-1,3,4-oxadiazol-2-yl)acetamide |
| 15 | 4-(4-(4-(aminomethyl)phenoxy)-1H-pyrrolo[2,3-b]pyridin-3-yl)-N-benzylpyridin-2-amine |
| 16 | N-(pyridin-3-ylmethyl)-4-(4-((1,2,3,4-tetrahydroisoquinolin-7-yl)oxy)-1H-pyrrolo[2,3-b]pyridin-3-yl)pyridin-2-amine |
| 17 | 4-(4-(4-((1,2,3,4-tetrahydroisoquinolin-7-yl)oxy)-1H-pyrrolo[2,3-b]pyridin-3-yl)pyridin-2-yl)morpholine |
| 18 | (S)-N-((tetrahydrofuran-2-yl)methyl)-4-(4-((1,2,3,4-tetrahydroisoquinolin-7-yl)oxy)-1H-pyrrolo[2,3-b]pyridin-3-yl)pyridin-2-amine |
| 19 | (R)-N-((tetrahydrofuran-2-yl)methyl)-4-(4-((1,2,3,4-tetrahydroisoquinolin-7-yl)oxy)-1H-pyrrolo[2,3-b]pyridin-3-yl)pyridin-2-amine |
| 20 | N-(2-methoxyethyl)-4-(4-((1,2,3,4-tetrahydroisoquinolin-7-yl)oxy)-1H-pyrrolo[2,3-b]pyridin-3-yl)pyridin-2-amine |
| 21 | 7-((3-(1-methyl-1H-pyrazol-4-yl)-1H-pyrrolo[2,3-b]pyridin-4-yl)oxy)-1,2,3,4-tetrahydroisoquinoline |
| 22 | (4-((3-(1-benzyl-1H-pyrazol-4-yl)-1H-pyrrolo[2,3-b]pyridin-4-yl)oxy)-3-fluorophenyl)methanamine |
| 23 | (3-fluoro-4-((3-(1-(pyridin-4-ylmethyl)-1H-pyrazol-4-yl)-1H-pyrrolo[2,3-b]pyridin-4-yl)oxy)phenyl)methanamine |
| 24 | (3-fluoro-4-((3-(1-((tetrahydro-2H-pyran-4-yl)methyl)-1H-pyrazol-4-yl)-1H-pyrrolo[2,3-b]pyridin-4-yl)oxy)phenyl)methanamine |
| 25 | N-benzyl-6-(4-((1,2,3,4-tetrahydroisoquinolin-7-yl)oxy)-1H-pyrrolo[2,3-b]pyridin-3-yl)pyrimidin-4-amine |
| 26 | N-benzyl-4-(4-((1,2,3,4-tetrahyrolsoquinolin-7-yl)oxy)-1H-pyrrolo[2,3-b]pyridin-3-yl)pyrimidin-2-amine |
| 27 | N-benzyl-6-(4-((1,2,3,4-tetrahyrolsoquinolin-7-yl)oxy)-1H-pyrrolo[2,3-b]pyridin-3-yl)pyridin-2-amine |
| 28 | N-benzyl-2-(4-((1,2,3,4-tetrahydroisoquinolin-7-yl)oxy)-1H-pyrrolo[2,3-b]pyridin-3-yl)pyrimidin-4-amine |
| 29 | N-(2-(phenylsulfonyl)ethyl)-6-(4-((1,2,3,4-tetrahydroisoquinolin-7-yl)oxy)-1H-pyrrolo[2,3-b]pyridin-3-yl)pyrimidin-4-amine |
| 30 | 6-(4-((1,2,3,4-tetrahydroisoquinolin-7-yl)oxy)-1H-pyrrolo[2,3-b]pyridin-3-yl)-N-(thiazol-2-ylmethyl)pyrimidin-4-amine |
| 31 | 6-(4-(4-(aminomethyl)-2-fluorophenoxy)-1H-pyrrolo[2,3-b]pyridin-3-yl)-N-benzylpyrimidin-4-amine |
| 32 | 4-(4-(4-(aminomethyl)-2-fluorophenoxy)-1H-pyrrolo[2,3-b]pyridin-3-yl)-N-benzylpyrimidin-2-amine |
| 33 | 4-(4-(4-(aminomethyl)-2-fluorophenoxy)-1H-pyrrolo[2,3-b]pyridin-3-yl)-N-benzylpyridin-2-amine |
| 34 | N-(5-(4-(4-(aminomethyl)phenoxy)-1H-pyrrolo[2,3-b]pyridin-3-yl)thiazol-2-yl)-2-phenylacetamide |
| 35 | 4-(4-((1,2,3,4-tetrahydroisoquinolin-7-yl)oxy)-1H-pyrrolo[2,3-b]pyridin-3-yl)-N-(thiazol-2-ylmethyl)pyridin-2-amine |
| 36 | N-(cyclopropylmethyl)-4-(4-((1,2,3,4-tetrahydroisoquinolin-7-yl)oxy)-1H-pyrrolo[2,3-b]pyridin-3-yl)pyridin-2-amine |
| 37 | N-((5-methylpyridin-2-yl)methyl)-4-(4-((1,2,3,4-tetrahydroisoquinolin-7-yl)oxy)-1H-pyrrolo[2,3-b]pyridin-3-yl)pyridin-2-amine |
| 38 | N-((tetrahydro-2H-pyran-4-yl)methyl)-4-(4-((1,2,3,4-tetrahydroisoquinolin-7-yl)oxy)-1H-pyrrolo[2,3-b]pyridin-3-yl)pyridin-2-amine |
| 39 | N-(4-(4-((1,2,3,4-tetrahydroisoquinolin-7-yl)oxy)-1H-pyrrolo[2,3-b]pyridin-3-yl)pyridin-2-yl)cyclopropanecarboxamide |
| 40 | N-(4-(4-((1,2,3,4-tetrahydroisoquinolin-7-yl)oxy)-1H-pyrrolo[2,3-b]pyridin-3-yl)pyridin-2-yl)cyclohexanecarboxamide |
| 41 | N-(4-(4-((1,2,3,4-tetrahydroisoquinolin-7-yl)oxy)-1H-pyrrolo[2,3-b]pyridin-3-yl)pyridin-2-yl)tetrahydro-2H-pyran-4-carboxamide |
| 42 | 2-isopropoxy-N-(4-(4-((1,2,3,4-tetrahydroisoquinolin-7-yl)oxy)-1H-pyrrolo[2,3-b]pyridin-3-yl)pyridin-2-yl)acetamide |
| 43 | 2-(piperidin-1-yl)-N-(4-(4-((1,2,3,4-tetrahydroisoquinolin-7-yl)oxy)-1H-pyrrolo[2,3-b]pyridin-3-yl)pyridin-2-yl)acetamide |
| 44 | 1-methyl-N-(4-(4-((1,2,3,4-tetrahydroisoquinolin-7-yl)oxy)-1H-pyrrolo[2,3-b]pyridin-3-yl)pyridin-2-yl)cyclopropanecarboxamide |
| 45 | 2-amino-2-phenyl-N-(4-(4-((1,2,3,4-tetrahydroisoquinolin-7-yl)oxy)-1H-pyrrolo[2,3-b]pyridin-3-yl)pyridin-2-yl)acetamide |
| 46 | 4,4-dimethyl-N-(4-(4-((1,2,3,4-tetrahydroisoquinolin-7-yl)oxy)-1H-pyrrolo[2,3-b]pyridin-3-yl)pyridin-2-yl)piperidine-1-carboxamide |
| 47 | 1-cyclohexyl-1-methyl-3-(4-(4-((1,2,3,4-tetrahydroisoquinolin-7-yl)oxy)-1H-pyrrolo[2,3-b]pyridin-3-yl)pyridin-2-yl)urea |
| 48 | 1-methyl-1-(tetrahydro-2H-pyran-4-yl)-3-(4-(4-((1,2,3,4-tetrahydroisoquinolin-7-yl)oxy)-1H-pyrrolo[2,3-b]pyridin-3-yl)pyridin-2-yl)urea |
| 49 | N-benzyl-4-(4-((5,6,7,8-tetrahydro-1,6-naphthyridin-3-yl)oxy)-1H-pyrrolo[2,3-b]pyridin-3-yl)pyridin-2-amine |
| 50 | N-(6-(4-((1,2,3,4-tetrahydroisoquinolin-7-yl)oxy)-1H-pyrrolo[2,3-b]pyridin-3-yl)pyrimidin-4-yl)benzamide |
| 51 | N-benzyl-5-(4-((1,2,3,4-tetrahydroisoquinolin-7-yl)oxy)-1H-pyrrolo[2,3-b]pyridin-3-yl)pyridin-3-amine |
| 52 | (R)-N-(1-phenylethyl)-6-(4-((1,2,3,4-tetrahydroisoquinolin-7-yl)oxy)-1H-pyrrolo[2,3-b]pyridin-3-yl)pyrimidin-4-amine |
| 53 | N-phenethyl-6-(4-((1,2,3,4-tetrahydroisoquinolin-7-yl)oxy)-1H-pyrrolo[2,3-b]pyridin-3-yl)pyrimidin-4-amine |
| 54 | (S)-N-(1-phenylethyl)-6-(4-((isoquinolin-7-yl)oxy)-1H-pyrrolo[2,3-b]pyridin-3-yl)pyrimidin-4-amine |
| 55 | N-(2-(pyridin-3-yl)ethyl)-6-(4-((1,2,3,4-tetrahydroisoquinolin-7-yl)oxy)-1H-pyrrolo[2,3-b]pyridin-3-yl)pyrimidin-4-amine |
| 56 | N-benzyl-4-(4-((1,2,3,4-tetrahydroisoquinolin-7-yl)oxy)-7H-pyrrolo[2,3-d]pyrimidin-5-yl)pyridin-2-amine |

The compounds of the invention, including all the compounds here above listed, can be prepared from readily available starting materials using the following general methods and procedures or by using slightly modified processes readily available to those of ordinary skill in the art. When typical or preferred process conditions (i.e. reaction temperatures, times, mole ratios of reactants, solvents, pressures, etc.) are given, other process conditions can also be used unless otherwise stated.

Thus, processes of preparation described below and reported in the following schemes should not be viewed as limiting the scope of the synthetic methods available for the preparation of the compounds of the invention.

In some cases where a step is needed in order to mask or protect sensitive or reactive moieties, generally known protective groups (PG) could be employed, in accordance with general principles of chemistry (Protective group in organic syntheses, 3rd ed. T. W. Greene, P. G. M. Wuts).

The compounds of formula **I**, including all the compounds here above listed, can be generally prepared according to the procedures shown in the schemes below. Where a specific step differs from the general schemes it has been detailed in the specific examples, and/or in additional schemes.

Compounds of formula **I** may contain one or more stereogenic centre in groups **R₁** and **B**. 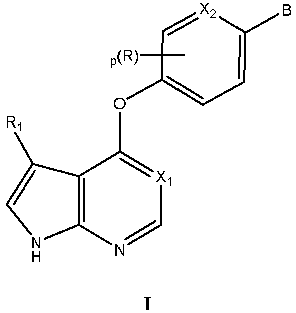 ( wherein **R₁** is a group **K** of formula -W₁-(CH₂)ᵣ-P-(CH₂)_{q}-W₂ )

Enantiomerically pure compounds can be prepared according to the reactions described below, by means of enantiomerically pure starting materials and suitable enantiomerically pure intermediates commercially available or readily produced from commercial sources by those of ordinary skill in the art.

In another approach, enantiomerically pure compounds can be prepared from the corresponding racemates by means of chiral chromatographic separation. Whenever, in compounds of formula **I,** there are two or more stereogenic centres, the structure is then characterized by different stereoisomers. Stereochemically pure compounds may be obtained by chiral separation of a diastereoisomeric mixture, or stepwise by chromatographic separation of diastereoisomers followed by further chiral separation into single stereoisomers.

For synthetic convenience, where group **B** contains one or more primary or secondary amino moiety, it may require one or more protective groups in order to mask amino group reactivity. For example, a suitable protective group for the amino moiety can be a carbamate such as Boc (tert-butoxycarbonyl), Cbz (benzyloxycarbonyl) or ethyl carbamate. For the sake of clarity, group **B** in its protected form, wherein all primary and secondary amino moiety are protected as carbamate, is represented by group w in the schemes.

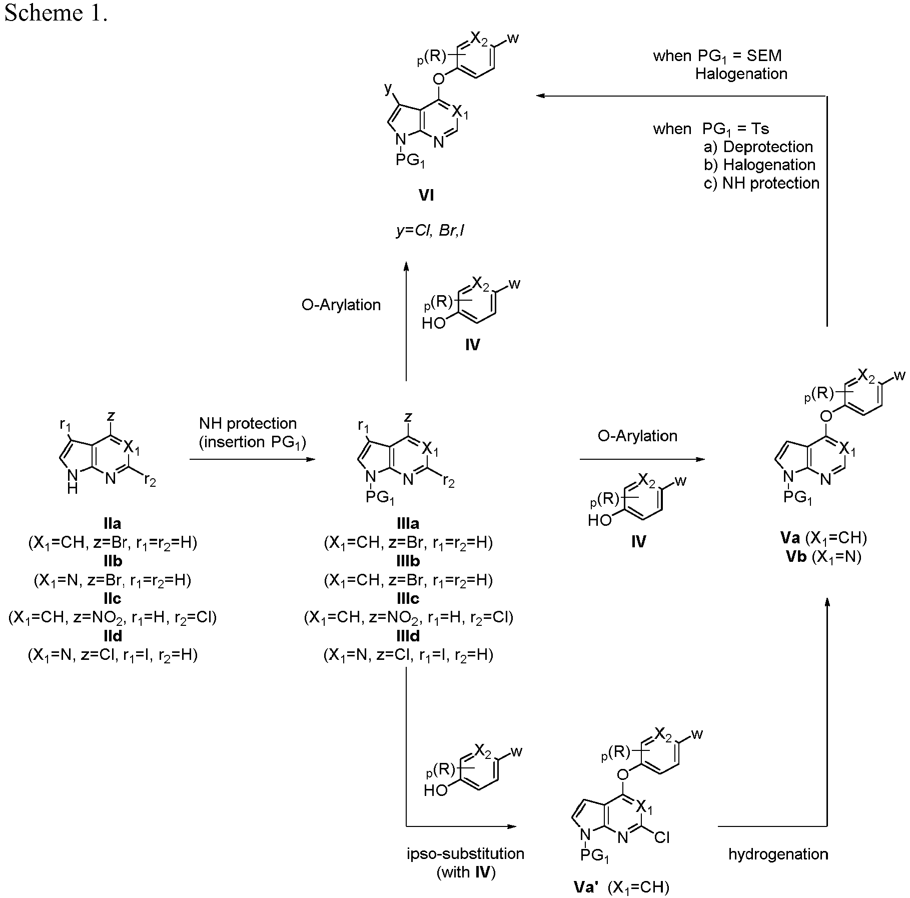

Typical protective groups (PG₁) for protection of the NH of the 5-membered ring of the bicyclic intermediate **IIa-d** may be 2-[(trimethylsilyl)ethoxy]methyl (SEM), 4-toluenesulfonyl (Ts) and p-methoxybenzyl (PMB), and anyhow not limiting the use of other protective groups. Intermediate **IIIa-d** may be prepared from the corresponding intermediate **IIa-d** and a suitable reagent for PG₁ introduction, for example SEM-C1 ([2-(trimethylsilyl)ethoxy]methyl chloride) when PG₁ is a SEM or Ts-Cl (tosyl chloride) when PG₁ is a Ts group. Reaction between said components (intermediate **IIa-d** and SEM-C1 or Ts-Cl) may be carried out in an organic solvent such as DMF, MeCN or DCM, in the presence of a strong base, such as NaH, or an organic base such as DIPEA at room temperature (RT) or lower.

Intermediate **Va** (or **Vb**) may be obtained from Intermediates **IIIa** (or **IIIb**) and **IV** through a palladium catalyzed O-arylation. For example the reaction may be carried out by reacting the aryl halide intermediate **IIIa** (or **IIIb**) and the phenol derivative **IV** in a suitable organic solvent such as toluene or THF, in the presence of an inorganic base such as K₂CO₃, with a suitable palladium catalytic system such as Pd₂dba₃ / XPhos or an alternative palladium source/phosphine based ligand at high temperature (around 100°C) for a time ranging from a few hours to overnight.

In a different approach, intermediate **Va** may be obtained with a two-step synthesis starting from intermediate **IIIc**. In the first step, the ipso-substitution of the nitro group of the intermediate **IIIc** by the phenol of intermediate **IV** gives intermediate **Va'**. The reaction may be carried out in a high boiling organic solvent such as DMSO, at a temperature equal to 100°C or higher and in the presence of an inorganic base such as K₂CO₃. In the second step, the intermediate **Va'** can be converted into intermediate **Va** by removing the chlorine atom by means of heterogeneous palladium catalyzed hydrogenation. The reaction can be performed by reacting intermediate **Va'** under a hydrogen atmosphere in the presence of Pd/C and an organic base such as TEA.

Intermediate **Va** (or **Vb**), wherein PG₁ is SEM, may be converted into the intermediate **VI** by an electrophilic halogenation with the corresponding N-halosuccinimide, for example when *y* is iodine, the reaction can be carried out by reacting **Va** (or **Vb**) with NIS (N-Iodosuccinimide) in an organic solvent such as MeCN and at temperature around RT for a few hours. Conversion of intermediate **Va** (or **Vb**) into intermediate **VI**, wherein PG₁ is Ts, can be accomplished as described above when PG1 is SEM or alternatively by means of a three step process that involves deprotection of Ts, halogenation and re-insertion of PG₁ as tosyl group (or SEM). The tosyl group (Ts) may be hydrolysed in a solution of inorganic base such as LiOH in water and organic solvent such as MeOH and/or THF at a temperature equal to RT or higher. Halogenation was performed as described above for the preparation of **VI** while the re-insertion of tosyl (or SEM) may be carried out by applying the same conditions already described in the current scheme for the conversion of intermediates **IIa-d** in the preparation of **IIIa-d**.

In another approach, intermediate **VI** (wherein X₁ is N) may be obtained from O-arylation of intermediate **IIId** by means of an aromatic nucleophilic substitution reaction (SNAr) by reacting said intermediates, **IV** and **IIId**, in a polar organic solvent such as DMSO, in presence of an inorganic base such as K₂CO₃ and at a temperature around 80°C or higher. The scheme 1 provides at least one non-limiting synthetic route for the preparation of intermediates 2b, 2c, 2e, 2g, 7a, 7b, 7f, 14a, and 14b that are used hereinafter for the preparation of compounds of invention of formula **I.**

Compounds of formula **I**, may be prepared according to scheme 2 that provides at least one non-limiting synthetic route for the preparation of examples 1 to 6, example 15, examples 21 to 34 and examples 50 to 55.

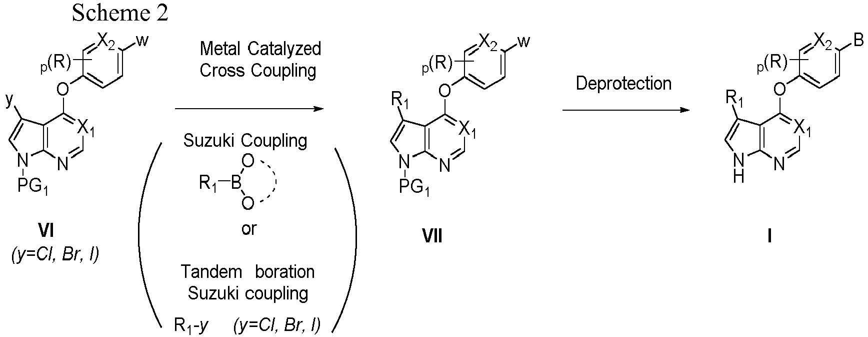

Intermediate **VI**, may be converted into the intermediate **VII** by direct introduction of group **R₁** by a metal catalyzed cross coupling reaction such as Suzuki coupling, tandem boration-Suzuki coupling or similar reactions (Strategic application of named reactions in organic synthesis, L. Kurti, B. Czako, Ed. 2005).

For example, a Suzuki coupling can be performed by reacting intermediate **VI** with the corresponding boronic acid or boron pinacolate ester of group **R₁,** in the presence of a Pd catalyst such as PdCl₂(dppf)₂ DCM adduct, tetrakistriphenylphosphinepalladium(0) or PdCl₂/ suitable phosphine ligand, in an organic solvent such as DME, THF or DMF with or without water, with an inorganic base such as an alkaline carbonate (for example Cs₂CO₃) or a phosphate (for example K₃PO₄), at high temperature (100 - 150°C). Boronic acid and boronic pinacolate esters are generally commercially available or may be readily prepared starting from commercially available reagents. Experimental procedures for the preparation of those boronic acid and boronic pinacolate not commercially available are reported into experimental section.

Alternatively, intermediate **VI** may be converted into the intermediate **VII** through tandem boration-Suzuki coupling. Intermediate **VI** can be reacted with a diboron acid or ester (such as tetrahydroxydiboron or bis-pinacol-diboron ester), in the presence of a palladium catalyst such as Pd-X-Phos catalyst, PdCl₂(dppf)₂ or tetrakistriphenylphosphinepalladium, with an inorganic base (such as KOAc), in a protic organic solvent such as MeOH or EtOH and at a temperature equal or higher than 80°C for a few hours. Subsequently, the resulting boron intermediate can be reacted in situ with the halide R₁-y (wherein y is Cl, Br or I) in the presence of a stronger inorganic base such as K₂CO₃. R₁-y (wherein y is Cl, Br or I) are generally commercially available or readily prepared starting from commercially available reagents. Experimental procedures for those R₁-y not commercially available are reported in the experimental section.

Removal of all protective groups from intermediate **VII**, (PG₁ and Boc carbamate contained in **w**) to give compounds of formula **I** may be achieved using generally know methods (Protective group in organic syntheses, 3rd ed. T. W. Greene, P. G. M. Wuts). For example, when PG₁ is SEM and **w** contains one or more Boc groups, cleavage may be achieved by an acidic treatment using TFA in an organic solvent such as DCM or by mineral acids in organic solvents such as hydrochloric acid in dioxane. Complete removal of SEM group may require an extra treatment with a solution of ammonia in methanol or aqueous sodium hydroxide. Removal of protective group from intermediate **VII**, when PG₁ is Ts, can be achieved by hydrolysis using an inorganic base such as LiOH in mixture of water and an organic solvent such as methanol and/or THF at RT or higher. Removal of a Boc group contained into **w** can be achieved by acidic treatment, for example using TFA in an organic solvent such as DCM or a mineral acid in an organic solvent such as hydrochloric acid in dioxane.

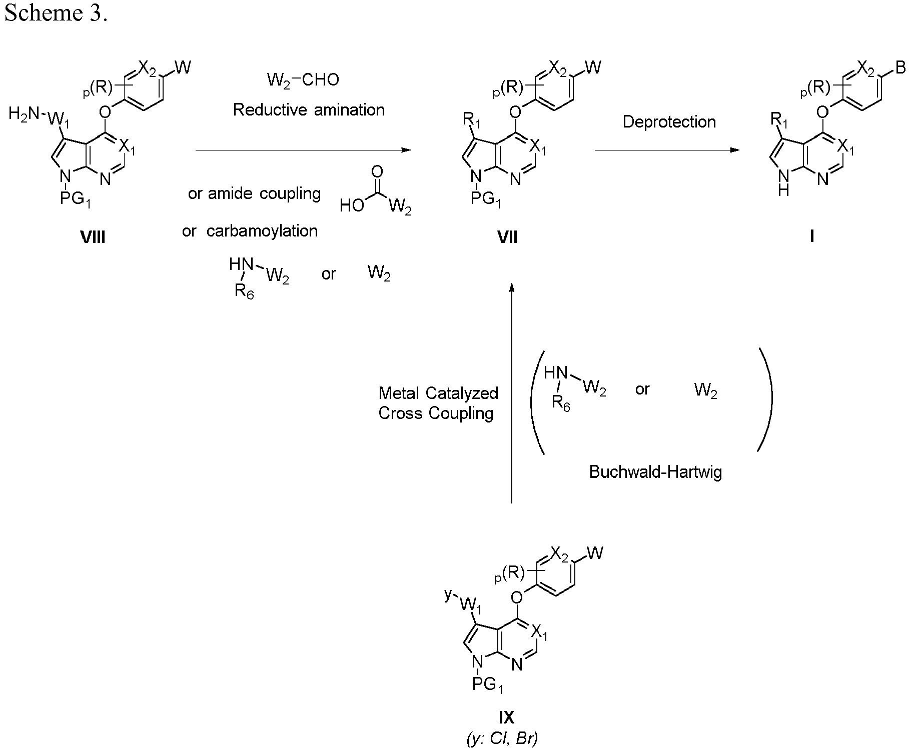

In another approach, a compound of formula **I** (wherein R₁ is -W₁-(CH₂)ᵣ-P-(CH₂)_{q}-W₂) may be prepared from intermediates **VIII** or **IX** according to scheme 3, providing at least one non-limiting synthetic route for the preparation of examples 7 to 8, examples 10-11, examples 16 to 20, examples 35 to 49 and example 56.

Intermediates **VIII** and **IX** can be prepared in a similar way to intermediate **VII** by Suzuki coupling starting from intermediate **VI** and the corresponding boronic acid or boronic pinacol ester to insert groups -W₁-NH₂ or respectively -W₁-y (wherein y is Cl or Br) as already described in scheme 2.

Intermediate **VIII** may be reacted with an aldehyde of formula W₂-CHO by means of a reductive amination reaction to give an intermediate **VII** (wherein R₁ is W₁-NH-CH₂-W₂). Reductive amination can be performed by reacting aldehyde W₂-CHO, intermediate **VIII** and a reducing agent such as NaBH(OAc)₃, NaBH₃CN or NaBH₄, in a suitable organic solvent such as DCE, DCM, THF or MeOH. The reaction proceeds smoothly at room temperature over a couple of hours. It could be useful to react the amine and the aldehyde to pre-form the imine before adding the reducing agent, also by the use of dry molecular sieves.

Intermediate **VII**, wherein R₁ contains an amide group N-linked to W₁, can be obtained by reaction of intermediate **VIII** by means of an amide coupling with an acid intermediate of formula W₂COOH. The reaction can be carried out by reacting **VIII** and W₂COOH in the presence of a coupling agent such as 1-(methylsulfonyl)-1H-benzotriazole, COMU or TBTU, and an organic base such as TEA, DIPEA or DMAP, in an organic solvent such as THF, DCM or DMF at room temperature or higher than 100°C, also by using microwave irradiation.

Intermediate **VII**, wherein R₁ contains a urea group N-linked to W₁, may be obtained from intermediate **VIII** by a two-step carbamoylation process. The first step involves the transformation of intermediate **VIII** into the corresponding isocyanate by reaction with a phosgene equivalent such as triphosgene or diphosgene, in an organic solvent such as THF or DCM and in the presence of an organic base such as TEA or DIPEA, then followed by tandem reaction with the corresponding amine of formula W₂-NHR₆ or W₂ (wherein W₂ is a (C₃-C₈)heterocycloalkyl moiety comprising a secondary amine) by heating at a temperature of 70°C or higher for times up to 18 hours.

Intermediate **VII**, wherein R₁ is -W₁-P-(CH₂)_{q}-W₂, may be obtained from intermediate **IX** and an amine of formula W₂ (wherein W₂ is a (C₃-C₈)heterocycloalkyl moiety comprising a secondary amine) or W₂-NHR₆ by means of a metal catalyzed cross-coupling such as a Buchwald-Hartwig coupling. Intermediate **IX** and an amine of formula W₂ or W₂-NHR₆ can be reacted in the presence of a Pd catalyst and a phosphine ligand such as Pd₂(dba)₃ / Xantphos or Pd(OAc)₂ / BINAP, in the presence of a base such as sodium tert-butoxide or Cs₂CO₃, in an organic solvent such as dioxane, toluene or THF by heating at a temperature up to 100-120°C for a few hours. In some cases, when PG₁ is Ts, reaction condition may concurrently lead to deprotection of the tosyl group.

Intermediates **VII** (wherein R₁ is -W₁-(CH₂)ᵣ-P-(CH₂)_{q}-W₂) can be converted into compounds of formula **I** following the same procedures already described above and reported for scheme 2.

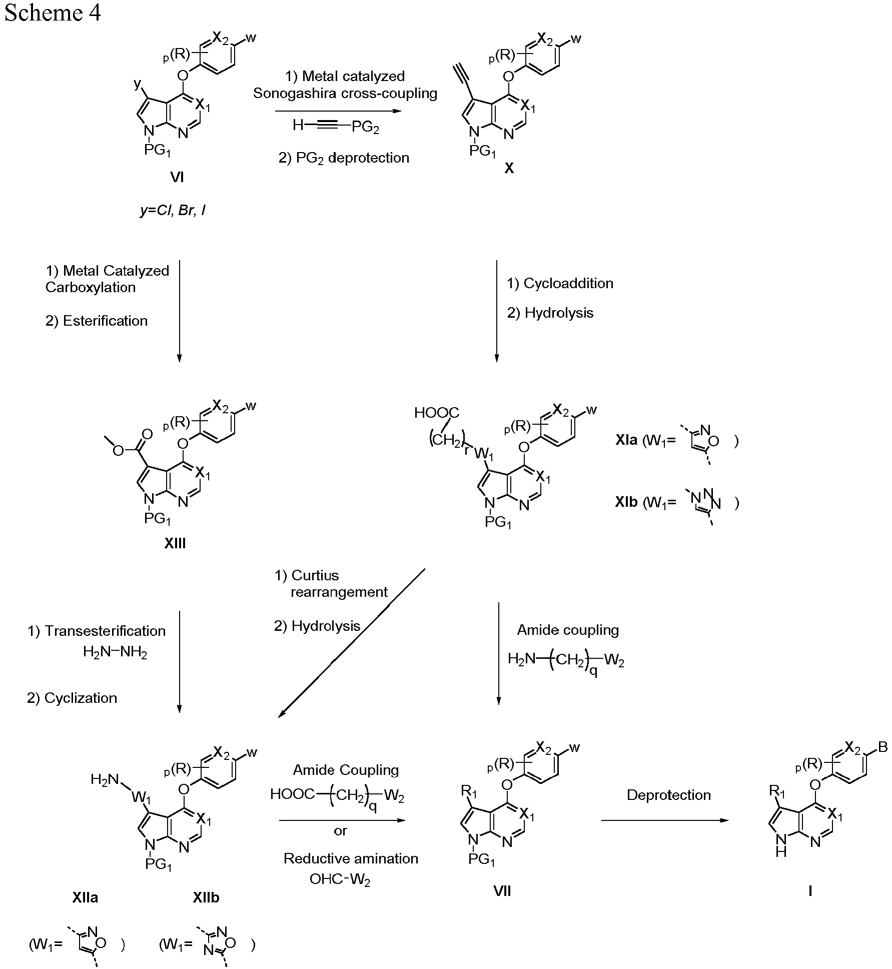

In an alternative approach compound of formula **I** (wherein W₁ arylene is a five-membered heterocycle A7, A8 or A10) may be obtained according to scheme 4 providing at least one non-limiting synthetic route for the preparation of example 9 and examples 12-14.

Intermediate **VI** may be ethynylated to give **X** by a two-step process, the first involves a metal catalyzed Sonogashira cross coupling to insert a PG₂ protected ethynyl group, followed by deprotection of PG₂ to give free ethynyl. For example, a Sonogashira coupling may be performed by heating the intermediate **VI** and a suitable alkyne PG₂C≡CH, in the presence of a Pd catalyst such as Pd(PPh₃)₂Cl₂ or tetrakistriphenylphosphinepalladium (0) and a copper iodide (I) salt, an organic base such as diisopropyl amine or trimethylamine, in an organic solvent such as DCM or THF, at a temperature around RT or higher for a few hours. When PG₂ is a trimethylsilane group (TMS), deprotection may be performed by hydrolysis in an organic protic solvent such as MeOH and in the presence of an inorganic base such as potassium carbonate. Intermediates **XIa** or **XIb** may be prepared from intermediate **X** and a suitable dipolarophile bearing a protected carboxylic acid, through a 1,3-dipolar cycloaddition reaction, followed by ester hydrolysis to give free acid intermediates.

Intermediate **XIa** (wherein W₁ is a isoxazol-3,5-diyl radical) can be prepared by reaction of intermediate **X** and a suitable dipolarophile precursor of a nitriloxide such as ethyl chloro(oximido)acetate by reaction in an organic solvent such as MeOH and in the presence of an inorganic base such as K₂CO₃, then followed by ethyl ester hydrolysis carried out in a mixture of water and an organic solvent such as THF with an inorganic base such as LiOH.

Intermediate **XIb** (wherein W₁ is a 1,2,3-triazol-1,4-diyl radical) may be prepared by reaction of intermediate **X** and N₃-(CH₂)ᵣ-COOEt in an organic solvent such as THF and in the presence of a Cu(II) salt / reducing agent such as copper sulfate (II) / L-ascorbate, then followed by ethyl ester hydrolysis carried out in a mixture of water and an organic solvent such as THF with an inorganic base such as LiOH.

Intermediate **VII** (wherein R₁: W₂-(CH₂)_{q}-NH-C(O)-W₁-, and W₁ : isoxazol-3,5-diyl radical) or intermediate **VII** (wherein R₁: W₂-(CH₂)_{q}-NH-C(O)-W₁-, and W₁ : 1,2,3-triazol-1,4-diyl radical) can be obtained by an amide coupling of respectively intermediate **XIa** or **XIb** with an amine of formula W₂-(CH₂)_{q}-NH₂ by using similar conditions described for the amide coupling to prepare intermediate **VII** in scheme 3.

Intermediate **XIIa** (wherein W₁ is a isoxazol-3,5-diyl radical) may be obtained from intermediate **XIa** by Curtius rearrangement and subsequent hydrolysis of the resulting carbamate. For example, intermediate **XIa** can be reacted with diphenylphosphorylazide in the presence of an organic base such as TEA, in an organic solvent such as toluene at temperature higher than RT to form *in situ* acylazide and subsequently degradation to isocyanate, followed by reaction with phenol to form the corresponding carbamate that upon aqueous hydrolysis can give free amine intermediate **XIIa**.

In another approach, intermediate **VI** may be converted into intermediate **XIII** by a metal catalyzed carbonylation followed by an esterification for introducing the methyl ester. An example of carbonylation may be performed by reaction of intermediate **VI** with a source of carbon monoxide such as lithium formate / acetic anhydride, in the presence of lithium chloride and an organic base such as DIPEA, in a polar organic solvent such as DMF and in the presence of a palladium catalyst such as Pd(OAc)₂ at a temperature around 50°C or higher. Conversion to the methyl ester may be achieved by reaction of the free carboxylic acid with an activating agent such as CDI followed by quenching with MeOH. Intermediate **XII** can be converted into intermediate **XIIb** (wherein W₁ is a 1,3,4-oxadiazol-2,5-diyl radical) by a two-step process that involves formation of a hydrazide by direct reaction of **XII** with hydrazine in MeOH at reflux, followed by cyclization with a methanimine equivalent such as di(1H-imidazol-1yl)methanimine.

Intermediate **XIIa** (wherein W₁ is a isoxazol-3,5-diyl radical) and intermediate **XIIb** (wherein W₁ is a 1,3,4-oxadiazol-2,5-diyl radical) may be converted into intermediate **VII** by amide coupling with W₂-(CH₂)_{q}-COOH or reductive amination with W₂-CHO following the same procedures already described for intermediate **VIII**.

Compound of formula **I** (wherein W₁ arylene is a five-membered heterocycle A7, A8 or A10) may be obtained from the corresponding intermediates **VII** obtained within this scheme and following the same procedure for deprotection already reported in scheme 2.

The compounds of the invention are suitable as inhibitors of kinase activity, in particular Rho-kinase activity.

In one embodiment, the disorders that can be treated by the compounds of the invention include glaucoma, inflammatory bowel disease (IBD) and pulmonary diseases selected from asthma, chronic obstructive pulmonary disease (COPD), interstitial lung disease such as idiopathic pulmonary fibrosis (IPF) and pulmonary arterial hypertension (PAH).

In another embodiment, the disorder that can be treated by the compounds of the invention is selected from the group consisting of asthma, chronic obstructive pulmonary disease (COPD) and interstitial lung disease such as idiopathic pulmonary fibrosis (IPF) and pulmonary arterial hypertension (PAH).

In a further embodiment, the disorder is preferably selected from idiopathic pulmonary fibrosis (IPF) and pulmonary arterial hypertension (PAH).

The methods of treatment of the invention comprise administering a safe and effective amount of a compound of formula (I), or a pharmaceutically acceptable salt thereof according to anyone of the above described preferred embodiments, to a patient in need thereof. As used herein, "safe and effective amount" in reference to a compound of formula (I) or other pharmaceutically-active agent means an amount of the compound sufficient to treat the patient's condition but low enough to avoid serious side effects and it can nevertheless be routinely determined by the skilled artisan. The compounds of formula (I) or pharmaceutically acceptable salts thereof may be administered once or according to a dosing regimen wherein a number of doses are administered at varying intervals of time for a given period of time. Typical daily dosages may vary depending upon the particular route of administration chosen.

The invention also provides pharmaceutical compositions of compounds of formula (I) or a pharmaceutically acceptable salt thereof, in admixture with one or more pharmaceutically acceptable carrier or excipient, for example those described in Remington's Pharmaceutical Sciences Handbook, XVII Ed., Mack Pub., N.Y., U.S.A.

Administration of the compounds of the invention and their pharmaceutical compositions may be accomplished according to patient needs, for example, orally, nasally, parenterally (subcutaneously, intravenously, intramuscularly, intrasternally and by infusion), by inhalation, rectally, vaginally, topically, locally, transdermally, and by ocular administration.

Various solid oral dosage forms can be used for administering compounds of the invention including such solid forms as tablets, gelcaps, capsules, caplets, granules, lozenges and bulk powders. The compounds of the present invention can be administered alone or combined with various pharmaceutically acceptable carriers, diluents (such as sucrose, mannitol, lactose, starches) and known excipients, including suspending agents, solubilizers, buffering agents, binders, disintegrants, preservatives, colorants, flavorants, lubricants and the like. Time release capsules, tablets and gels are also advantageous.

Various liquid oral dosage forms can also be used for administering compounds of the invention, including aqueous and non-aqueous solutions, emulsions, suspensions, syrups, and elixirs. Such dosage forms can also contain suitable known inert diluents such as water and suitable known excipients such as preservatives, wetting agents, sweeteners, flavorants, as well as agents for emulsifying and/or suspending the compounds of the invention. The compounds of the invention may be injected, for example, intravenously, in the form of an isotonic sterile solution. Other preparations are also possible.

Suppositories for rectal administration of the compounds of the invention can be prepared by mixing the compound with a suitable excipient such as cocoa butter, salicylates and polyethylene glycols.

Formulations for vaginal administration can be in the form of cream, gel, paste, foam, or spray formula containing, in addition to the active ingredient, such as suitable carriers, are also known.

For topical administration the pharmaceutical composition can be in the form of creams, ointments, liniments, lotions, emulsions, suspensions, gels, solutions, pastes, powders, sprays, and drops suitable for administration to the skin, eye, ear or nose. Topical administration may also involve transdermal administration via means such as transdermal patches.

For the treatment of the diseases of the respiratory tract, the compounds of the invention are preferably administered by inhalation.

Inhalable preparations include inhalable powders, propellant-containing metering aerosols or propellant-free inhalable formulations.

For administration as a dry powder, known single- or multi-dose inhalers may be utilized. In that case the powder may be filled in gelatine, plastic or other capsules, cartridges or blister packs or in a reservoir.

A diluent or carrier, generally non-toxic and chemically inert to the compounds of the invention, e.g. lactose or any other additive suitable for improving the respirable fraction may be added to the powdered compounds of the invention.

Inhalation aerosols containing propellant gas such as hydrofluoroalkanes may contain the compounds of the invention either in solution or in dispersed form. The propellant-driven formulations may also contain other ingredients such as co-solvents, stabilizers and optionally other excipients.

The propellant-free inhalable formulations comprising the compounds of the invention may be in form of solutions or suspensions in an aqueous, alcoholic or hydroalcoholic medium and they may be delivered by jet or ultrasonic nebulizers known from the prior art or by soft-mist nebulizers such as Respimat^{®}.

The compounds of the invention can be administered as the sole active agent or in combination (i.e. as co-therapeutic agents administered in fixed dose combination or in combined therapy of separately formulated active ingredients) with other pharmaceutical active ingredients selected from organic nitrates and NO donors; inhaled NO; stimulator of soluble guanylate cyclase (sGC); prostaciclin analogue PGI2 and agonist of prostacyclin receptors; compounds that inhibit the degradation of cyclic guanosine monophosphate (cGMP) and/or cyclic adenosine monophosphate (cAMP), such as inhibitors of phosphodiesterases (PDE) 1 , 2, 3, 4 and/or 5, especially PDE 5 inhibitors; human neutrophilic elastase inhibitors; compounds inhibiting the signal transduction cascade, such as tyrosine kinase and/or serine/threonine kinase inhibitors; antithrombotic agents, for example platelet aggregation inhibitors, anticoagulants or profibrinolytic substances; active substances for lowering blood pressure, for example calcium antagonists, angiotensin II antagonists, ACE inhibitors, endothelin antagonists, renin inhibitors, aldosterone synthase inhibitors, alpha receptor blockers, beta receptor blockers, mineralocorticoid receptor antagonists; neutral endopeptidase inhibitor; osmotic agents; ENaC blockers; antiinflammatories including corticosteroids and antagonists of chemokine receptors; bronchodilators for example beta2agonist and muscarinic antagonists; antihistamine drugs; anti-tussive drugs; antibiotics such as macrolide and DNase drug substance and selective cleavage agents such as recombinant human deoxyribonuclease I (rhDNase); agents that inhibit ALK5 and/or ALK4 phosphorylation of Smad2 and Smad3; tryptophan hydroylase 1 (TPH1) inhibitors and multi-kinase inhibitors.

In a preferred embodiment, the compounds of the invention are dosed in combination with phosphodiesterase V such as sildenafil, vardenafil and tadalafil; organic nitrates and NO donors (for example sodium nitroprusside, nitroglycerin, isosorbide mononitrate, isosorbide dinitrate, molsidomine or SIN-1 , and inhaled NO); synthetic prostaciclin analogue PGI2 such as iloprost, treprostinil, epoprostenol and beraprost; agonist of prostacyclin receptors such as selexipag and compounds of WO 2012/007539; stimulator of soluble guanylate cyclase (sGC) like riociguat and tyrosine kinase like imatinib, sorafenib and nilotinib and endothelin antagonist (for example macitentan, bosentan, sitaxentan and ambrisentan).

The dosages of the compounds of the invention depend upon a variety of factors including the particular disease to be treated, the severity of the symptoms, the route of administration, the frequency of the dosage interval, the particular compound utilized, the efficacy, toxicology profile, and pharmacokinetic profile of the compound.

The compounds of formula (I) can be administered for example, at a dosage comprised between 0.001 and 1000 mg/day, preferably between 0.1 and 500 mg/day.

When the compounds of formula (I) are administered by inhalation route, they are preferably given at a dosage comprised between 0.001 and 500 mg/day, preferably between 0.1 and 100 mg/day.

A pharmaceutical composition comprising a compound of the invention suitable to be administered by inhalation, such as inhalable powders, propellant-containing metering aerosols or propellant-free inhalable formulations.

The invention is also directed to a device comprising the pharmaceutical composition comprising a compound according to the invention , which may be selected from a single- or multi-dose dry powder inhaler (DPI), a metered dose inhaler (PMDI) or a soft mist nebulizer.

The following examples illustrate the invention in more detail.

### PREPARATIONS OF INTERMEDIATES AND EXAMPLES

### General Experimental Details

Purification by chromatography refers to purification using a CombiFlash^{®} Companion purification system or a Biotage SP1 purification system. Where products were purified using an Si cartridge, this refers to an Isolute^{®} pre-packed polypropylene column containing unbounded activated silica with irregular particles with average size of 50 µm and nominal 60Å porosity. Fractions containing the required product (identified by TLC and/or LCMS analysis) were pooled and concentrated *in vacuo.* Where an SCX-2 cartridge was used, 'SCX-2 cartridge' refers to an Isolute^{®} pre-packed polypropylene column containing a non-end-capped propylsulphonic acid functionalised silica strong cation exchange sorbent. Where HPLC was used for purification (purification by MDAP) fractions containing the required product (identified by TLC and/or LCMS analysis) were pooled and the solvent removed using a Biotage EV10 Evaporator. Alternatively the pooled product fraction was lyophilised.

NMR spectra were obtained on a Varian Unity Inova 400 spectrometer with a 5 mm inverse detection triple resonance probe operating at 400 MHz or on a Bruker Avance DRX 400 spectrometer with a 5 mm inverse detection triple resonance TXI probe operating at 400 MHz or on a Bruker Avance DPX 300 spectrometer with a standard 5 mm dual frequency probe operating at 300 MHz or on a Bruker Fourier 300 spectrometer with a 5 mm dual probe operating at 300 MHz or on Bruker AVANCE III HD 600 spectrometer with a 5mm probe operating at 600 Mhz. Shifts are given in ppm relative to tetramethylsilane.

### LCMS Method 1

Acquity UPLC (binary pump/PDA detector) + ZQ Mass Spectrometer with a C18-reverse-phase column (ACQUITY UPLC BEH C18 1.7µm, 100 × 2.1mm) maintained at 40°C, elution with A: water + 0.1% formic acid; B: MeCN + 0.1% formic acid.
Gradient:

| Gradient - Time | flow (mL/min) | %A | %B |
|---|---|---|---|
| 0.00 | 0.4 | 95 | 5 |
| 0.40 | 0.4 | 95 | 5 |
| 6.00 | 0.4 | 5 | 95 |
| 6.80 | 0.4 | 5 | 95 |
| 7.00 | 0.4 | 95 | 5 |
| 8.00 | 0.4 | 95 | 5 |

Detection - MS, UV PDA
MS ionisation method - Electrospray (positive/negative ion).

### LCMS Method 2

Acquity i-Class (quarternary pump/PDA detector) + Quattro Micro Mass Spectrometer with a C18-reverse-phase column (ACQUITY UPLC BEH C18 1.7µm, 100 × 2.1mm) maintained at 40°C, elution with A: water + 0.1% formic acid; B: MeCN + 0.1% formic acid.
Gradient:

| Gradient - Time | flow (mL/min) | %A | %B |
|---|---|---|---|
| 0.00 | 0.4 | 95 | 5 |
| 0.40 | 0.4 | 95 | 5 |
| 6.00 | 0.4 | 5 | 95 |
| 6.80 | 0.4 | 5 | 95 |
| 7.00 | 0.4 | 95 | 5 |
| 8.00 | 0.4 | 95 | 5 |

Detection - MS, UV PDA
MS ionisation method - Electrospray (positive/negative ion).

### LCMS Method 3

Acquity UPLC (binary pump/PDA detector) + ZQ Mass Spectrometer with a C18-reverse-phase column (ACQUITY UPLC BEH C18 1.7µm, 100 × 2.1mm) maintained at 40°C, elution with A: water + 0.1% ammonia; B: MeCN + 0.1% ammonia.
Gradient:

| Gradient - Time | flow (mL/min) | %A | %B |
|---|---|---|---|
| 0.00 | 0.4 | 95 | 5 |
| 0.40 | 0.4 | 95 | 5 |
| 6.00 | 0.4 | 5 | 95 |
| 6.80 | 0.4 | 5 | 95 |
| 7.00 | 0.4 | 95 | 5 |
| 8.00 | 0.4 | 95 | 5 |

Detection - MS, UV PDA
MS ionisation method - Electrospray (positive/negative ion).

### LCMS Method 4

Acquity H-Class (quaternary pump/PDA detector) + QDa Mass Spectrometer with a C18-reverse-phase column (Acquity UPLC CSH C18 1.7µm, 50 × 2.1mm) maintained at 40°C, elution with A: water + 0.1% formic acid; B: MeCN + 0.1% formic acid.
Gradient:

| Gradient - Time | flow (mL/min) | %A | %B |
|---|---|---|---|
| 0.00 | 1.0 | 97 | 3 |
| 0.15 | 1.0 | 97 | 3 |
| 4.00 | 1.0 | 01 | 99 |
| 4.4 | 1.0 | 01 | 99 |
| 4.5 | 1.0 | 97 | 03 |
| 5.0 | 1.0 | 97 | 03 |

Detection - MS, UV PDA
MS ionisation method - Electrospray (positive/negative ion).

### LCMS Method 5

Waters Acquity Classic + 996 PDA detector + Waters ZMD Mass Spectrometer with a C18-reverse-phase column (Acquity UPLC CSH C18 1.7µm, 50 × 2.1mm) maintained at 40°C, elution with A: water + 0.1% formic acid; B: MeCN + 0.1% formic acid.
Gradient:

| Gradient - Time | flow (mL/min) | %A | %B |
|---|---|---|---|
| 0.00 | 1.0 | 97 | 03 |
| 0.1 | 1.0 | 97 | 03 |
| 2.3 | 1.0 | 01 | 99 |
| 2.4 | 1.0 | 01 | 99 |
| 2.5 | 1.0 | 97 | 03 |

Detection - MS, UV PDA
MS ionisation method - Electrospray (positive/negative ion).

### LCMS Method 6

Waters Acquity Classic + 996 PDA detector + Waters QDa Mass Spectrometer (Acquity UPLC CSH C18 1.7µm, 50 × 2.1mm) maintained at 40°C, elution with A: water + 0.05% formic acid; B: MeCN + 0.05% formic acid.
Gradient:

| Gradient - Time | flow (mL/min) | %A | %B |
|---|---|---|---|
| 0.00 | 1.0 | 99 | 01 |
| 1.5 | 1.0 | 01 | 99 |
| 1.9 | 1.0 | 01 | 99 |
| 2.0 | 1.0 | 99 | 01 |

Detection - MS, UV PDA
MS ionisation method - Electrospray (positive/negative ion).

### MDAP Method (acidic)

Agilent Technologies 1260 Infinity purification system with a Phenomenex Luna PhenylHexyl Prep column (21.2 × 150 mm, 10 µm) maintained at RT

| | |
|---|---|
| Mobile Phase A: | 0.1% aqueous formic acid |
| Mobile Phase B: | 0.1% formic acid in methanol |
| Flow Rate: | 20 ml/min |
| Gradient Program: | 5%-60%, 28 min, centered around a specific focused gradient |
| Sample: | Injection of a 20-60 mg/ml solution in DMSO (+ optional formic acid and water) |

### MDAP Method (basic)

Agilent Technologies 1260 Infinity purification system with an XBridge Prep C18 OBD column (19 × 250 mm, 5 µm OBD) maintained at RT

| | |
|---|---|
| Mobile Phase A: | 0.1% aqueous ammonia |
| Mobile Phase B: | 0.1% ammonia in acetonitrile |
| Flow Rate: | 20 ml/min |
| Gradient Program: | 10%-95%, 22 min, centered around a specific focused gradient |
| Sample: | Injection of a 20-60 mg/ml solution in DMSO + optional formic acid and water) |

### Abbreviations used

- Ac: Acetyl
- BINAP: (±)-2,2'-Bis(diphenylphosphino)-1,1'-binaphthalene
- CDI: 1,1'-Carbonyldiimidazole
- COMU: (1-Cyano-2-ethoxy-2-oxoethylidenaminooxy)dimethylamino-morpholino-carbenium hexafluorophosphate
- DCM: Dichloromethane
- DIPEA: Di-isopropylethylamine
- DCE: 1,2-dichloroethane
- DMAP: 4-N,N-dimethylaminopyridine
- DME: 1,2-dimethoxyethane
- DMF: *N*,*N*-Dimethylformamide
- DMSO: Dimethylsulphoxide
- h: Hour(s)
- UPLC: Ultra high performance liquid chromatography
- HPLC: High performance liquid chromatography
- IMS: Industrial methylated spirits
- LCMS: Liquid chromatography-mass spectrometry
- MDAP: Mass-directed autopurification
- MeCN: Acetonitrile
- NIS: N-Iodosuccinimide
- Pd₂(dba)₃: Tris(dibenzylideneacetone)dipalladium(0)
- Pd(dppf)Cl₂.DCM: Bis(diphenylphosphino)ferrocene]dichloropalladium(II), complex with dichloromethane
- Rac: Racemic
- Rt: Retention time
- RT: Room temperature
- SCX: Strong cation exchange
- TBTU: N,N,N',N'-Tetramethyl-O-(benzotriazol-1-yl)uronium tetrafluoroborate
- TEA: Triethylamine
- TFA: Trifluoroacetic acid
- THF: Tetrahydrofuran
- Tosyl/Ts: 4-Toluenesulfonyl
- Xantphos: 4,5-Bis(diphenylphosphino)-9,9-dimethylxanthene
- XPhos: 2-Dicyclohexylphosphino-2',4',6'-triisopropylbiphenyl
- XPhos-Pd-G2: 2nd generation XPhos precatalyst (chloro(2-dicyclohexylphosphino-2',4',6'-triisopropyl-1,1'-biphenyl)[2-(2'-amino-1,1'-biphenyl)]palladium (II)

In the procedures that follow, some of the starting materials are identified through an "Intermediate" or "Example". This is provided merely for assistance to the skilled chemist.

When reference is made to the use of a "similar" or "analogous" procedure, as will be appreciated by those skilled in the art, such a procedure may involve minor variations, for example reaction temperature, reagent/solvent amount, reaction time, work-up conditions or chromatographic purification conditions.

The stereochemistry of the compounds in the Examples, where indicated, has been assigned on the assumption that absolute configuration at resolved stereogenic centers of starting materials is maintained throughout any subsequent reaction conditions.

### Preparation of Intermediates

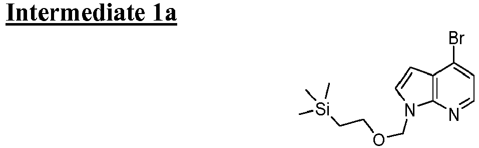

### 4-Bromo-1-((2-(trimethylsilyl)ethoxy)methyl)-1H-pyrrolo[2,3-b]pyridine

4-Bromo-7-azaindole (4.0 g, 20.30 mmol) was dissolved in DMF (37 mL) and the solution was cooled in an ice bath. Sodium hydride (60% in mineral oil, 1.14 g, 28.43 mmol) was added and the mixture was stirred under a stream of nitrogen for 1 h. 2-(Trimethylsilyl)ethoxymethyl chloride (4.0 mL, 22.74 mmol) was added dropwise and then the reaction mixture was stirred for a further 30 min. After quenching with water (20 mL), the product was extracted three times into ethyl acetate. The combined extracts were dried (Na₂SO₄) and evaporated. The residue was chromatographed on a 120 g Si cartridge eluting with 0-25% ethyl acetate in cyclohexane to give Intermediate 1a (3.78 g).
LCMS (Method 4): Rt = 1.94 min, m/z 327.2/329.1 [M+H]⁺

### Intermediates 1b to 1c

The following intermediates were prepared from the starting materials given using a procedure analogous to that used for Intermediate 1a.

| Intermediate | Structure | Starting material | LCMS |
|---|---|---|---|
| 1b | 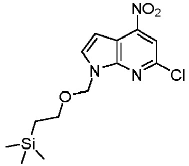 | 6-Chloro-4-nitro-1H-pyrrolo[2,3-b]pyridine | Rt = 1.76 min, m/z 328.1 [M+H]⁺ (Method 4) |
| 1c | 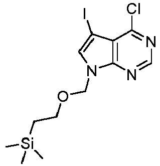 | 4-Chloro-5-iodo-7H-pyrrolo[2,3-d]pyrimidine | Rt = 1.69 min, m/z 409.8 [M+H]⁺ (Method 4) |

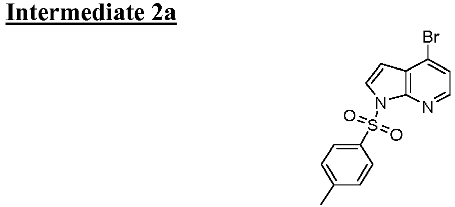

### 4-Bromo-1-tosyl-1H-pyrrolo[2,3-b]pyridine

4-Bromo-7-azaindole (5.0 g, 25.38 mmol) was dissolved in DMF (40 mL) and the solution was stirred at RT under a stream of nitrogen. Sodium hydride (60% in mineral oil, 1.50 g, 37.58 mmol) was added portion wise and the reaction mixture was stirred for 30 min. A solution of 4-toluenesulfonyl chloride (5.77 g, 30.37 mmol) in DMF (10 mL) was added dropwise over 10 min, and then the reaction mixture was stirred for a further 2 h. The reaction mixture was carefully poured into cold water (100 mL) and stirred for 30 min. The resulting precipitate was collected by filtration and dried *in vacuo.* The product was obtained as an off-white solid (9.12 g).
LCMS (Method 4): Rt = 1.57 min, m/z 350.9/352.9 [M+H]⁺

### Intermediates 2b to 2g

The following intermediates were prepared from the starting materials given using a procedure analogous to that used for Intermediate 2a.

| Intermediate | Structure | Starting material | LCMS |
|---|---|---|---|
| 2b | 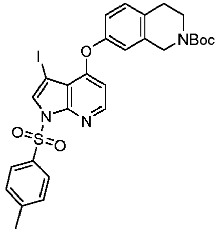 | Intermediate 7d | Rt = 1.87 min, m/z 646.2 [M+H]⁺ (Method 4) |
| 2c | 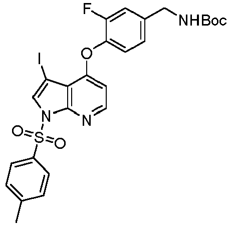 | Intermediate 7e | Rt = 1.72 min, m/z 638.2 [M+H]⁺ (Method 4) |
| 2d | 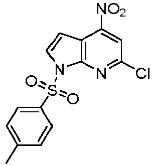 | 6-Chloro-4-nitro-1H-pyrrolo[2,3-b]pyridine | Rt = 1.58 min, m/z 352.1/354.1 [M+H]⁺ (Method 4) |
| 2e | 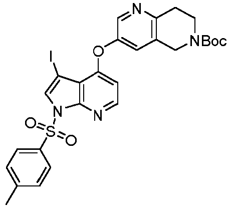 | Intermediate 7g | Rt = 1.65 min, m/z 647.2 [M+H]⁺ (Method 4) |
| 2f | 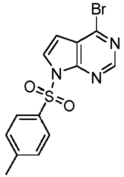 | 4-Bromo-7H-pyrrolo[2,3-d]pyrimidine | Rt = 3.75 min, m/z 351.9/353.9 [M+H]⁺ (Method 4) |
| 2g | 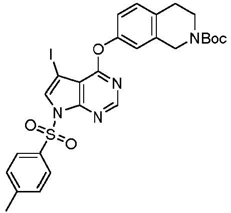 | Intermediate 7h | Rt = 1.84 min, m/z 647.0 [M+H]⁺ (Method 4) |

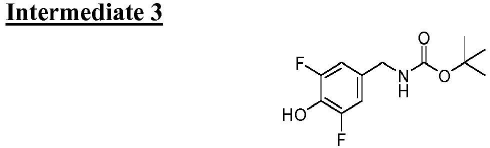

### tert-Butyl (3,5-difluoro-4-hydroxybenzyl)carbamate

4-(Aminomethyl)-2,6-difluorophenol (0.5 g, 3.14 mmol) was suspended in a mixture of DCM (10 mL) and THF (5 mL) and DIPEA (1.1 mL, 6.28 mmol) was added. The mixture was cooled in an ice bath and di-tert-butyl dicarbonate (0.76 g, 3.46 mmol) was added. The reaction mixture was allowed to warm to RT and stirred for 16 h. The solvent was evaporated *in vacuo* and the crude product was chromatographed on a 25 g Si cartridge eluting with 0-100% ethyl acetate in cyclohexane to give the title compound (0.15 g).
LCMS (Method 4): Rt = 1.45 min, m/z 260.1 [M+H]⁺

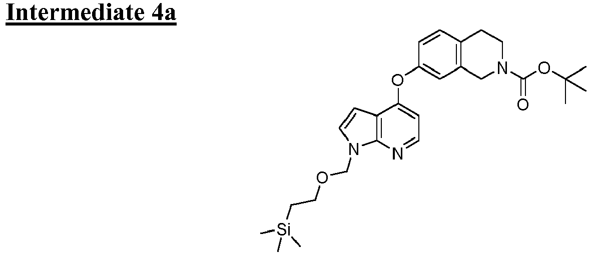

### tert-Butyl 7-((1-((2-(trimethylsilyl)ethoxy)methyl)-1H-pyrrolo[2,3-b]pyridin-4-yl)oxy)-3,4-dihydroisoquinoline-2(1H)-carboxylate

A mixture of Intermediate 1a (1.3 g, 4.0 mmol) and tert-butyl 7-hydroxy-3,4-dihydroisoquinoline-2(1H)-carboxylate (1.09 g, 4.4 mmol), Pd₂(dba)₃ (0.183 g, 0.2 mmol), XPhos (0.19 g, 0.4 mmol), and potassium carbonate (1.21 g, 8.8 mmol) in toluene (50 mL) was sonicated for 5 min under a blanket of argon. The mixture was heated at 100°C for 16 h, and then allowed to cool to RT before filtering through Celite^{®}. The solvent was evaporated and the residue was taken up into water (25 mL) and extracted with ethyl acetate (3 × 50 mL). The combined organic extracts were washed with brine (20 mL), dried (Na₂SO₄) and evaporated. The crude product was chromatographed on an Si cartridge (40 g) eluting with 0-50% ethyl acetate in cyclohexane to give the title compound (1.66 g).
LCMS (Method 4): Rt = 1.97 min, m/z 496.4 [M+H]⁺

### Intermediate 4b to 4f

The following intermediates were prepared from the starting materials given using a procedure analogous to that used for Intermediate 4a.

| Intermediate | Structure | Starting material | LCMS |
|---|---|---|---|
| 4b | 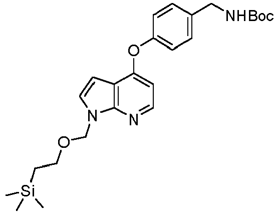 | Intermediate 1a and tert-butyl (4-hydroxybenzyl)-carbamate | Rt = 1.82 min, m/z 470.3 [M+H]⁺ (Method 4) |
| 4c | 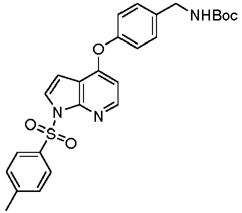 | Intermediate 2a and tert-butyl (4-hydroxybenzyl)-carbamate | Rt = 1.70 min, m/z 494.2 [M+H]⁺ (Method 4) |
| 4d | 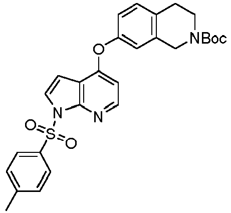 | Intermediate 2a and tert-butyl 7-hydroxy-3,4-dihydroisoquinoline-2(1H)-carboxylate | Rt = 1.79 min, m/z 520.3 [M+H]⁺ (Method 4) |
| 4e | 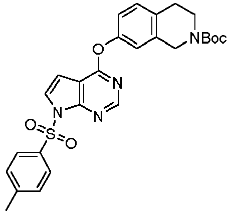 | Intermediate 2f and tert-butyl 7-hydroxy-3,4-di-hydroisoquinoline-2(1H)-carboxylate | Rt = 1.71 min, m/z 521.0 [M+H]⁺ (Method 4) |
| 4f | 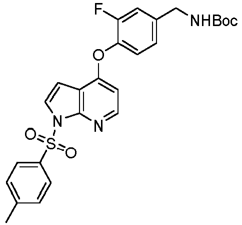 | Intermediate 2a and tert-butyl (3-fluoro-4-hydroxybenzyl)-carbamate | Rt = 1.63 min, m/z 512.2 [M+H]⁺ (Method 4) |

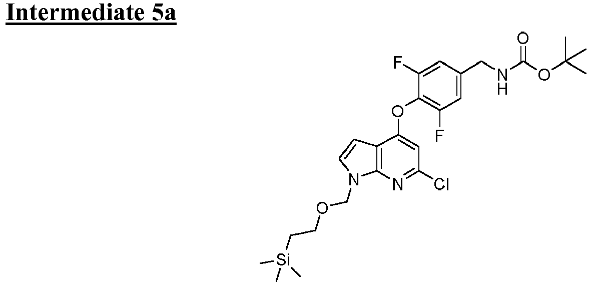

### tert-Butyl (4-((6-chloro-1-((2-(trimethylsilyl)ethoxy)methyl)-1H-pyrrolo[2,3-b]-pyridin-4-yl)oxy)-3,5-difluorobenzyl)carbamate

Intermediate 1b (107 mg, 0.326 mmol), Intermediate 3 (129 mg, 0.49 mmol) and potassium carbonate (140 mg, 0.979 mmol) were heated at 120°C in DMSO (3 mL) for 2 h. The reaction mixture was allowed to cool and then poured into water (10 mL). The product was extracted into ethyl acetate (3 × 20 mL) and the combined extracts were dried (Na₂SO₄) and evaporated. The residue was purified by chromatography on a 12 g Si cartridge eluting with 0-40% ethyl acetate in cyclohexane. The crude product was an off-white solid (240 mg).
LCMS (Method 4): Rt = 1.81 min, m/z 540.3/542.3 [M+H]⁺

### Intermediate 5b

Using similar conditions to those used for the synthesis of Intermediate 5a, Intermediate 5b was prepared from the starting materials indicated. The basic reaction conditions resulted in tosyl deprotection.

| Intermediate | Structure | Starting materials | LCMS |
|---|---|---|---|
| 5b | 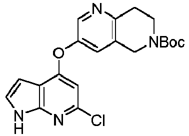 | Intermediates 2d and tert-butyl 3-hydroxy-7,8-dihydro-1,6-naphthyridine-6(5H)-carboxylate | Rt = 1.34 min, m/z 401.2/403.2 [M+H]⁺ (Method 4) |

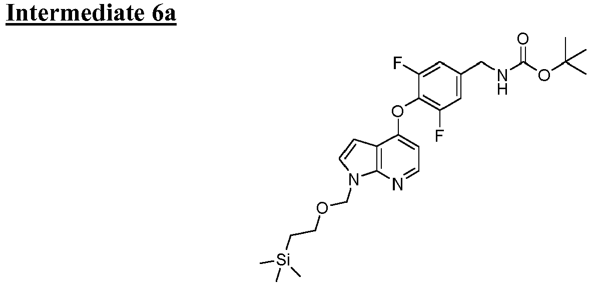

### tert-Butyl (3,5-difluoro-4-((1-((2-(trimethylsilyl)ethoxy)methyl)-1H-pyrrolo-[2,3-b]pyridin-4-yl)oxy)benzyl)carbamate

A solution of Intermediate 5a (240 mg, 0.45 mmol) and triethylamine (60 µL, 0.535 mmol) in IMS (20 mL) was stirred over 10% palladium on carbon (24 mg) under a blanket of hydrogen gas. After 18 h at RT the mixture was filtered through Celite^{®} and the solvent was evaporated to give the title compound (170 mg).
LCMS (Method 4): Rt = 1.80 min, m/z 506.3 [M+H]⁺

### Intermediate 6b

Using similar conditions to those used for the synthesis of Intermediate 6a, Intermediate 6b was prepared from the starting materials indicated.

| Intermediate | Structure | Starting materials | LCMS |
|---|---|---|---|
| 6b | 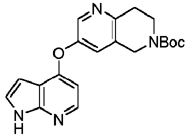 | Intermediate 5b | Rt = 1.02 min, m/z 367.3 [M+H]⁺ (Method 4) |

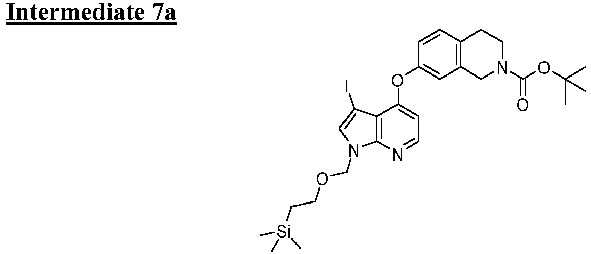

### tert-Butyl 7-((3-iodo-1-((2-(trimethylsilyl)ethoxy)methyl)-1H-pyrrolo[2,3-b]-pyridin-4-yl)oxy)-3,4-dihydroisoquinoline-2(1H)-carboxylate

Intermediate 4a (1.66 g, 3.35 mmol) was dissolved in acetonitrile (35 mL) and the solution was stirred at 0°C. NIS (0.79 g, 3.52 mmol) was added and the reaction mixture was stirred for 30 min at 0°C. Stirring was continued at RT for 1 h and then 1M sodium thiosulfate (15 mL) was added. The product was extracted into ethyl acetate (3 × 10 mL) and the combined extracts were dried (Na₂SO₄) and evaporated. The residue was chromatographed on a 40 g Si cartridge eluting with 0-50% ethyl acetate in cyclohexane to give the title compound (1.0 g).
LCMS (Method 4): Rt = 2.02 min, m/z 622.3 [M+H]⁺

### Intermediates 7b to 7h

The following intermediates were prepared from the starting materials given using a procedure analogous to that used for Intermediate 7a.

| Intermediate | Structure | Starting material | LCMS |
|---|---|---|---|
| 7b | 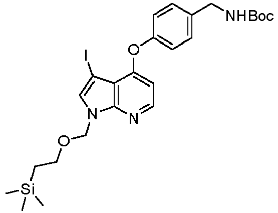 | Intermediate 4b | Rt = 1.92 min, m/z 596.2 [M+H]⁺ (Method 4) |
| 7c | 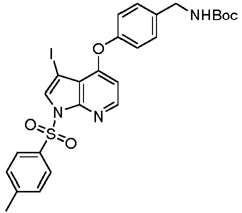 | Intermediate 4c | Rt = 1.67 min, m/z 620.2 [M+H]⁺ (Method 4) |
| 7d | 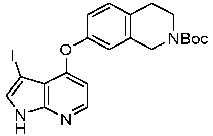 | Intermediate 35c | Rt = 1.59 min, m/z 492.1 [M+H]⁺ (Method 4) |
| 7e | 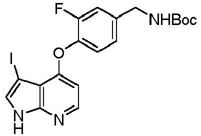 | Intermediate 35b | Rt = 1.44 min, m/z 484.1 [M+H]⁺ (Method 4) |
| 7f | 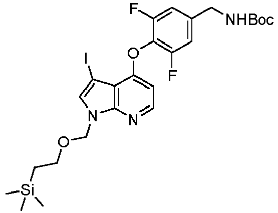 | Intermediate 6a | Rt = 1.84 min, m/z 632.0 [M+H]⁺ (Method 4) |
| 7g | 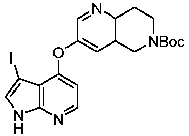 | Intermediate 6b | Rt = 1.28 min, m/z 493.1 [M+H]⁺ (Method 4) |
| 7h | 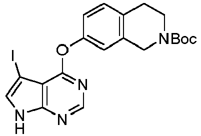 | Intermediate 35d | Rt = 1.59 min, m/z 492.9 [M+H]⁺ (Method 4) |

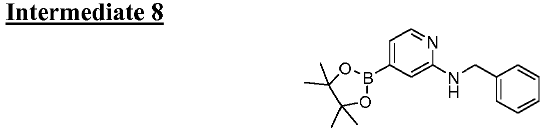

### N-Benzyl-4-(4,4,5,5-tetramethyl-1,3,2-dioxaborolan-2-yl)pyridin-2-amine

N-Benzyl-4-bromopyridin-2-amine (300 mg, 1.14 mmol), bis(pinacolato)diboron (434 mg, 1.71 mmol), potassium acetate (336 mg, 3.42 mmol) and Pd(dppf)Cl₂.DCM (93 mg, 0.11 mmol) were added together in dioxane (12 mL) in a 20 mL microwave vial. The vial was sealed and the reaction mixture was sonicated and degassed with argon for 5 min and then heated at 100 °C for 3 h. The reaction mixture was allowed to cool to RT, diluted with ethyl acetate and filtered through Celite^{®}. The filtrate was evaporated under reduced pressure to give Intermediate 8 which was used without further purification in the next steps (754 mg).

LCMS (Method 4): Rt = 0.61 min, m/z 229.1 [M+H]⁺ (assumed conversion to boronic acid under LC conditions)

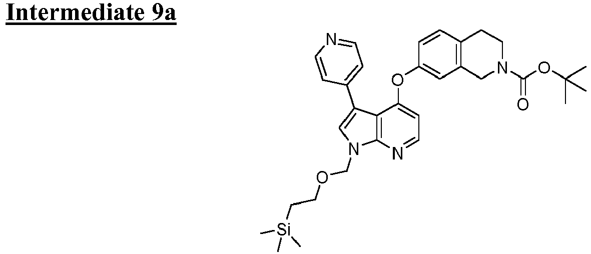

### tert-Butyl 7-((3-(pyridin-4-yl)-1-((2-(trimethylsilyl)ethoxy)methyl)-1H-pyrrolo-[2,3-b]pyridin-4-yl)oxy)-3,4-dihydroisoquinoline-2(1H)-carboxylate

Intermediate 7a (1.0 g, 1.61 mmol), pyridine-4-boronic acid (600 mg, 3.22 mmol), Pd(dppf)Cl₂.DCM (66 mg, 0.08 mmol), and cesium carbonate (1.14g, 3.54 mmol) in a mixture of DME (10 mL) and water (1 mL) was bubbled with argon for 5 min. The mixture was stirred at 140°C for 7 h and then allowed to cool to RT. The mixture was filtered through Celite^{®}. The filtrate was evaporated to dryness and the residue was taken up into water (20 mL) and extracted with ethyl acetate (3 × 40 mL). The combined organic extracts were washed with brine (20 mL), dried (Na₂SO₄) and evaporated. The crude product was chromatographed on an Si cartridge (40 g) eluting with 0-50% ethyl acetate in cyclohexane. The product was obtained as yellow oil (680 mg).
LCMS (Method 4): Rt = 1.28 min, m/z 573.5 [M+H]⁺

### Intermediates 9b to 9n

The following intermediates were prepared from the starting materials given using a procedure analogous to that used for Intermediate 9a.

| Intermediate | Structure | Starting material | LCMS |
|---|---|---|---|
| 9b | 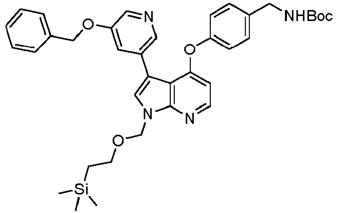 | 3-(benzyloxy)-5-(4,4,5,5-tetramethyl-1,3,2-dioxaborolan-2-yl)pyridine and Intermediate 7b | Rt = 1.54 min, m/z 653.4 [M+H]⁺ (Method 6) |
| 9c | 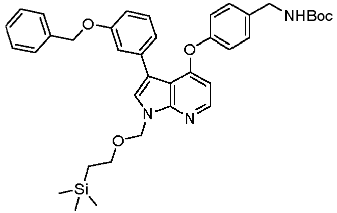 | (3-(Benzyloxy)-phenyl)boronic acid and Intermediate 7b | Rt = 2.01 min, m/z 652.4 [M+H]⁺ (Method 4) |
| 9d | 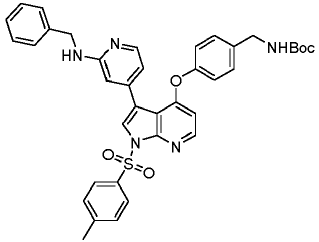 | Intermediates 8 and 7c | Rt = 1.28 min, m/z 676.4 [M+H]⁺ (Method 4) |
| 9e | 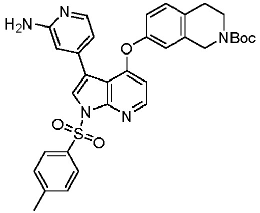 | 4-(4,4,5,5-Tetramethyl-1,3,2-dioxaborolan-2-yl)pyridin-2-amine and Intermediate 2b | Rt = 1.15 min, m/z 612.3 [M+H]⁺ (Method 4) |
| 9f | 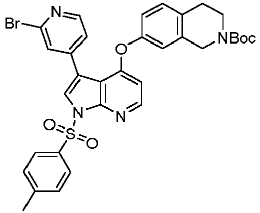 | 2-Bromo-4-(4,4,5,5-tetramethyl-1,3,2-dioxaborolan-2-yl)pyridine and Intermediate 2b | Rt = 1.89 min, m/z 675.0/676.9 [M+H]⁺ (Method 4) |
| 9g | 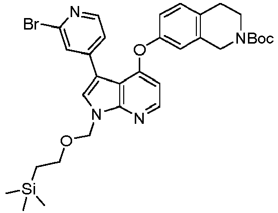 | 4-(4,4,5,5-Tetramethyl-1,3,2-dioxaborolan-2-yl)pyridin-2-amine and Intermediate 7a | Rt = 2.03 min, m/z 651.1/652.9 [M+H]⁺ (Method 4) |
| 9h | 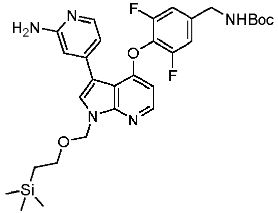 | 4-(4,4,5,5-Tetramethyl-1,3,2-dioxaborolan-2-yl)pyridin-2-amine and Intermediate 7f | Rt = 1.27 min, m/z 598.1 [M+H]⁺ (Method 4) |
| 9i | 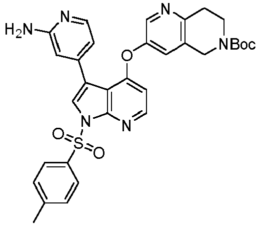 | 4-(4,4,5,5-Tetramethyl-1,3,2-dioxaborolan-2-yl)pyridin-2-amine and Intermediate 2e | Rt = 1.05 min, m/z 613.3 [M+H]⁺ (Method 4) |
| 9j | 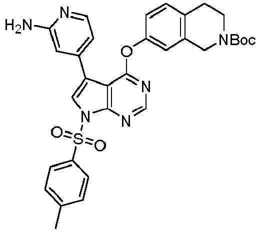 | 4-(4,4,5,5-Tetramethyl-1,3-dioxolan-2-yl)pyridin-2-amine and Intermediate 2g | Rt = 1.18 min, m/z 613.1 [M+H]⁺ (Method 4) |
| 9k | 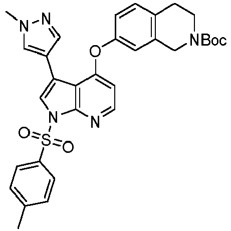 | (1-Methyl- 1H-pyrazol-4-yl)boronic acid and Intermediate 2b | Rt = 1.62 min, m/z 600.3 [M+H]⁺ (Method 4) |
| 91 | 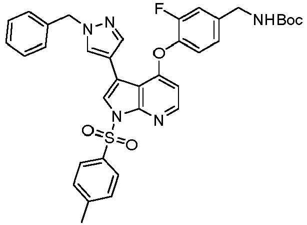 | 1-Benzyl-4-(4,4,5,5-tetramethyl-1,3,2-dioxaborolan-2-yl)-1H-pyrazole and Intermediate 2c | Rt = 1.62 min, m/z 668.3 [M+H]⁺ (Method 4) |
| 9m | 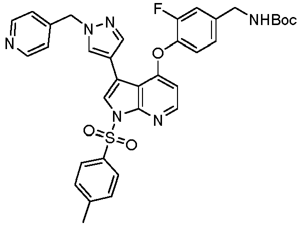 | 4-(2-(4-(4,4,5,5-Tetramethyl-1,3,2-dioxaborolan-2-yl)-1H-pyrazol-1-yl)methyl)pyridine and Intermediate 2c | Rt = 1.31 min, m/z 669.3 [M+H]⁺ (Method 4) |
| 9n | 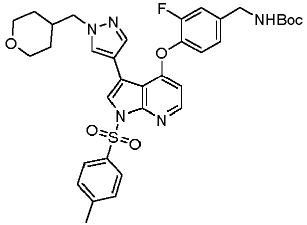 | 1-((Tetrahydro-2H-pyran-4-yl)methyl)-4-(4,4,5,5-tetramethyl-1,3,2-dioxaborolan-2-yl)-1H-pyrazole and Intermediate 2c | Rt = 1.56 min, m/z 676.4 [M+H]⁺ (Method 4) |

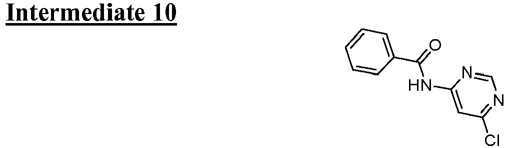

### N-(6-Chloropyrimidin-4-yl)benzamide

To a solution of 6-chloropyrimidin-4-amine (0.75 g, 5.79 mmol), benzoic acid (0.78 g, 6.37 mmol) in trimethylamine (2.05 mL, 20.3 mmol) and THF (10 mL) was added 1-(methylsulfonyl)-1H-benzo[d][1,2,3]triazole (1.26 g, 6.37 mmol) and the reaction mixture was stirred for 6 h at 140°C under microwave irradiation. After quenching with water (50 mL), the product was extracted into ethyl acetate (3 × 50 mL). The combined extracts were dried (Na₂SO₄) and evaporated. The residue was triturated with diethyl ether and the solid formed was collected by filtration and dried *in vacuo* to give the desired product (0.55 g).
LCMS (Method 4): Rt = 1.27 min, m/z 234.1/236.1 [M+H]⁺

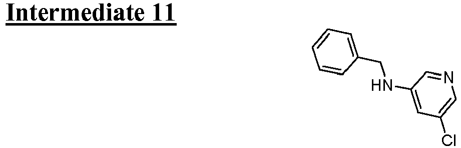

### N-Benzyl-5-chloropyridin-3-amine

To a solution of benzylamine (0.57 mL, 5.2 mmol), tris(dibenzylideneacetone)-dipalladium(0) (0.095 g, 0.104 mmol) and rac-BINAP (0.16 g, 0.260 mmol) in toluene (20 mL) were added sodium tert-butoxide (1.00 g, 10.39 mmol) and 3-bromo-5-chloropyridine (1 g, 5.2 mmol). The reaction mixture was stirred at 85°C for 18 h and then diluted with ethyl acetate (50 mL) The mixture was filtered through Celite^{®}, washed with 0.1 N HCl aqueous (20 mL) and brine (20 mL), dried (MgSO₄) and evaporated to give the desired product (0.705 g).
LCMS (Method 4): Rt = 1.55 min, m/z 219.0/221.0 [M+H]⁺

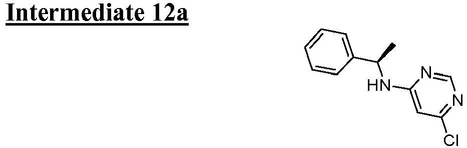

### (R)-6-Chloro-N-(1-phenylethyl)pyrimidin-4-amine

A solution of 4,6-dichloropyrimidine (1 g, 6.71 mmol), (R)-1-phenylethan-1-amine (0.94 mL, 7.38 mmol) and triethylamine (1.03 mL, 7.38 mmol) in IMS (17 mL) was stirred for 3 h at 85°C. The reaction mixture was concentrated *in vacuo.* The residue was diluted with ethyl acetate (50 mL). The solution was washed with water (50 mL). The organic layer was dried (Na₂SO₄) and evaporated to give the desired product (1.60 g).
LCMS (Method 4): Rt = 1.33 min, m/z 234.1/236.1 [M+H]⁺

### Intermediates 12b to 12g

The following aryl chlorides were prepared from 4,6-dichloropyrimidine and the given amine using similar methodology to that used for Intermediate 12a.

| Intermediate | Structure | Amine | LCMS |
|---|---|---|---|
| 12b | 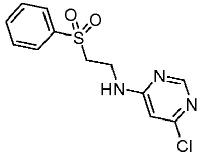 | 2-(Phenylsulfonyl)-ethan-1-amine | Rt = 1.04 min, m/z 297.9/299.7 [M+H]⁺ (Method 4) |
| 12c | 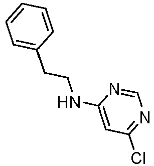 | Phenethylamine | Rt = 1.26 min, m/z 234.1/236.1 [M+H]⁺ (Method 4) |
| 12d | 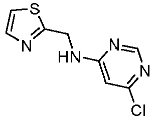 | Thiazol-2-ylmethanamine | Rt = 0.85 min, m/z 226.9/229.0 [M+H]⁺ (Method 4) |
| 12e | 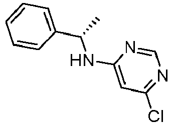 | (S)-1-Phenylethan-1-amine | Rt = 1.24 min, m/z 234.1/236.1 [M+H]⁺ (Method 4) |
| 12f | 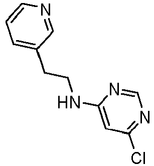 | 2-(Pyridin-3-yl)ethan-1-amine | Rt = 0.59 min, m/z 234.9/236.7 [M+H]⁺ (Method 4) |
| 12g | 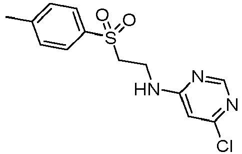 | 2-Tosylethan-1-amine hydrochloride | Rt = 1.08 min, m/z 311.9/313.7 [M+H]⁺ (Method 4) |

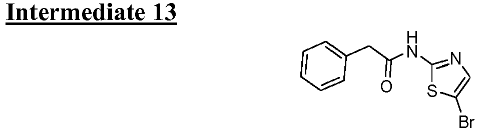

### N-(5-Bromothiazol-2-yl)-2-phenylacetamide

A solution of 2-amino-5-bromothiazole hydrobromide (1.04 g, 4.00 mmol) and triethylamine (1.7 mL, 12.0 mmol) in THF (6.8 mL) was cooled in an ice bath under nitrogen and 2-phenylacetyl chloride (0.87 g, 5.6 mmol) was added. The reaction mixture was allowed to warm to RT and then stirred for 18 h. Water (15 mL) was added and the product was extracted into ethyl acetate (2 × 15 mL). The combined organic extracts were dried (Na₂SO₄) and evaporated. The residue was treated with DCM and an off-white solid was filtered off and dried under vacuum at 50°C for 1 h. The filtrate was evaporated and the residue chromatographed on an 80 g Si cartridge eluting with 0-70% ethyl acetate in isohexane to afford the desired product (299 mg).
LCMS (Method 4): Rt = 1.34 min, m/z 297.0/299.0 [M+H]⁺

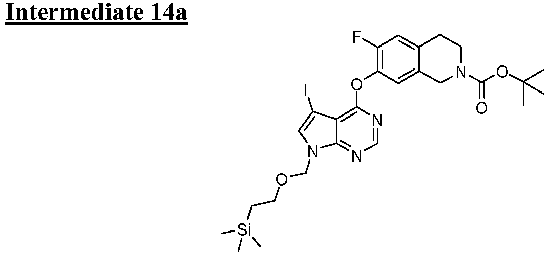

### tert-Butyl 6-fluoro-7-((5-iodo-7-((2-(trimethylsilyl)ethoxy)methyl)-7H-pyrrolo[2,3-d]pyrimidin-4-yl)oxy)-3,4-dihydroisoqiuinoline-2(1H)-carboxylate

A mixture of Intermediate 1c (426 mg, 1.04 mmol), tert-butyl 6-fluoro-7-hydroxy-3,4-dihydroisoquinoline-2(1H)-carboxylate (317 mg, 1.19 mmol) and potassium carbonate (288 mg, 2.08 mmol) in DMSO (4 mL) was heated at 85°C for 1.25 h. The reaction mixture was partitioned between water and ethyl acetate and the phases separated. The organics were dried (Na₂SO₄), filtered and evaporated. The crude material was purified on a 40 g Si cartridge eluting with 0-5% ethyl acetate in DCM to give the title compound (348 mg).
LCMS (Method 4) 1.91 mins, m/z 641.0 [M+H]⁺

### Intermediate 14b

Intermediate 14b was prepared in a manner similar to Intermediate 14a from the starting materials given.

| Intermediate | Structure | Starting materials | LCMS |
|---|---|---|---|
| 14b | 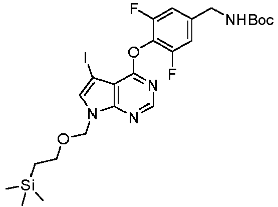 | Intermediates 1c and 3 | Rt = 1.83 min, m/z 633.0 [M+H]⁺ (Method 4) |

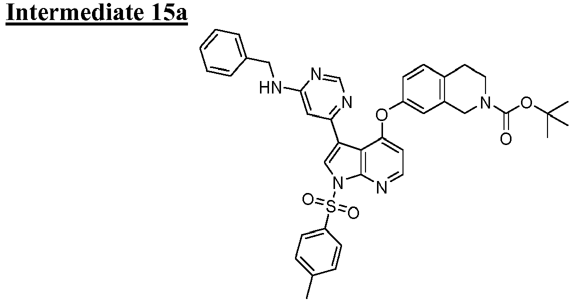

### tert-Butyl 7-((3-(6-(benzylamino)pyrimidin-4-yl)-1-tosyl-1H-pyrrolo[2,3-b]-pyridin-4-yl)oxy)-3,4-dihydroisoquinoline-2(1H)-carboxylate

Intermediate 2b (224 mg, 0.35 mmol), XPhos-Pd-G2 (5.5 mg, 0.007 mmol), XPhos (6.6 mg, 0.014 mmol), tetrahydroxydiboron (93 mg, 1.04 mmol) and potassium acetate (102 mg, 1.04 mmol) were added in IMS (3.5 mL) to a 20 mL microwave vial. The vial was sealed and the mixture was sonicated and degassed with argon for 5 min and then heated at 80°C for 2 h. A degassed solution of aqueous potassium carbonate (1.8 M, 0.58 mL) followed by a degassed solution of N-benzyl-6-chloropyrimidin-4-amine (76 mg, 0.35 mmol) in THF (1.1 mL) were added and the resulting mixture was heated at 80°C for 1.5 h. The reaction mixture was allowed to cool to RT, diluted with water (10 mL) and extracted with ethyl acetate (3 × 10 mL). The ethyl acetate layers were separated, combined, dried (Na₂SO₄) and evaporated under reduced pressure. The residue was chromatographed on a 25 g Si cartridge eluting with 0-100% ethyl acetate in iso-hexane to give Intermediate 15a (59 mg).
LCMS (Method 4): Rt = 1.48 min, m/z 703.4 [M+H]⁺

### Intermediates 15b to 15s

The following intermediates were prepared from the indicated starting materials using a method similar to that used for Intermediate 15a.

| Intermediate | Structure | Starting materials | LCMS |
|---|---|---|---|
| 15b | 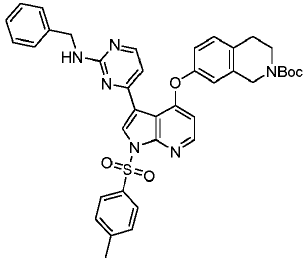 | N-Benzyl-4-chloropyrimidin - 2-amine and Intermediate 2b | Rt = 1.83 min, m/z 703.4 [M+H]⁺ (Method 4) |
| 15c | 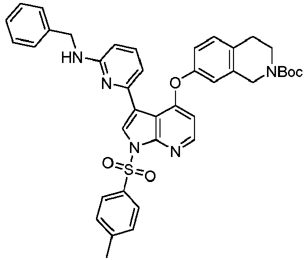 | N-Benzyl-6-chloropyridin-2-amine and Intermediate 2b | Rt = 1.61 min, m/z 702.4 [M+H]⁺ (Method 4) |
| 15d | 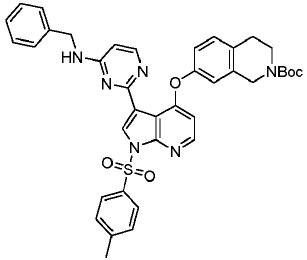 | N-Benzyl-2-chloropyrimidin - 4-amine and Intermediate 2b | Rt = 1.39 min, m/z 703.4 [M+H]⁺ (Method 4) |
| 15e | 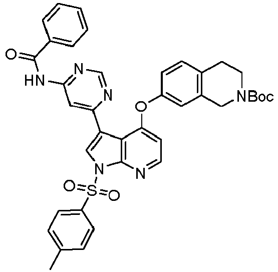 | Intermediates 10 and 2b | Rt = 1.83 min, m/z 717.4 [M+H]⁺ LCMS (Method 4): |
| 15f | 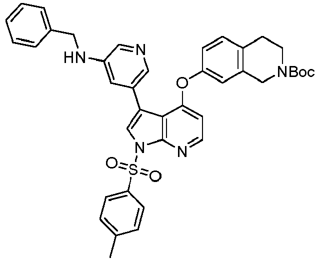 | Intermediates 11 and 2b | Rt = 1.64 min, m/z 702.0 [M+H]⁺ (Method 5) |
| 15g | 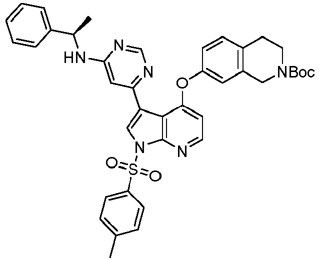 | Intermediates 12a and 2b | Rt = 1.52 min, m/z 717.4 [M+H]⁺ (Method 4) |
| 15h | 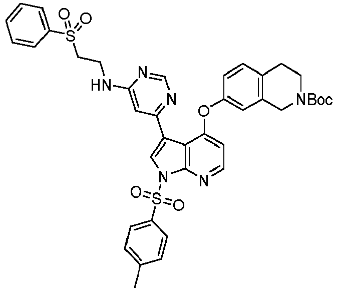 | Intermediates 12b and 2b | Rt = 1.48 min, m/z 781.1 [M+H]⁺ (Method 4) |
| 15i | 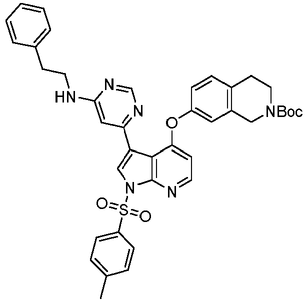 | Intermediates 12c and 2b | Rt = 1.49 min, m/z 717.4 [M+H]⁺ (Method 4) |
| 15j | 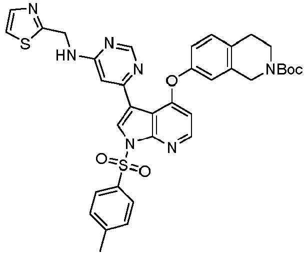 | Intermediates 12d and 2b | Rt = 2.02 min, m/z 710.3 [M+H]⁺ (Method 4) |
| 15k | 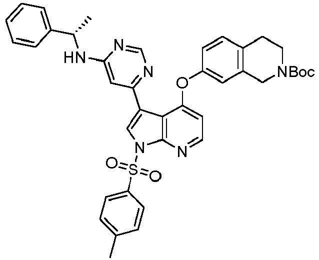 | Intermediates 12e and 2b | Rt = 1.99 min, m/z 717.2 [M+H]⁺ (Method 4) |
| 151 | 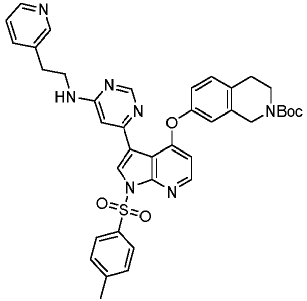 | Intermediates 12f and 2b | Rt = 1.27 min, m/z 718.2 [M+H]⁺ (Method 4) |
| 15m | 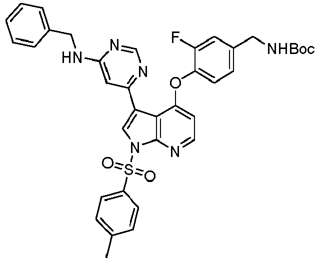 | N-Benzyl-6-chloropyrimidin - 4-amine and Intermediate 2c | Rt = 1.37 min, m/z 695.4 [M+H]⁺ (Method 4) |
| 15n | 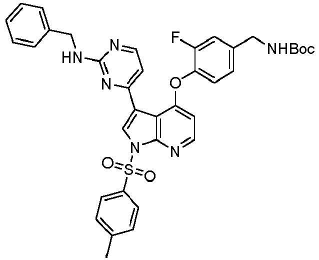 | N-Benzyl-4-chloropyrimidin - 2-amine and Intermediate 2c | Rt = 1.77 min, m/z 695.4 [M+H]⁺ (Method 4) |
| 15o | 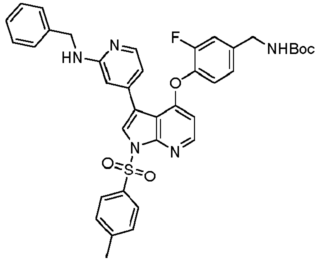 | N-Benzyl-4-bromopyridin-2-amine and Intermediate 2c | Rt = 1.33 min, m/z 694.4 [M+H]⁺ (Method 4) |
| 15p | 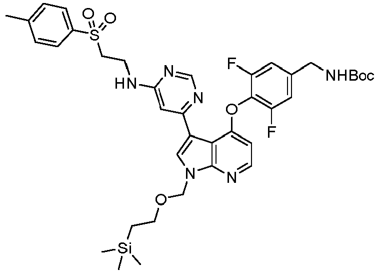 | Intermediates 12g and 7f | Rt = 1.37 min, m/z 781.1 [M+H]⁺ (Method 4) |
| 15q | 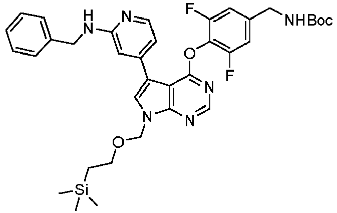 | N-Benzyl-4-bromopyridin-2-amine and Intermediate 14b | Rt = 1.35 min, m/z 689.1 [M+H]⁺ (Method 4) |
| 15r | 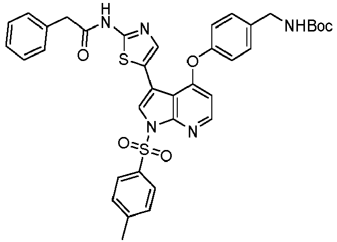 | Intermediates 13 and 7c | Rt = 1.69 min, m/z 710.3 [M+H]⁺ (Method 4) |
| 15s | 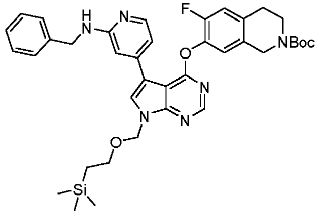 | N-Benzyl-4-bromopyridin-2-amine and Intermediate 14a | Rt = 1.41 min, m/z 697.3 [M+H]⁺ (Method 4) |

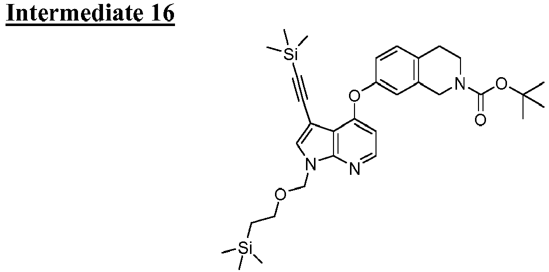

### tert-Butyl 7-((1-((2-(trimethylsilyl)ethoxy)methyl)-3-((trimethylsilyl)ethynyl)-1H-pyrrolo[2,3-b]pyridin-4-yl)oxy)-3,4-dihydroisoquinoline-2(1H)-carboxylate

A stirred mixture of Intermediate 7a (932 mg, 1.5 mmol) ethynyl trimethylsilane (295 mg, 0,42 mL, 3mmol), diisopropyl amine (379 mg, 0.53 mL, 3.75 mmol) and DCM (9.0 mL) was degassed by purging with argon. Copper (I) iodide (57 mg, 0.3mmol) then bis(triphenylphosphine)palladium (II) dichloride (53 mg, 0.075mmol) were added and purging was stopped after a further 1 min. As the mixture darkened, stirring was continued at ambient temperature under argon. After 1 h the dark mixture was filtered through a short pad of Celite^{®}. The pad was washed with DCM and the combined filtrates were concentrated *in vacuo.* The residue was purified on a 20 g Si cartridge eluting with 0-8% ethyl acetate in DCM to afford the desired product (0.78 g).
LCMS (Method 4): Rt = 1.96 min, m/z 592.5 [M+H]⁺

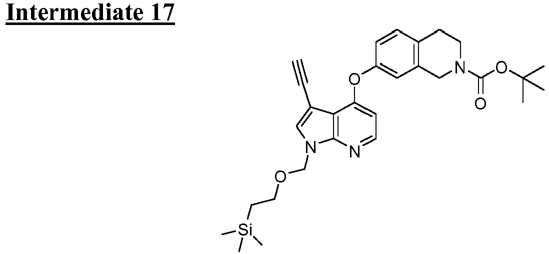

### tert-Butyl 7-((3-ethynyl-1-((2-(trimethylsilyl)ethoxy)methyl)-1H-pyrrolo[2,3-b]pyridin-4-yl)oxy)-3,4-dihydroisoquinoline-2(1H)-carboxylate

A mixture of potassium carbonate (138 mg, 1.0 mmol), Intermediate 16 (770 mg, 1.3 mmol) and methanol (15 mL) was stirred at ambient temperature for 1.25 h. The mixture was then concentrated *in vacuo* to a small volume (ca 3 mL) and diluted with DCM (15 mL). The mixture was filtered through a pad of Celite^{®} and the pad was washed with DCM. The combined filtrate was concentrated *in vacuo.* The residue was purified on a 10 g Si cartridge eluting with 0-8% ethyl acetate in DCM to afford the desired product (570 mg).
LCMS (Method 4): Rt = 1.80 min, m/z 520.5 [M+H]⁺

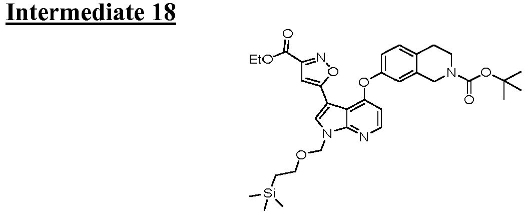

### Ethyl 5-(4-((2-(tert-butoxycarbonyl)-1,2,3,4-tetrahydroisociuinolin-7-yl)oxy)-1-((2-(trimethylsilyl)ethoxy)methyl)-1H-pyrrolo[2,3-b]pyridin-3-yl)isoxazole-3-carboxylate

Triethylamine (220 µL,1.59 mmol) was added in four equal portions to a solution of Intermediate 17 (330 mg, 0.64 mmol) and ethyl (Z)-2-chloro-2-(hydroxyimino)acetate (212 mg, 1.4 mmol) in THF (2 mL). The mixture was stirred for a further 5.5 h then further aliquots of ethyl (Z)-2-chloro-2-(hydroxyimino)acetate (53 mg, 0.35 mmol) and triethylamine (55 µL, 0.40 mmol) were added. The mixture was stirred for a further 16 h and was then diluted with DCM (10 mL) and water (5 mL). The phases were separated. The aqueous phase was washed with DCM (2 × 10 mL). The combined organic extracts were dried (Na₂SO₄) and concentrated *in vacuo.* The residue was purified on a 5 g Si cartridge eluting with 0-12% ethyl acetate in DCM to afford the desired product (280 mg).
LCMS (Method 4): Rt = 2.03 min, m/z 635.4 [M+H]⁺

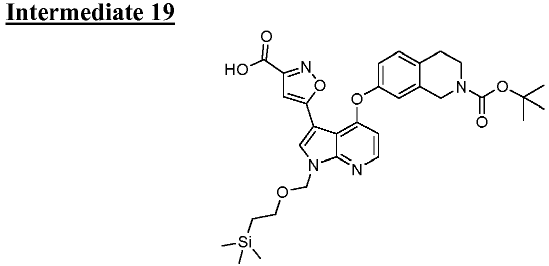

### 5-(4-((2-(tert-Butoxycarbonyl)-1,2,3,4-tetrahydroisoquinolin-7-yl)oxy)-1-((2-(trimethylsilyl)ethoxy)methyl)-1H-pyrrolo[2,3-b]pyridin-3-yl)isoxazole-3-carboxylic acid

Lithium hydroxide monohydrate (23 mg, 0.55 mmol) was added to a mixture of Intermediate 18 (280 mg, 0.44 mmol), THF (5 mL) and water (1 mL). The mixture was stirred at room temperature for 2 h then treated with a solution of potassium hydrogen sulfate (70 mg) in water (10 ml). The mixture was extracted with ethyl acetate (3 × 10 mL). The combined organic phase was dried (Na₂SO₄) and concentrated *in vacuo* to afford the desired product as a solid (260 mg).
LCMS (Method 4): Rt = 1.83 min, m/z 607.5 [M+H]⁺

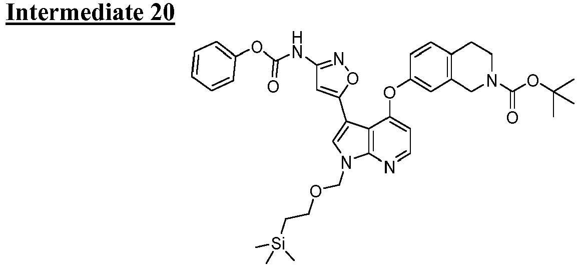

### tert-Butvl 7-((3-(3-((phenoxycarbony)amino)isoxazol-5-yl)-1-((2-(trimethylsilyl)ethoxy)methyl)-1H-pyrrolo[2,3-b]pyridin-4-yl)oxy)-3,4-dihydroisoquinoline-2(1H)-carboxylate

Diphenylphosphoryl azide (0.11 mL, 0.49 mmol) was added to a solution of Intermediate 19 (250 mg, 0.41 mmol) and triethylamine (0.07 mL, 0.49 mmol) in toluene (5 mL). The mixture was stirred at ambient temperature for 2 h then at 35°C for 45 min. Phenol (65 mg, 0.69 mmol) was added and the mixture was then heated at 80°C for 2 h. The cold mixture was diluted with ethyl acetate (15 mL) and washed with saturated aqueous sodium carbonate (10 mL). The aqueous phase was back-washed with ethyl acetate (2 × 10 mL). The combined organic phase was dried (MgSO₄) and concentrated *in vacuo* to afford the crude product. This was purified by chromatography on a 5 g Si cartridge eluting with 0-20% ethyl acetate in DCM to afford the title compound as a solid (80 mg).
LCMS (Method 4): Rt=1.92 min, m/z 698.4 [M+H]⁺

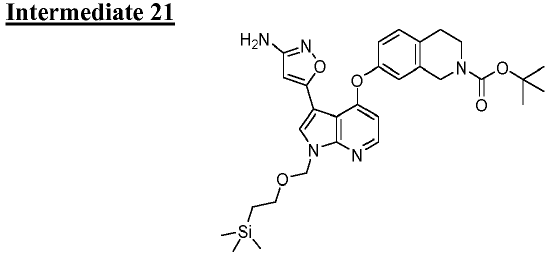

### tert-Butyl 7-((3-(3-aminoisoxazol-5-yl)-1-((2-(trimethylsilyl)ethoxy)methyl)-1H-pyrrolo[2,3-b]pyridin-4-yl)oxy)-3,4-dihydroisoqluinoline-2(1H)-carboxylate

A mixture of Intermediate 20 (80 mg, 0.115 mmol), potassium hydroxide (95 mg, 1.69 mmol) THF (4.0 mL) and water (4.0 mL) was stirred until a homogeneous solution was obtained. This solution was allowed to stand for 16 h then it was diluted with water (10 mL) and extracted with ethyl acetate (2 × 10 mL). The aqueous phase was neutralised with 5% aqueous potassium hydrogen sulfate solution and extracted with ethyl acetate (10 mL). The combined organic phase was dried (MgSO₄) and concentrated *in vacuo* to afford the crude product. This was purified by chromatography on a Si cartridge (2 g) eluting with 0-40% ethyl acetate in DCM to give the desired product (55 mg).
LCMS (Method 4): Rt = 1.71 min, m/z 578.4 [M+H]⁺

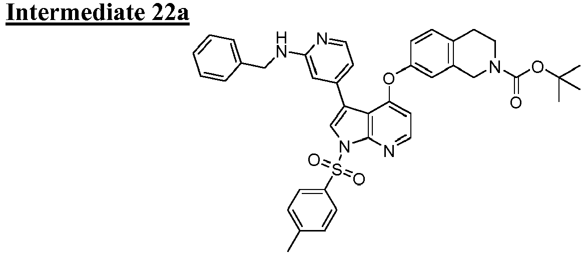

### tert-Butyl 7-((3-(2-(benzylamino)pyridin-4-yl)-1-tosyl-1H-pyrrolo[2,3-b]pyridin-4-yl)oxy)-3,4-dihydroisoquinoline-2(1H)-carboxylate

Acetic acid (0.060 mL, 1.05 mmol) was added to a solution of Intermediate 9e (165 mg, 0.27 mmol), benzaldehyde (0.041 ml, 0.41 mmol) and 4Å molecular sieves (63 mg) in 1,2-dichloroethane (3.7 mL) and the resulting mixture was stirred at ambient temperature under nitrogen for 2.5 h. Sodium triacetoxyborohydride (143 mg, 0.67 mmol) was added and the mixture stirred for a further 3.5 h. The reaction was quenched with aqueous saturated sodium bicarbonate and extracted with DCM. The organic layer was dried (Na₂SO₄) and evaporated *in vacuo.* The residue was chromatographed on a 25 g Si cartridge eluting with 0-100% ethyl acetate in iso-hexane to give Intermediate 22a (141 mg).
LCMS (Method 4): Rt = 1.31 min, m/z 702.4 [M+H]⁺

### Intermediates 22b to 22j

The following intermediates were prepared from the indicated starting materials using a method analogous to that used for Intermediate 22a.

| Intermediate | Structure | Starting materials | LCMS |
|---|---|---|---|
| 22b | 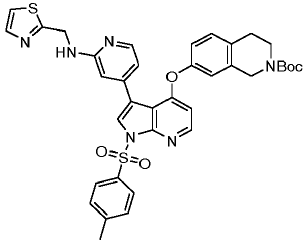 | Intermediate 9e and 2-thiazole-carboxaldehyde | Rt = 1.93 min, m/z 709.3 [M+H]⁺ (Method 4) |
| 22c | 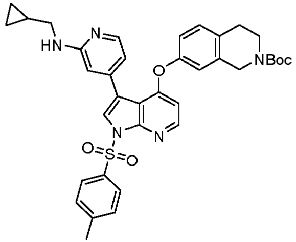 | Intermediate 9e and cyclopropane-carboxaldehyde | Rt = 1.79 min, m/z 666.3 [M+H]⁺ (Method 4) |
| 22d | 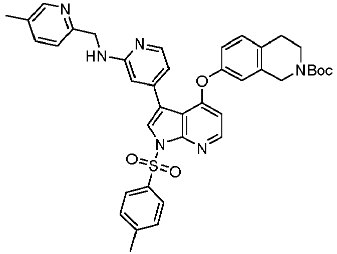 | Intermediate 9e and 5-methylpicolin-aldehyde | Rt = 1.31 min, m/z 717.1 [M+H]⁺ (Method 4) |
| 22e | 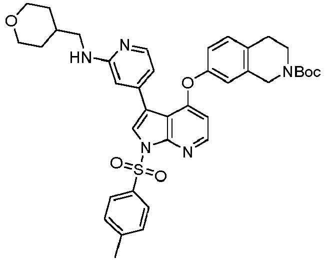 | Intermediate 9e and tetrahydro-2H-pyran-4-carbaldehyde | Rt = 1.30 min, m/z 710.2 [M+H]⁺ (Method 4) |
| 22f | 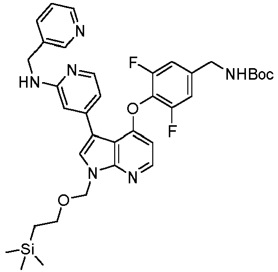 | Intermediate 9h and nicotin-aldehyde | Rt = 1.40 min, m/z 689.2 [M+H]⁺ (Method 4) |
| 22g | 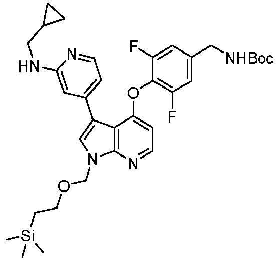 | Intermediate 9h and cyclopropane-carboxaldehyde | Rt = 1.35 min, m/z 652.4[M+H]⁺ (Method 4) |
| 22h | 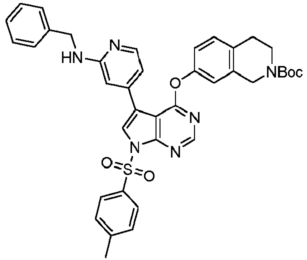 | Intermediate 9j and benzaldehyde | Rt = 1.35 min, m/z 703.2 [M+H]⁺ (Method 4) |
| 22i | 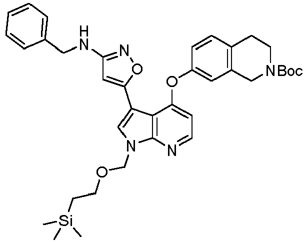 | Intermediate 21 and benzaldehyde | Rt = 1.86 min, m/z 668.5 [M+H]⁺ (Method 4) |
| 22j | 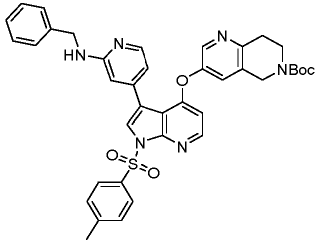 | Intermediate 9i and benzaldehyde | Rt = 1.68 min, m/z 703.4 [M+H]⁺ (Method 4) |

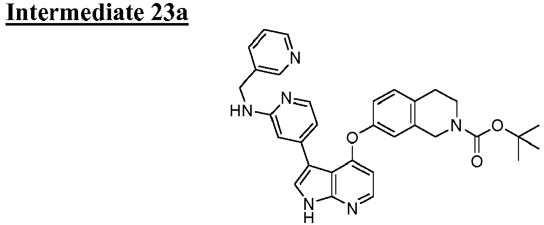

### tert-Butyl 7-((3-(2-((pyridin-3-ylmethyl)amino)pyridin-4-yl)-1H-pyrrolo[2,3-b]pyridin-4-yl)oxy)-3,4-dihydroisoquinoline-2(1H)-carboxylate

A solution of Intermediate 9f (50 mg, 0.074 mmol), pyridin-3-ylmethanamine (32 mg, 0.296 mmol), Pd₂(dba)₃ (9.5 mg, 0.011 mmol), Xantphos (9.4 mg, 0.017 mmol) and sodium tert-butoxide (50 mg, 0.518 mmol) in dioxane (2 mL) was stirred at 100°C for 1 h. The solution was diluted with ethyl acetate (50 mL) was washed with water (50 mL). The product was extracted into ethyl acetate (2 × 50 mL). The combined extracts were dried (Na₂SO₄) and evaporated to give Intermediate 23a (0.041 g).
LCMS (Method 4): Rt = 1.04 min, m/z 549.1 [M+H]⁺

### Intermediates 23b to 23e

The following intermediates were prepared from the indicated starting materials using a method similar to that used in the synthesis of Intermediate 23a.

| Intermediate | Structure | Starting materials | LCMS |
|---|---|---|---|
| 23b | 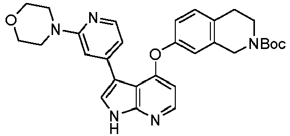 | Intermediate 9f and morpho line | Rt = 1.02 min, m/z 528.4 [M+H]⁺ (Method 4) |
| 23c | 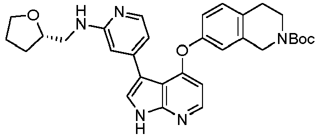 | Intermediate 9f and (S)-(+)-tetrahydro-furfurylamine | Rt = 1.10 min, m/z 542.2 [M+H]⁺ (Method 4) |
| 23d | 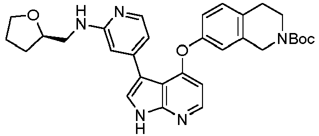 | Intermediate 9f and (R)-(-)-tetrahydro-furfurylamine | Rt = 1.10 min, m/z 542.2 [M+H]⁺ (Method 4) |
| 23e | 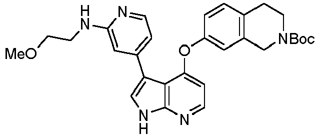 | Intermediate 9f and 2-methoxy-ethylamine | Rt = 1.00 min, m/z 516.4 [M+H]⁺ (Method 4) |

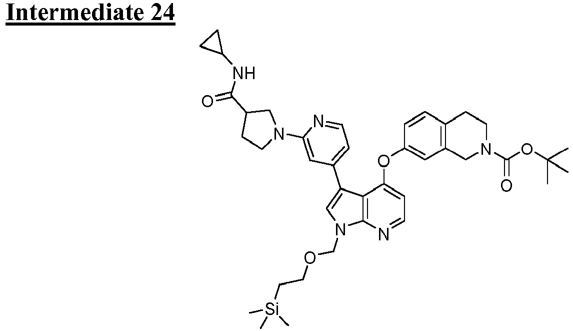

### tert-butyl 7-((3-(2-(3-(cyclopropylcarbamoyl)pyrrolidin-1-yl)pyridin-4-yl)-1-((2-(trimethylsilyl)ethoxy)methyl)-1H-pyrrolo[2,3-b]pyridin-4-yl)oxy)-3,4-dihydroisoquinoline-2(1H)-carboxylate

A degassed mixture of Intermediate 9g (200 mg, 0.307 mmol), N-cyclopropylpyrrolidine-3-carboxamide (71 mg, 0.460 mmol), Pd₂(dba)₃ (28 mg, 0.031 mmol), BINAP (38mg, 0.062 mmol) and sodium tert-butoxide (59 mg, 0.614 mmol) in toluene (10 mL) was heated at 100°C for 1 h. The resulting mixture was diluted with water and extracted with ethyl acetate. The organic layer was dried (Na₂SO₄) and evaporated *in vacuo.* The residue was chromatographed on a 25 g Si cartridge eluting with 0-100% ethyl acetate in iso-hexane to give Intermediate 24 (136 mg).
LCMS (Method 4): Rt = 1.43 min, m/z 725.3 [M+H]⁺

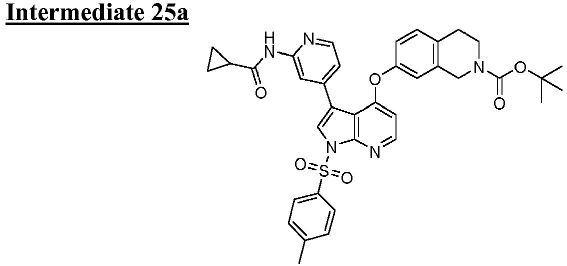

### tert-Butyl 7-((3-(2-(cyclopropanecarboxamido)pyridin-4-yl)-1-tosyl-1H-pyrrolo [2,3-b]pyridin-4-yl)oxy)-3,4-dihydroisoquinoline-2(1H)-carboxylate

Intermediate 9e (348 mg, 0.57 mmol), cyclopropanecarboxylic acid (98 mg, 1.14 mmol), 1-(methylsulfonyl)-1H-benzotriazole (224 mg, 1.14 mmol) and triethylamine (0.24 mL, 1.71 mmol) were added together in THF (5 mL) and the resulting mixture was heated at 120 °C in the microwave for 2 h. A further quantity of cyclopropanecarboxylic acid (49 mg, 0.57 mmol) was added and heating was continued for 4 h. The reaction was quenched with saturated aqueous sodium bicarbonate and extracted with ethyl acetate. The organic layer was dried over (Na₂SO₄) and evaporated *in vacuo.* The residue was chromatographed on a 25 g Si cartridge eluting with 0-100% ethyl acetate in iso-hexane to give Intermediate 25a (231 mg).
LCMS (Method 4): Rt = 1.66 min, m/z 680.2 [M+H]⁺

### Intermediates 25b to 25h

The following intermediates were prepared in a similar manner to Intermediate 25a from the given intermediate and the carboxylic acid.

| Intermediate | Structure | Starting materials | LCMS |
|---|---|---|---|
| 25b | 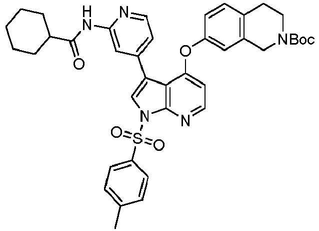 | Cyclohexanecar boxylic acid and Intermediate 9e | Rt = 1.88 min, m/z 722.2 [M+H]⁺ (Method 4) |
| 25c | 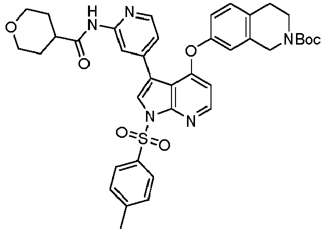 | Tetrahydropyran -4-yl carboxylic acid and Intermediate 9e | Rt = 1.68 min, m/z 724.2 [M+H]⁺ (Method 4) |
| 25d | 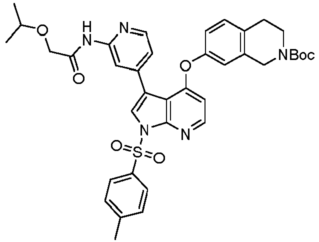 | Isopropoxy acetic acid and Intermediate 9e | Rt = 1.86 min, m/z 712.1 [M+H]⁺ (Method 4) |
| 25e | 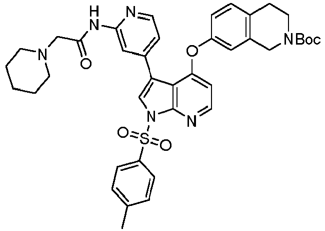 | 2-(Piperidin-1-yl)acetic acid and Intermediate 9e | Rt = 1.24 min, m/z 737.2 [M+H]⁺ (Method 4) |
| 25f | 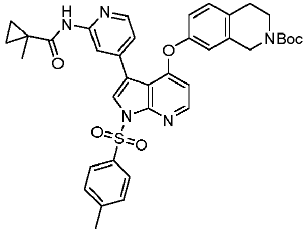 | 1-Methylcyclo-propanecarboxyl ic acid and Intermediate 9e | Rt = 1.78 min, m/z 694.1 [M+H]⁺ (Method 4) |
| 25g | 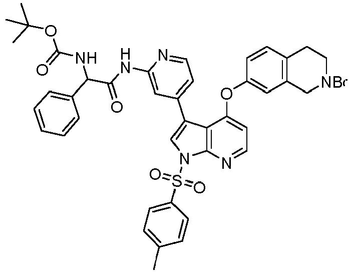 | 2-((tert-Butoxycarbonyl)amino) -2-phenylacetic acid and Intermediate 9e | Rt = 1.87 min, m/z 845.5 [M+H]⁺ (Method 4) |
| 25h | 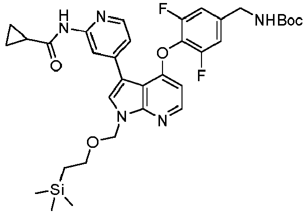 | Cyclopropanecarboxylic acid and Intermediate 9h | Rt=1.59 min. m/z 666.4 [M+H]⁺ (Method 4) |

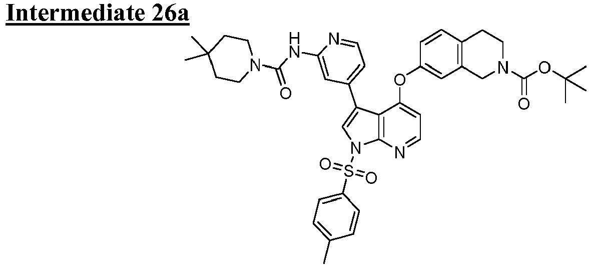

### tert-Butyl 7-((3-(2-(4,4-dimethylpiperidine-1-carboxamido)pyridin-4-yl)-1-tosyl-1H-pyrrolo[2,3-b]pyridin-4-yl)oxy)-3,4-dihydroisoquinoline-2(1H)-carboxylate

To a solution of Intermediate 9e (362 mg, 0.59 mmol) in THF (9.4 mL) was added triethylamine (1.6 mL, 19.3 mmol) followed by triphosgene (211 mg, 0.71 mmol) and the resulting mixture was stirred at RT for 0.75 h. 4,4-Dimethylpiperidine (804 mg, 7.10 mmol) was added and the mixture was heated at 70°C for 18 h. The mixture was allowed to cool to RT, diluted with water and extracted with ethyl acetate. The organic layer was dried (Na₂SO₄) and evaporated under reduced pressure. The residue was chromatographed on a 40 g Si cartridge eluting with 0-70% ethyl acetate in iso-hexane to give the title compound (223 mg).
LCMS (Method 4): Rt = 1.61 min, m/z 751.2 [M+H]⁺

### Intermediates 26b to 26c

The following intermediates were prepared in a similar manner from Intermediate 9e and the amine given.

| Intermediate | Structure | Amine | LCMS |
|---|---|---|---|
| 26b | 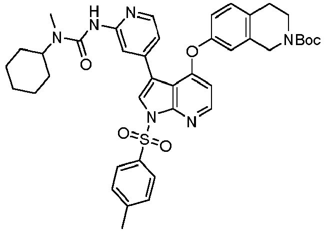 | N-Methyl-cyclo hexanamin e | Rt = 1.60 min, m/z 751.2 [M+H]⁺ (Method 4) |
| 26c | 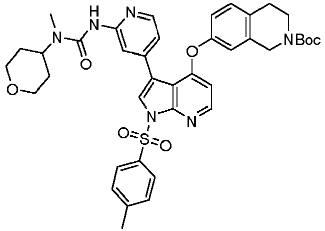 | N-Methyl-tetrahydro-2H-pyran-4-amine | Rt = 1.45 min, m/z 753.4 [M+H]⁺ (Method 4) |

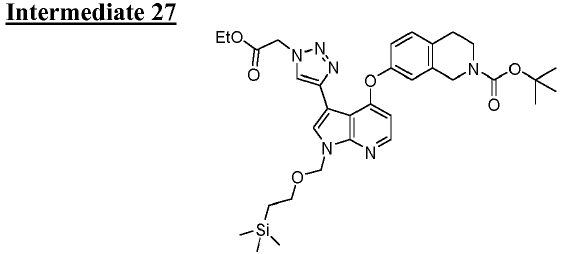

### tert-Butyl 7-((3-(1-(2-ethoxy-2-oxoethyl)-1H-1,2,3-triazol-4-yl)-1-((2-(trimethylsilyl)ethoxy)methyl)-1H-pyrrolo[2,3-b]pyridin-4-yl)oxy)-3,4-dihydroisoquinoline-2(1H)-carboxylate

A mixture of Intermediate 17 (285 mg, 0.55 mmol), water (2.3 mL), THF (4.5 mL) and copper sulfate pentahydrate (14 mg, 0.055 mmol) was treated with (+)-sodium L-ascorbate (16 mg, 0.082 mmol) then ethyl azidoacetate (25% solution by NMR) (0.15 mL, ca 0.55 mmol) was added. The mixture was stirred at ambient temperature for 6 h. A second aliquot of (+)-sodium L-ascorbate (16 mg, 0.082 mmol) was added and the mixture was stirred for a further 60 h. The mixture was diluted with ethyl acetate (20 mL) and partitioned with water (20 mL). The aqueous phase was washed with ethyl acetate (2 × 20 mL). The combined organic phase was filtered through a hydrophobic frit then concentrated *in vacuo* to afford the crude product. This was purified on a 5 g Si cartridge eluting with 0-25% ethyl acetate in DCM to give the desired product (160 mg).
LCMS (Method 4): Rt = 1.72 min, m/z 649.5 [M+H]⁺

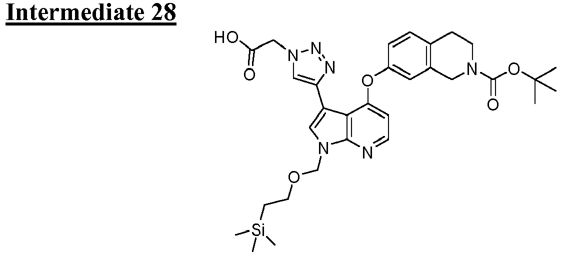

### 2-(4-(4-((2-(tert-Butoxycarbonyl)-1,2,3,4-tetrahydroisoquinolin-7-yl)oxy)-1-((2-(trimethylsilyl)ethoxy)methyl)-1H-pyrrolo[2,3-b]pyridin-3-yl)-1H-1,2,3-triazol-1-yl)acetic acid

Intermediate 28 was prepared from intermediate 27 using a method similar to that used for Intermediate 19.
LCMS (Method 4): Rt = 1.66 min, m/z 621.5 [M+H]⁺

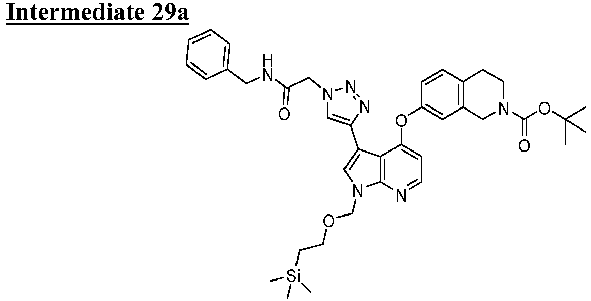

### tert-Butyl 7-((3-(1-(2-(benzylamino)-2-oxoethyl)-1H-1,2,3-triazol-4-yl)-1-((2-(trimethylsilyl)ethoxy)methyl)-1H-pyrrolo[2,3-b]pyridin-4-yl)oxy)-3,4-dihydroisoquinoline-2(1H)-carboxylate

A mixture of Intermediate 28 (240 mg, 0.39 mmol), benzylamine (62 mg, 0.063 mL, 0.58 mmol) and DIPEA (110 mg, 0.15mL, 0.85 mmol) in DCM (5 mL) was treated with COMU (331 mg, 0.77 mmol). The mixture was stirred at ambient temperature for 2 h then treated with water (15 mL) and extracted with DCM (3 × 15 mL). The combined organic phase was dried (Na₂SO₄) and concentrated *in vacuo.* The residue was purified on a 5 g Si cartridge eluting with 0-40% ethyl acetate in DCM to afford the desired product (250 mg).
LCMS (Method 4): Rt = 1.70 min, m/z 710.6 [M+H]⁺

### Intermediates 29b

Intermediate 29b was prepared in a similar manner to Intermediate 29a starting from the indicated acid and benzylamine.

| Intermediate | Structure | Starting material | LCMS |
|---|---|---|---|
| 29b | 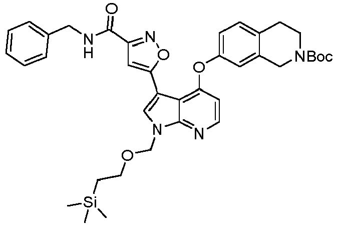 | Intermediate 19 | Rt = 1.88 min, m/z 696.5 [M+H]⁺ (Method 4) |

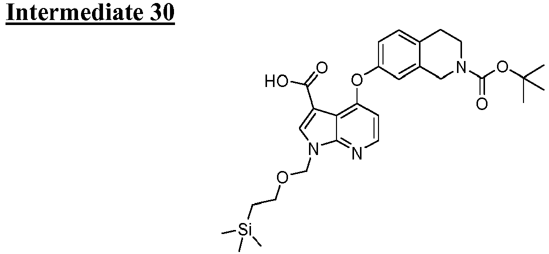

### 4-((2-(tert-Butoxycarbonyl)-1,2,3,4-tetrahydroisoquinolin-7-yl)oxy)-1-((2-(trimethylsilyl)ethoxy)methyl)-1H-pyrrolo[2,3-b]pyridine-3-carboxylic acid

Intermediate 7a (750 mg, 1.21 mmol), lithium chloride (163 mg, 3.85 mmol), lithium formate monohydrate (269 mg, 3.85 mmol), acetic anhydride (243 µL, 2.57 mmol) and N,N-diisopropylethylamine (782 µL, 4.49 mmol) in DMF (12.5 mL) was purged with argon. Palladium acetate (29 mg, 0.129 mmol) was added and the reaction was heated at 55°C under argon for 3 h. The mixture was allowed to cool and ethyl acetate (25 mL) was added followed by water (10 mL), saturated brine (10 mL) and 5% aqueous KHSO₄ (10 mL). The phases were separated and the aqueous phase was extracted with ethyl acetate (25 mL). The combined organics were washed with brine (3 × 10 mL), dried (Na₂SO₄) and evaporated. The crude mixture was chromatographed on a 10 g Si cartridge eluting with 0-40% ethyl acetate in DCM to afford the title compound (370 mg).
LCMS (Method 4): Rt = 1.71 min, m/z 540.4 [M+H]⁺

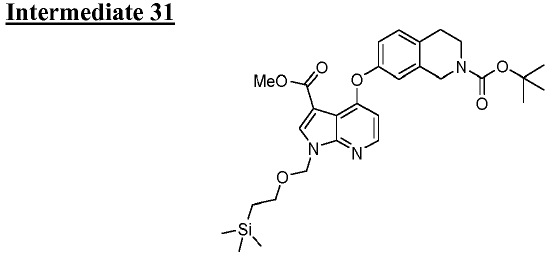

### tert-Butyl 7-((3-(methoxycarbonyl)-1-((2-(trimethylsilyl)ethoxy)methyl)-1H-pyrrolo[2,3-b]pyridin-4-yl)oxy)-3,4-dihydroisoquinoline-2(1H)-carboxylate

A solution of Intermediate 30 (370 mg, 0.686 mmol) in DCM (5 mL) was treated with 1,1'-carbonyldiimidazole (122 mg, 0.754 mmol). The mixture was stirred for 1 h and then methanol (0.5 mL, 12.34 mmol) was added and stirring was continued for 1 h. A further quantity of methanol (1 mL, 24.68 mmol) was added and the mixture was allow to stand at RT overnight. The mixture was diluted with water and extracted with DCM (2 × 10 mL). The combined organic extracts were dried (MgSO₄) and evaporated. The residue was chromatographed on a 5 g Si cartridge eluting with 4-20% ethyl acetate in DCM to afford the title compound (300 mg).
LCMS (Method 4): Rt = 1.80 min, m/z 554.5 [M+H]⁺

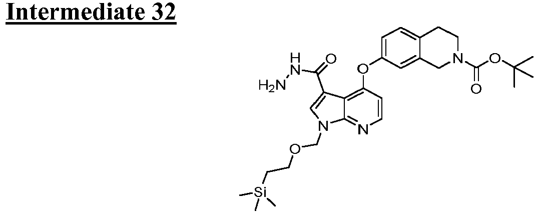

### tert-Butyl 7-((3-(hydrazinecarbonyl)-1-((2-(trimethylsilyl)ethoxy)methyl)-1H-pyrrolo[2,3-b]pyridin-4-yl)oxy)-3,4-dihydroisoquinoline-2(1H)-carboxylate

Intermediate 31 (300 mg, 0.542 mmol) and hydrazine hydrate (0.169 mL, 5.42 mmol) in methanol (5 mL) were heated at reflux for 40 h. The reaction mixture was allowed to cool and diluted with ethyl acetate. The solution was washed with a mixture of water and saturated brine and the aqueous phase was extracted with ethyl acetate. The combined organic extracts were washed with brine, dried (Na₂SO₄) and evaporated. The crude product was purified by chromatography on a 5 g Si cartridge eluting with 2% methanol in DCM. The product was obtained as an off-white solid (260 mg).
LCMS (Method 4): Rt = 1.57 min, m/z 554.5 [M+H]⁺

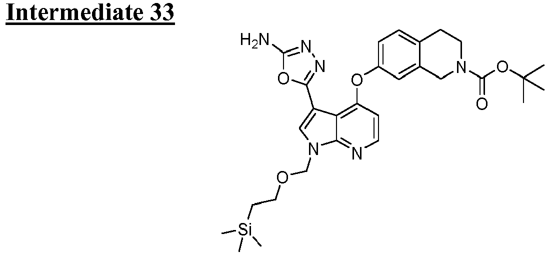

### tert-Butyl 7-((3-(5-amino-1,3,4-oxadiazol-2-yl)-1-((2-(trimethylsilyl)ethoxy)-methyl)-1H-pyrrolo[2,3-b]pyridin-4-yl)oxy)-3,4-dihydroisoquinoline-2(1H)-carboxylate

A solution of Intermediate 32 (260 mg, 0.470 mmol) in DMSO (2 mL) was treated with di(1H-imidazol-1-yl)methanimine (151 mg, 0.939 mmol) and the mixture was stirred until all of the latter had dissolved. Stirring was then continued for a further 3 h and the mixture was allowed to stand at RT overnight. The mixture was diluted with ethyl acetate and the solution was washed with water. The aqueous was further extracted with ethyl acetate and the combined organics were washed with brine, dried (MgSO₄) and evaporated. The product was purified on a 5 g Si cartridge eluting with 1:1 ethyl acetate in DCM to give the desired product (180 mg).
LCMS (Method 4): Rt = 1.55 min, m/z 579.5 [M+H]⁺

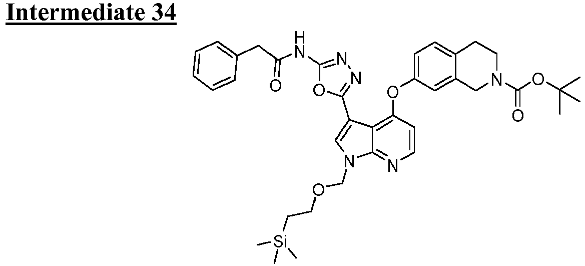

### tert-Butyl 7-((3-(5-(2-phenylacetamido)-1,3,4-oxadiazol-2-yl)-1-((2-(trimethyl-silyl)ethoxy)methyl)-1H-pyrrolo[2,3-b]pyridin-4-yl)oxy)-3,4-dihydroisoquinoline-2(1H)-carboxylate

Intermediate 34 was prepared from Intermediate 33 and phenylacetic acid using a method similar to that used for Intermediate 25a.
LCMS (Method 5): Rt = 2.16 min, m/z 697.1 [M+H]⁺

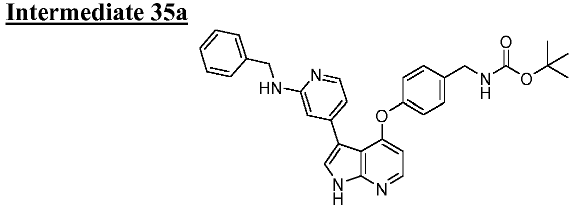

### tert-Butyl (4-((3-(2-(benzylamino)pyridin-4-yl)-1H-pyrrolo[2,3-b]pyridin-4-yl)oxy)benzyl)carbamate

Lithium hydroxide monohydrate (72 mg, 1.72 mmol) was added to a solution of Intermediate 9d (194 mg, 0.29 mmol) in a mixture of methanol (3 mL), water (0.8 mL) and THF (3 mL) and the resulting mixture was stirred at RT for 1.5 h. The solvent was removed under reduced pressure and the residue was diluted with water and extracted with ethyl acetate (3 × 5 mL). The ethyl acetate layers were separated, combined, dried (Na₂SO₄) and evaporated under reduced pressure to give Intermediate 35a (149 mg) that was used without further purifications in the next step.
LCMS (Method 4): Rt = 1.05 min, m/z 522.4 [M+H]⁺

### Intermediates 35b to 35ff

The following intermediates were prepared from the starting materials given using a procedure analogous to that used for Intermediate 35a.

| Intermediate | Structure | Starting material | LCMS |
|---|---|---|---|
| 35b | 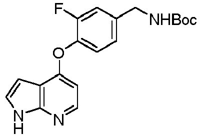 | Intermediate 4f | Rt = 1.15 min, m/z 358.2 [M+H]⁺ (Method 4) |
| 35c | 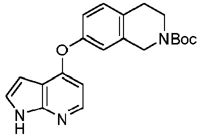 | Intermediate 4d | Rt = 1.27 min, m/z 366.3 [M+H]⁺ (Method 4) |
| 35d | 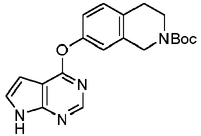 | Intermediate 4e | Rt = 1.45 min, m/z 367.0 [M+H]⁺ LCMS (Method 4) |
| 35e | 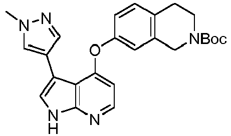 | Intermediate 9k | Rt = 1.15 min, m/z 446.3 [M+H]⁺ (Method 4) |
| 35f | 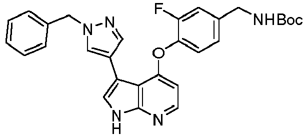 | Intermediate 91 | Rt = 1.30 min, m/z 514.3 [M+H]⁺ (Method 4) |
| 35g | 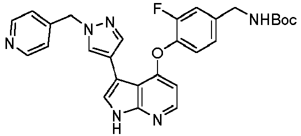 | Intermediate 9m | Rt = 0.93 min, m/z 515.3 [M+H]⁺ (Method 4) |
| 35h | 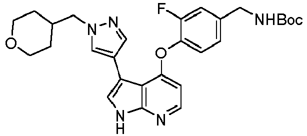 | Intermediate 9n | Rt = 1.18 min, m/z 522.3 [M+H]⁺ (Method 4) |
| 35i | 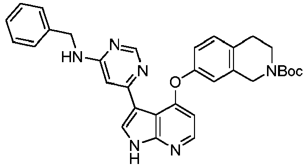 | Intermediate 15a | Rt = 1.15 min, m/z 549.3 [M+H]⁺ (Method 4) |
| 35j | 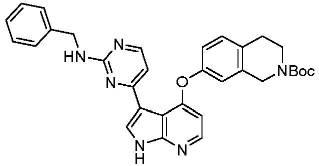 | Intermediate 15b | Rt = 1.32 min, m/z 549.4 [M+H]⁺ (Method 4) |
| 35k | 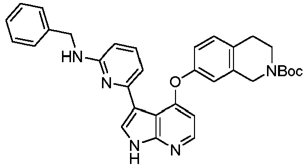 | Intermediate 15c | Rt = 1.19 min, m/z 548.3 [M+H]⁺ (Method 4) |
| 351 | 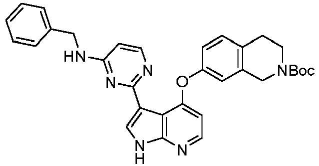 | Intermediate 15d | Rt = 1.17 min, m/z 549.3 [M+H]⁺ (Method 4) |
| 35m | 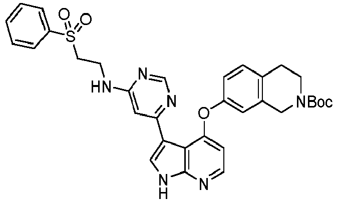 | Intermediate 15h | Rt = 1.13 min, m/z 627.1 [M+H]⁺ (Method 4) |
| 35n | 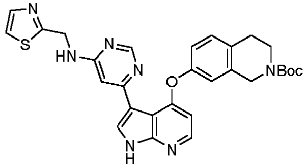 | Intermediate 15j | Rt = 1.36 min, m/z 556.3 [M+H]⁺ (Method 4) |
| 35o | 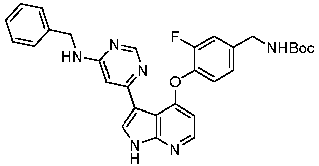 | Intermediate 15m | Rt = 1.06 min, m/z 541.3 [M+H]⁺ (Method 4) |
| 35p | 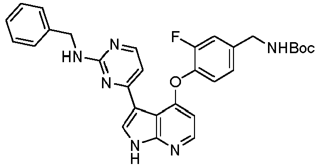 | Intermediate 15n | Rt = 1.20 min, m/z 541.3 [M+H]⁺ (Method 4) |
| 35q | 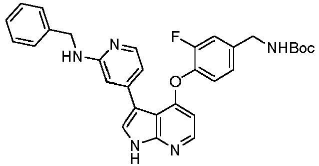 | Intermediate 15o | Rt = 1.10 min, m/z 540.3 [M+H]⁺ (Method 4) |
| 35r | 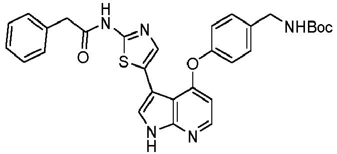 | Intermediate 15r | Rt = 1.37 min, m/z 556.3 [M+H]⁺ (Method 4) |
| 35s | 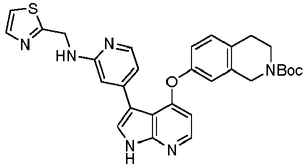 | Intermediate 22b | Rt = 1.37 min, m/z 555.2 [M+H]⁺ (Method 4) |
| 35t | 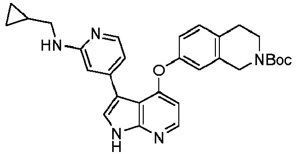 | Intermediate 22c | Rt = 1.44 min, m/z 512.3 [M+H]⁺ (Method 4) |
| 35u | 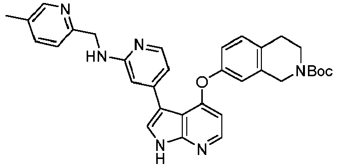 | Intermediate 22d | Rt = 1.05 min, m/z 563.2 [M+H]⁺ (Method 4) |
| 35v | 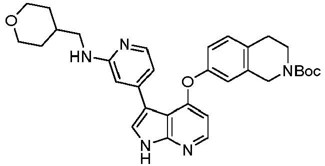 | Intermediate 22e | Rt = 1.06 min, m/z 556.2 [M+H]⁺ (Method 4) |
| 35w | 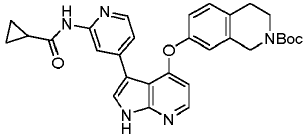 | Intermediate 25a | Rt = 1.48 min, m/z 526.1 [M+H]+ (Method 4) |
| 35x | 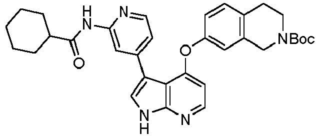 | Intermediate 25b | Rt = 1.38 min, m/z 568.2 [M+H]+ (Method 4) |
| 35y | 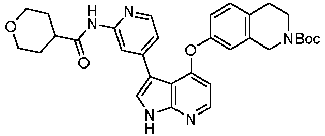 | Intermediate 25c | Rt = 1.21 min, m/z 570.2 [M+H]+ (Method 4) |
| 35z | 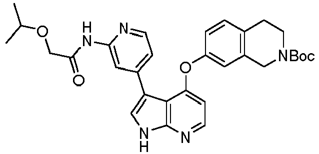 | Intermediate 25d | Rt = 1.47 min, m/z 558.2 [M+H]+ (Method 4) |
| 35aa | 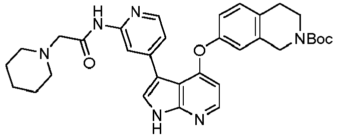 | Intermediate 25e | Rt = 1.03 min, m/z 583.2 [M+H]+ (Method 4) |
| 35bb | 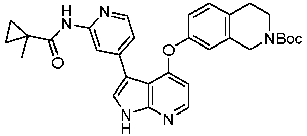 | Intermediate 25f | Rt = 1.33 min, m/z 540.4 [M+H]+ (Method 4) |
| 35cc | 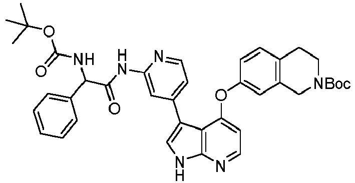 | Intermediate 25g | Rt = 1.49 min, m/z 691.4 [M+H]+ (Method 4) |
| 35dd | 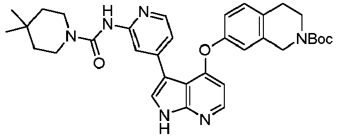 | Intermediate 26a | Rt = 1.23 min, m/z 597.2 [M+H]⁺ (Method 4) |
| 35ee | 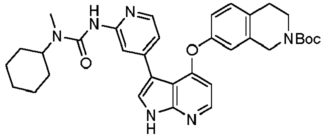 | Intermediate 26b | Rt = 1.27 min, m/z 597.2 [M+H]⁺ (Method 4) |
| 35ff | 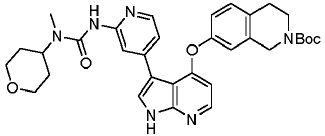 | Intermediate 26c | Rt = 1.06 min, m/z 599.4 [M+H]⁺ (Method 4) |

### PREPARATION OF EXAMPLES

### Example 1

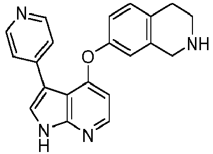

### 7-((3-(Pyridin-4-yl)-1H-pyrrolo[2,3-b]pyridin-4-yl)oxy)-1,2,3,4-tetrahydroisoquinoline

Intermediate 9a (0.68 g, 1.19 mmol) was dissolved in a mixture of DCM (5 mL) and TFA (5 mL), and the reaction mixture was stirred at RT for 2 h. The mixture was passed down a 20 g SCX-2 cartridge eluting with methanol and then 2M methanolic ammonia. After standing for 18 h, the ammonia solution was evaporated to give a residue which was purified by MDAP (basic). The product was obtained as an off-white solid (150 mg).
LCMS (Method 3): Rt = 3.08 min, m/z 343.4 [M+H]⁺

¹H NMR (400 MHz, DMSO) δ 12.29 (s, 1H), 8.46 (d, J=6.2 Hz, 2H), 8.23 (s, 1H), 8.13 (d, J=5.4 Hz, 1H), 7.91 (s, 1H), 7.71 (d, J=6.2 Hz, 2H), 7.20 (d, J=8.3 Hz, 1H), 7.04 - 6.99 (m, 1H), 6.98 - 6.95 (m, 1H), 6.35 (d, J=5.4 Hz, 1H), 3.94 (s, 2H), 3.07 (t, J=6.0 Hz, 2H), 2.77 (t, J=5.8 Hz, 2H).

### Example 2 to 14

The following examples were prepared from the Intermediate (I) indicated using a method similar to Example 1.

| Ex. | I | Structure | NMR | LCMS |
|---|---|---|---|---|
| 2 | 9b | 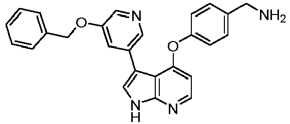 | ¹H NMR (600 MHz, DMSO-d6) δ 12.22 (br. s., 1 H), 8.48 (d, J=1.6 Hz, 1 H), 8.02 - 8.22 (m, 3 H), 7.81 (s, 1 H), 7.75 (dd, J=2.6, 2.0 Hz, 1 H), 7.51 - 7.58 (m, 2 H), 7.37 - 7.43 (m, 4 H), 7.31 - 7.36 (m, 1 H), 7.22 - 7.29 (m, 2 H), 6.34 (d, J=5.3 Hz, 1 H), 5.11 (s, 2 H), 4.03 (s, 2 H). | Rt = 0.51 min, m/z 423.3 [M+H]+ (Method 6) |
| | | (4-((3-(5-(benzyloxy)pyridin-3-yl)-1H-pyrrolo[2,3-b]pyridin-4-yl)oxy)phenyl)methanamine | | |
| 3 | 9c | 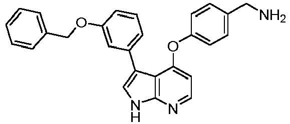 | ¹H NMR (400 MHz, DMSO) δ 12.01 (s, 1H), 8.09 (d, J=5.5 Hz, 1H), 7.63 (s, 1H), 7.42-7.35 (m, 4H), 7.35-7.22 (m, 6H), 7.16-7.13 (m, 2H), 6.83 (ddd, J=1.2, 2.5, 7.9 Hz, 1H), 6.31 (d, J=5.4 Hz, 1H), 4.98 (s, 2H), 3.71 (s, 2H). | Rt = 3.19 min, m/z 422.2 [M+H]⁺ (Method 1) |
| | | (4-((3-(3-(benzyloxy)phenyl)-1H-pyrrolo[2,3-b]pyridin-4-yl)oxy)phenyl)methanamine | | |
| 4 | 15p | 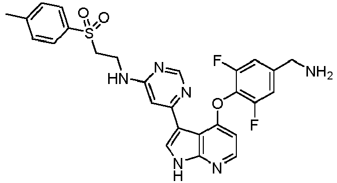 | ¹H NMR (400 MHz, d6-DMSO) δ 12.37 (s, 1H), 8.34 (d, J=1.0 Hz, 1H), 8.15-8.10 (m, 2H), 7.73 (d, J=8.1 Hz, 2H), 7.40-7.33 (m, 5H), 7.06 (s, 1H), 6.30 (d, J=5.6 Hz, 1H), 3.79 (s, 2H), 3.52 (s, 4H), 2.3 1 (s, 3H). | Rt = 2.11 min, m/z 551.2 [M+H]⁺ (Method 1) |
| | | 6-(4-(4-(aminomethyl)-2,6-difluorophenoxy)-1H-pyrrolo[2,3-b]pyridin-3-yl)-N-(2-tosylethyl)pyrimidin-4-amine | | |
| 5 | 15q | 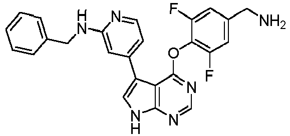 | ¹H NMR (400 MHz, DMSO) δ 8.35 (s, 1H), 7.96 (dd, J=0.5, 5.4 Hz, 1H), 7.91 (s, 1H), 7.30-7.23 (m, 6H), 7.20 - 7.13 (m, 1H), 7.01 (t, J=6.2 Hz, 1H), 6.92 - 6.88 (m, 2H), 4.46 (d, J=6.2 Hz, 2H), 3.76 (s, 2H). | Rt = 2.09 min, m/z 459.2 [M+H]⁺ (Method 1) |
| | | 4-(4-(4-(aminomethyl)-2,6-difluorophenoxy)-7H-pyrrolo[2,3-d]pyrimidin-5-yl)-N-benzylpyridin-2-amine | | |
| 6 | 15s | 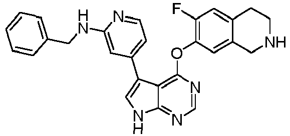 | ¹H NMR (400 MHz, DMSO) δ 12.61 (s, 1H), 8.31 (s, 1H), 7.95-7.92 (m, 1H), 7.84 (s, 1H), 7.30-7.23 (m, 4H), 7.20-7.15 (m, 1H), 7.08 (t, J=9.3 Hz, 2H), 6.98 (t, J=6.2 Hz, 1H), 6.91-6.89 (m, 2H), 4.47 (d, J=6.1 Hz, 2H), 3.80 (s, 2H), 2.94 (t, J=5.8 Hz, 2H), 2.73-2.67 (m, 2H). | Rt = 2.19 min, m/z 467.1 [M+H]⁺ (Method 1) |
| | | N-benzyl-4-(4-((6-fluoro-1,2,3,4-tetrahydroisoquinolin-7-yl)oxy)-7H-pyrrolo[2,3-d]pyrimidin-5-yl)pyridin-2-amine | | |
| 7 | 22f | 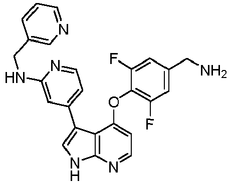 | ¹H NMR (400 MHz, DMSO) □ 12.24 (s, 1H), 8.51 (d, J=1.7 Hz, 1H), 8.39 (dd, J=1.7, 4.8 Hz, 1H), 8.11 (d, J=5.5 Hz, 1H), 7.93-7.91 (m, 1H), 7.75 (s, 1H), 7.70-7.65 (m, 1H), 7.35-7.27 (m, 3H), 6.99 (t, J=6.2 Hz, 1H), 6.89 (dd, J=1.5, 5.6 Hz, 1H), 6.85-6.82 (m, 1H), 6.28 (d, J=5.4 Hz, 1H), 4.48 (d, J=6.1 Hz, 2H), 3.77 (s, 2H), 2.13 (s, 2H). | Rt = 2.95 min, m/z 459.4 [M+H]⁺ (Method 3) |
| | | 4-(4-(4-(aminomethyl)-2,6-difluorophenoxy)-1H-pyrrolo[2,3 - b]pyridin-3-yl)-N-(pyridin-3-ylmethyl)pyridin-2-amine | | |
| 8 | 22g | 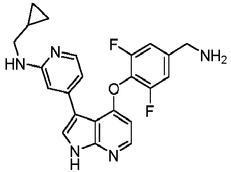 | ¹H NMR (400 MHz, DMSO) δ 12.24 (s, 1H), 8.12 (d, J=5.4 Hz, 1H), 7.90 (d, J=5.3 Hz, 1H), 7.77 (s, 1H), 7.34 (d, J=9.2 Hz, 2H), 6.84 (dd, J=1.5, 5.3 Hz, 1H), 6.80 (s, 1H), 6.33 (t, J=5.8 Hz, 1H), 6.28 (d, J=5.4 Hz, 1H), 3.77 (s, 2H), 3.04 (t, J=6.2 Hz, 2H), 1.05-0.97 (m, 1H), 0.35 (ddd, J=4.1, 5.8, 8.0 Hz, 2H), 0.15-0.10 (m, 2H). | Rt = 1.83 and 1.88 min, m/z 422.1 [M+H]⁺ (Method 2) |
| | | 4-(4-(4-(aminomethyl)-2,6-difluorophenoxy)-1H-pyrrolo[2,3 - b]pyridin-3-yl)-N-(cyclopropylmethyl)pyridin-2-amine | | |
| 9 | 22i | 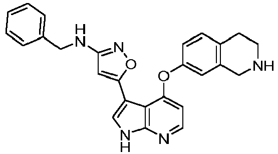 | ¹H NMR (400 MHz, DMSO) δ 12.33 (s, 1H), 8.11 (d, J=5.5 Hz, 1H), 7.89 (s, 1H), 7.33-7.25 (m, 4H), 7.24-7.18 (m, 2H), 6.99 (dd, J=2.6, 8.3 Hz, 1H), 6.92 (d, J=2.4 Hz, 1H), 6.62 (t, J=6.1 Hz, 1H), 6.34 (d, J=5.5 Hz, 1H), 6.28 (s, 1H), 4.22 (d, J=6.1 Hz, 2H), 3.85 (s, 2H), 2.96 (t, J=5.8 Hz, 2H), 2.72 (t, J=5.6 Hz, 2H). | Rt = 2.86 min, m/z 438.2 [M+H]⁺ (Method 1) |
| | | N-benzyl-5-(4-((1,2,3,4-tetrahydroisoquinolin-7-yl)oxy)-1H-pyrrolo[2,3-b]pyridin-3-yl)isoxazol-3-amine | | |
| 10 | 24 | 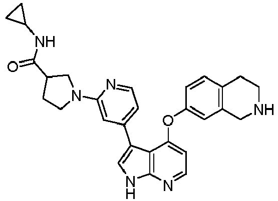 | ¹H NMR (400 MHz, DMSO) δ 12.14 (s, 1H), 8.10 (d, J=5.4 Hz, 1H), 8.02 (d, J=4.2 Hz, 1H), 7.96 (d, J=5.3 Hz, 1H), 7.81 (s, 1H), 7.13 (d, J=8.2 Hz, 1H), 6.95-6.84 (m, 3H), 6.80 (s, 1H), 6.33 (d, J=5.4 Hz, 1H), 3.81 (s, 2H), 3.52 (dd, J=8.2, 10.3 Hz, 1H), 3.48-3.39 (m, 1H), 3.38-3.33 (m, 1H), 3.28-3.20 (m, 1H), 2.95 (t, J=5.8 Hz, 2H), 2.88 (t, J=7.8 Hz, 1H), 2.68 (t, J=5.5 Hz, 2H), 2.63 (dt, J=3.8, 7.2 Hz, 1H), 2.06-1.95 (m, 2H), 0.64-0.58 (m, 2H), 0.42-0.37 (m, 2H). | Rt = 1.94 min, m/z 495.3 [M+H]⁺ (Method 1) |
| | | N-cyclopropyl-1-(4-(4-((1,2,3,4-tetrahydroisoquinolin-7-yl)oxy)-1H-pyrrolo[2,3-b]pyridin-3-yl)pyridin-2-yl)pyrrolidine-3-carboxamide | | |
| 11 | 25h | 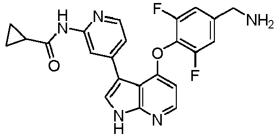 | ¹H NMR (400 MHz, DMSO) δ 12.34 (s, 1H), 10.64 (s, 1H), 8.43 (d, J=0.9 Hz, 1H), 8.24 (d, J=5.2 Hz, 1H), 8.14 (d, J=5.4 Hz, 1H), 7.85 (s, 1H), 7.40 (dd, J=1.6, 5.3 Hz, 1H), 7.32 (d, J=9.1 Hz, 2H), 6.32 (d, J=5.4 Hz, 1H), 3.76 (s, 2H), 2.01-1.93 (m, 3H), 0.78-0.71 (m, 4H). | Rt = 2.02 min. m/z 436.1[M +H]⁺ (Method 1) |
| | | N-(4-(4-(4-(aminomethyl)-2,6-difluorophenoxy)-1H-pyrrolo[2,3 - b]pyridin-3-yl)pyridin-2-yl)cyclopropanecarboxamide | | |
| 12 | 29a | 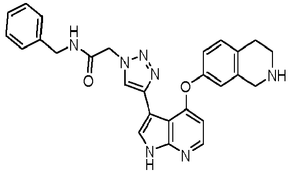 | ¹H NMR (400 MHz, DMSO) 12.03 (s, 1H), 8.80 (t, J=5.8 Hz, 1H), 8.14 (s, 1H), 8.08 (d, J=5.4 Hz, 1H), 7.88 (d, J=1.8 Hz, 1H), 7.34-7.18 (m, 6H), 7.04-6.95 (m, 2H), 6.29 (d, J=5.5 Hz, 1H), 5.17 (s, 2H), 4.30 (d, J=5.8 Hz, 2H), 3.90 (s, 2H), 3.02 (t, J=5.8 Hz, 2H), 2.78-2.72 (m, 2H). | Rt = 2.29 min, m/z 480.3 [M+H]⁺ (Method 1) |
| | | N-benzyl-2-(4-(4-((1,2,3,4-tetrahydroisoquinolin-7-yl)oxy)-1H-pyrrolo[2,3-b]pyridin-3-yl)-1H-1,2,3-triazol-1-yl)acetamide | | |
| 13 | 29b | 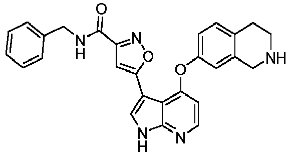 | ¹H NMR (400 MHz, DMSO) δ 9.29 (t, J=6.3 Hz, 1H), 8.17 (s, 1H), 8.16 (d, J=5.4 Hz, 1H), 7.34-7.21 (m, 5H), 7.19 (d, J=8.1 Hz, 1H), 7.01 (dd, J=2.4, 8.2 Hz, 1H), 6.94 (s, 2H), 6.39 (d, J=5.5 Hz, 1H), 4.42 (d, J=6.2 Hz, 2H), 4.01 (s, 1H), 3.86-3.82 (m, 3H), 2.96 (t, J=5.4 Hz, 2H), 2.71 (t, J=5.4 Hz, 2H). | Rt = 2.93 min, m/z 466.4 [M+H]⁺ (Method 1) Rt = 2.45 min, m/z 467.2 [M+H]⁺ (Method 1) |
| | | N-benzyl-5-(4-((1,2,3,4-tetrahydroisoquinolin-7-yl)oxy)-1H-pyrrolo [2,3-b]pyridin-3-yl)isoxazole-3-carboxamide | | |
| 14 | 34 | 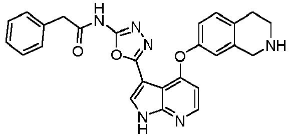 | ¹H NMR (400 MHz, DMSO) δ 8.28 (s, 1H), 8.19 (d, J=5.5 Hz, 1H), 8.07 (s, 1H), 7.33-7.25 (m, 5H), 7.12 (d, J=8.3 Hz, 1H), 6.90 (dd, J=2.6,8.3 Hz, 1H), 6.85 (d, J=2.5 Hz, 1H), 6.48 (d, J=5.5 Hz, 1H), 4.05-3.50 (m, 2H), 3.89 (s, 2H), 3.74 (s, 2H), 3.03 (t, J=5.9 Hz, 2H), 2.74 (t, J=5.7 Hz, 2H). | |
| | | 2-phenyl-N-(5-(4-((1,2,3,4-tetrahydroisoquinolin-7-yl)oxy)-1H-pyrrolo[2,3-b]pyridin-3-yl)-1,3,4-oxadiazol-2-yl)acetamide | | |

### Example 15

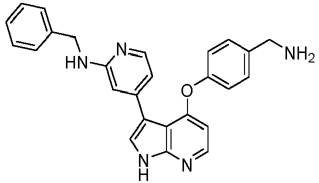

### 4-(4-(4-(Aminomethyl)phenoxy)-1H-pyrrolo[2,3-b]pyridin-3-yl)-N-benzylpyridin-2-amine

TFA (2.8 mL) was added to a solution of Intermediate 35a (149 mg, 0.29 mmol) in DCM (2.8 mL) and the resulting mixture was stirred at RT for 1 h. The reaction mixture was diluted with methanol and passed down a 10 g SCX-2 cartridge eluting with methanol and then a mixture of 7N methanolic ammonia and methanol (1:1). Basic product fraction were combined and evaporated under reduced pressure. The residue was chromatographed on a 12 g Si cartridge eluting with 0-10% 7N methanolic ammonia in DCM. Product fractions were combined and evaporated under reduced pressure. The residue was taken up in DCM and the solid was collected by filtration and dried under high vacuum at 50 °C for 18 h to give Example 15 as a yellow solid (27 mg).
LCMS (Method 1): Rt = 2.04 min, m/z 422.2 [M+H]⁺

¹H NMR (400 MHz, DMSO) δ 8.10 (d, J=5.4 Hz, 1H), 7.86 (d, J=5.7 Hz, 1H), 7.68 (s, 1H), 7.40 (d, J=8.6 Hz, 2H), 7.28 - 7.26 (m, 6H), 7.13 (d, J=8.6 Hz, 2H), 6.88 (t, J= 6.2 Hz, 1H), 6.84 - 6.81 (m, 2H), 6.33 (d, J=5.4 Hz, 1H), 4.44 (d, J=6.1 Hz, 2H), 3.73 (s, 2H).

### Example 16 to 48

The following examples were prepared from the indicated Intermediate (I) using a method similar to that used for Example 15.

| Ex. | I | Structure | NMR | LCMS |
|---|---|---|---|---|
| 16 | 23a | 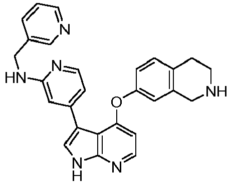 | ¹H NMR (400 MHz, DMSO) δ 12.12 (s, 1H), 8.51 (d, J=1.8 Hz, 1H), 8.40 (dd, J=1.6, 4.7 Hz, 1H), 8.10 (d, J=5.4 Hz, 1H), 7.86 (d, J=6.2 Hz, 1H), 7.71-7.65 (m, 2H), 7.28 (dd, J=4.8, 7.8 Hz, 1H), 7.13 (d, J=8.3 Hz, 1H), 6.99 (t, J=6.2 Hz, 1H), 6.96-6.92 (m, 1H), 6.89-6.83 (m, 3H), 6.34 (d, J=5.4 Hz, 1H), 4.46 (d, J=6.1 Hz, 2H), 3.83 (s, 2H), 2.97 (t, J=5.8 Hz, 2H), 2.70 (t, J=5.7 Hz, 2H). | Rt = 3.13 min, m/z 449.4[M+ H]⁺ (Method 3) |
| | | N-(pyridin-3-ylmethyl)-4-(4-((1,2,3,4-tetrahydroisoquinolin- 7-yl)oxy)-1H-pyrrolo[2,3-b]pyridin-3-yl)pyridin-2-amine | | |
| 17 | 23b | 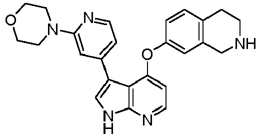 | ¹H NMR (400 MHz, DMSO) δ 12.18 (s, 1H), 8.11 (d, J=5.5 Hz, 1H), 8.04 (d, J=5.2 Hz, 1H), 7.85 (s, 1H), 7.17-7.13 (m, 2H), 7.03 (dd, J=1.3, 5.2 Hz, 1H), 6.95 (dd, J=2.5, 8.2 Hz, 1H), 6.89 (d, J=2.4 Hz, 1H), 6.36 (d, J=5.4 Hz, 1H), 3.82 (s, 2H), 3.59 (t, J=4.8 Hz, 4H), 3.34-3.30 (m, 4H), 2.96 (t, J=5.9 Hz, 2H), 2.70 (t, J=5.7 Hz, 2H). | Rt = 3.35 min, m/z 428.2 [M+H]⁺ (Method 3) |
| | | 4-(4-(4-((1,2,3,4-tetrahydroisoquinolin-7-yl)oxy)-1H-pyrrolo[2,3-b]pyridin-3-yl)pyridin-2-yl)morpholine | | |
| 18 | 23c | 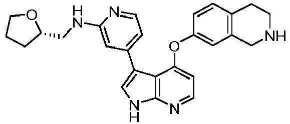 | ¹H NMR (400 MHz, DMSO) δ 12.12 (s, 1H), 8.11 (d, J=5.5 Hz, 1H), 7.85 (d, J=5.3 Hz, 1H), 7.69 (s, 1H), 7.19 (d, J=8.4 Hz, 1H), 7.01 (dd, J=2.5, 8.3 Hz, 1H), 6.96 (d, J=2.2 Hz, 1H), 6.83-6.79 (m, 2H), 6.34 (d, J=5.4 Hz, 1H), 6.26 (t, J=5.9 Hz, 1H), 3.99-3.93 (m, 3H), 3.77-3.71 (m, 1H), 3.60 (q, J=7.3 Hz, 1H), 3.27-3.22 (m, 2H), 3.10 (t, J=5.8 Hz, 2H), 2.80 (t, J=5.6 Hz, 2H), 1.90-1.73 (m, 3H), 1.56-1.46 (m, 1H). | Rt = 1.81/1.85 min, m/z 442.2 [M+H]⁺ (Method 2) |
| | | (S)-N-((tetrahydrofuran-2-yl)methyl)-4-(4-((1,2,3,4-tetrahydroisoquinolin-7-yl)oxy)-1H-pyrrolo[2,3-b]pyridin-3-yl)pyridin-2-amine | | |
| 19 | 23d | 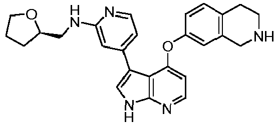 | ¹H NMR (400 MHz, DMSO) δ 12.12 (s, 1H), 8.11 (d, J=5.4 Hz, 1H), 7.85 (d, J=5.3 Hz, 1H), 7.69 (s, 1H), 7.20 (d, J=8.4 Hz, 1H), 7.01 (dd, J=2.5, 8.3 Hz, 1H), 6.96 (d, J=2.2 Hz, 1H), 6.83-6.79 (m, 2H), 6.34 (d, J=5.4 Hz, 1H), 6.26 (dd, J=5.8, 5.8 Hz, 1H), 4.00-3.93 (m, 3H), 3.77-3.71 (m, 1H), 3.60 (q, J=7.3 Hz, 1H), 3.27-3.22 (m, 2H), 3.11 (t, J=5.7 Hz, 2H), 2.81 (t, J=5.6 Hz, 2H), 1.90-1.73 (m, 3H), 1.56-1.46 (m, 1H). | Rt = 1.81/1.85 min, m/z 442.2 [M+H]⁺ (Method 2) |
| | | (R)-N-((tetrahydrofuran-2-yl)methyl)-4-(4-((1,2,3,4-tetrahydroisoquinolin-7-yl)oxy)-1H-pyrrolo[2,3-b]pyridin-3-yl)pyridin-2-amine | | |
| 20 | 23e | 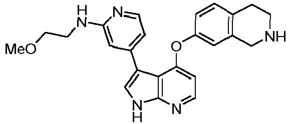 | ¹H NMR (400 MHz, DMSO) δ 12.09 (s, 1H), 8.09 (d, J=5.4 Hz, 1H), 7.86 (d, J=5.2 Hz, 1H), 7.68 (s, 1H), 7.13 (d, J=8.3 Hz, 1H), 6.93 (dd, J=2.5, 8.2 Hz, 1H), 6.88 (d, J=2.4 Hz, 1H), 6.83-6.81 (m, 2H), 6.32 (d, J=5.5 Hz, 1H), 6.27 (t, J=5.6 Hz, 1H), 3.82 (s, 2H), 3.44-3.34 (m, 4H), 3.23 (s, 3H), 2.94 (t, J=5.9 Hz, 2H), 2.71-2.66 (m, 2H). | Rt = 3.18 min, m/z 416.1 [M+H]⁺ (Method 3) |
| | | N-(2-methoxyethyl)-4-(4-((1,2,3,4-tetrahydroisoquinolin- 7-yl)oxy)-1H-pyrrolo[2,3-b]pyridin-3-yl)pyridin-2-amine | | |
| 21 | 35e | 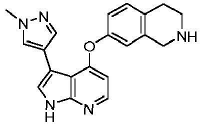 | ¹H NMR (400 MHz, DMSO) δ 11.77 (s, 1H), 8.03 (d, J=5.4 Hz, 1H), 7.86 (s, 1H), 7.71 (d, J=0.7 Hz, 1H), 7.54 (d, J=1.0 Hz, 1H), 7.16 (d, J=8.3 Hz, 1H), 6.98-6.88 (m, 2H), 6.26 (d, J=5.4 Hz, 1H), 3.84 (s, 2H), 3.80 (s, 3H), 2.95 (t, J=5.8 Hz, 2H), 2.70 (t, J=5.6 Hz, 2H). | Rt = 1.82 min, m/z 346.4 [M+H]⁺ (Method 1) |
| | | 7-((3-(1-methyl-1H-pyrazol-4-yl)-1H-pyrrolo[2,3-b]pyridin-4-yl)oxy)-1,2,3,4-tetrahydroisoquinoline | | |
| 22 | 35f | 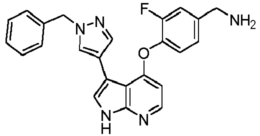 | ¹H NMR (400 MHz, DMSO) δ 11.83 (s, 1H), 8.04 (d, J=5.4 Hz, 1H), 7.98 (d, J=0.6 Hz, 1H), 7.78 (s, 1H), 7.59 (s, 1H), 7.42 (dd, J=1.7, 12.0 Hz, 1H), 7.31-7.19 (m, 7H), 6.20 (dd, J=0.9, 5.4 Hz, 1H), 5.29 (s, 2H), 3.77 (s, 2H), 2.20 (s, 2H). | Rt = 2.63 min, m/z 414.4 [M+H]⁺ (Method 1) |
| | | (4-((3-(1-benzyl-1H-pyrazol-4-yl)-1H-pyrrolo[2,3-b]pyridin-4-yl)oxy)-3-fluorophenyl)methanamine | | |
| 23 | 35g | 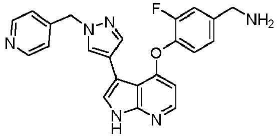 | ¹H NMR (400 MHz, DMSO) δ 11.87 (s, 1H), 8.46-8.43 (m, 2H), 8.06-8.04 (m, 2H), 7.83 (s, 1H), 7.62 (s, 1H), 7.41 (dd, J=1.8, 12.0 Hz, 1H), 7.34-7.21 (m, 2H), 7.10 (d, J=6.0 Hz, 2H), 6.21 (d, J=4.6 Hz, 1H), 5.39 (s, 2H), 3.76 (s, 2H), 2.26 (s, 2H). | Rt = 2.12 min, m/z 415.3 [M+H]⁺ (Method 1) |
| | | (3-fluoro-4-((3-(1-(pyridin-4-ylmethyl)-1H-pyrazol-4-yl)-1H-pyrrolo[2,3-b]pyridin-4-yl)oxy)phenyl)methanamine | | |
| 24 | 35h | 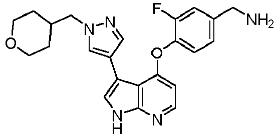 | ¹H NMR (400 MHz, DMSO) δ 11.82 (s, 1H), 8.04 (d, J=5.4 Hz, 1H), 7.88 (s, 1H), 7.72 (s, 1H), 7.57 (s, 1H), 7.44 (dd, J=1.8, 12.0 Hz, 1H), 7.35-7.22 (m, 2H), 6.21 (dd, J=0.9, 5.4 Hz, 1H), 3.96 (d, J=7.1 Hz, 2H), 3.76 (s, 2H), 3.71 (dd, J=2.7, 11.3 Hz, 2H), 3.16 (dt, J=1.8, 11.6 Hz, 2H), 2.33 - 1.83 (s, 2H), 2.02-1.91 (m, 1H), 1.36 (dd, J=1.8, 12.8 Hz, 2H), 1.16 (dq, J=4.2, 12.3 Hz, 2H). | Rt = 2.28 min, m/z 421.4 [M+H]⁺ LCMS (Method 1) |
| | | (3-fluoro-4-((3-(1-((tetrahydro-2H-pyran-4-yl)methyl)-1H-pyrazol-4-yl)-1H-pyrrolo[2,3-b]pyridin-4-yl)oxy)phenyl)methanamine | | |
| 25 | 35i | 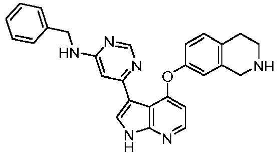 | ¹H NMR (400 MHz, DMSO) δ 12.25 (s, 1H), 8.34 (s, 1H), 8.11 (d, J=5.4 Hz, 1H), 8.04 (s, 1H), 7.75 (s, 1H), 7.28-7.23 (m, 2H), 7.22-7.18 (m, 3H), 7.13 (d, J=8.3 Hz, 1H), 6.93 (dd, J=1.6, 8.2 Hz, 1H), 6.85-6.84 (m, 1H), 6.40 (d, J=5.2 Hz, 1H), 4.44 (s, 2H), 3.80 (s, 2H), 2.95 (t, J=5.8 Hz, 2H), 2.71-2.66 (m, 2H). | Rt = 2.12 min, m/z 449.2 [M+H]⁺ (Method 1) |
| | | N-benzyl-6-(4-((1,2,3,4-tetrahydroisoquinolin-7-yl)oxy)-1H-pyrrolo[2,3-b]pyridin-3-yl)pyrimidin-4-amine | | |
| 26 | 35j | 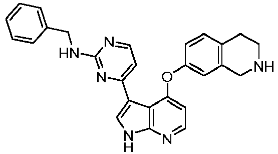 | ¹H NMR (400 MHz, DMSO) δ 12.31 (s, 1H), 8.16 (d, J=5.2 Hz, 1H), 8.10 (d, J=5.4 Hz, 1H), 8.03 (s, 1H), 7.47 (d, J=5.8 Hz, 1H), 7.35 (d, J=7.4 Hz, 2H), 7.32-7.26 (m, 3H), 7.22-7.14 (m, 2H), 6.96 (dd, J=2.5, 8.3 Hz, 1H), 6.89 (d, J=2.3 Hz, 1H), ;6.37 (d, J=5.4 Hz, 1H), 4.55 (d, J=6.2 Hz, 2H), 3.82 (s, 2H), 2.95 (t, J=5.8 Hz, 2H), 2.69 (t, J=5.8 Hz, 2H). | Rt = 2.32 min, m/z 449.4 [M+H]⁺ (Method 1) |
| | | N-benzyl-4-(4-((1,2,3,4-tetrahydroisoquinolin-7-yl)oxy)-1H-pyrrolo[2,3-b]pyridin-3-yl)pyrimidin-2-amine | | |
| 27 | 35k | 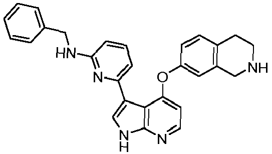 | ¹H NMR (400 MHz, DMSO) δ 11.96 (s, 1H), 8.05 (d, J=5.5 Hz, 1H), 7.74 (d, J=1.5 Hz, 1H), 7.36 (d, J=7.1 Hz, 2H), 7.32-7.27 (m, 3H), 7.20 (dd, J=7.3,14.6 Hz, 2H), 7.13 (d, J=8.3 Hz, 1H), 6.94-6.83 (m, 3H), 6.33-6.29 (m, 2H), 4.53 (d, J=5.9 Hz, 2H), 3.81 (s, 2H), 2.94 (t, J=5.8 Hz, 2H), 2.68 (t, J=5.7 Hz, 2H). | Rt = 2.16 min, m/z 448.4 [M+H]⁺ (Method 1) |
| | | N-benzyl-6-(4-((1,2,3,4-tetrahydroisoquinolin-7-yl)oxy)-1H-pyrrolo[2,3-b]pyridin-3-yl)pyridin-2-amine | | |
| 28 | 35l | 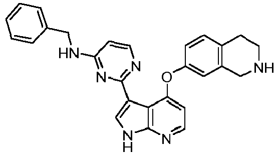 | ¹H NMR (400 MHz, DMSO) δ 12.07 (s, 1H), 8.07 (d, J=5.4 Hz, 1H), 8.02 (d, J=5.9 Hz, 1H), 7.91 (s, 1H), 7.48 (s, 1H), 7.26 (t, J=7.1 Hz, 2H), 7.22-7.12 (m, 3H), 7.04 (d, J=8.4 Hz, 1H), 6.79 (dd, J=2.2, 8.2 Hz, 1H), 6.70 (s, 1H), 6.35 (d, J=4.9 Hz, 1H), 6.28 (d, J=5.7 Hz, 1H), 4.50 (d, J=5.1 Hz, 2H), 3.71 (s, 2H), 2.91 (t, J=5.6 Hz, 2H), 2.63 (t, J=5.5 Hz, 2H). | Rt = 2.18 min, m/z 449.3 [M+H]⁺ (Method 1) |
| | | N-benzyl-2-(4-((1,2,3,4-tetrahydroisoquinolin- 7-yl)oxy)-1H-pyrrolo[2,3-b]pyridin-3-yl)pyrimidin-4-amine | | |
| 29 | 35m | 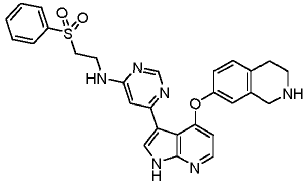 | ¹H NMR (400 MHz, DMSO) δ 12.25 (s, 1H), 8.32 (d, J=1.0 Hz, 1H), 8.10 (d, J=5.5 Hz, 1H), 8.03 (s, 1H), 7.88 (d, J=7.5 Hz, 2H), 7.72-7.68 (m, 1H), 7.61 (t, J=7.6 Hz, 2H), 7.26 (s, 1H), 7.14 (d, J=8.3 Hz, 1H), 7.09 (d, J=1.2 Hz, 1H), 6.94 (dd, J=2.6, 8.2 Hz, 1H), 6.88 (d, J=2.4 Hz, 1H), 6.37 (d, J=5.4 Hz, 1H), 3.82 (s, 2H), 3.52 (s, 4H), 2.94 (t, J=5.9 Hz, 2H), 2.71-2.66 (m, 2H). | Rt = 2.04 min, m/z 527.4 [M+H]⁺ (Method 1) |
| | | N-(2-(phenylsulfonyl)ethyl)-6-(4-((1,2,3,4-tetrahydroisoquinolin- 7-yl)oxy)-1H-pyrrolo[2,3-b]pyridin-3-yl)pyrimidin-4-amine | | |
| 30 | 35n | 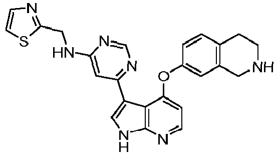 | ¹H NMR (400 MHz, DMSO) δ 12.30 (s, 1H), 8.40 (d, J=1.0 Hz, 1H), 8.11 (d, J=5.4 Hz, 2H), 8.07 (s, 1H), 7.70 (d, J=3.2 Hz, 1H), 7.55 (d, J=3.2 Hz, 1H), 7.29 (d, J=1.0 Hz, 1H), 7.13 (d, J=8.3 Hz, 1H), 6.96 (dd, J=2.2, 8.2 Hz, 1H), 6.89 (s, 1H), 6.39 (d, J=5.2 Hz, 1H), 4.75 (s, 2H), 3.82 (s, 2H), 2.95 (t, J=5.8 Hz, 2H), 2.68 (t, J=5.6 Hz, 2H). | Rt = 1.76 min, m/z 456.2 [M+H]+ (Method 1) |
| | | 6-(4-((1,2,3,4-tetrahydroisoquinolin-7-yl)oxy)-1H-pyrrolo[2,3-b]pyridin-3-yl)-N-(thiazol-2-ylmethyl)pyrimidin-4-amine | | |
| 31 | 35o | 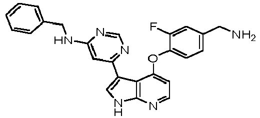 | ¹H NMR (400 MHz, DMSO) δ 12.33 (s, 1H), 8.37 (d, J=1.0 Hz, 1H), 8.11-8.07 (m, 2H), 7.79 (s, 1H), 7.45 (dd, J=2.0, 11.9 Hz, 1H), 7.36-7.18 (m, 8H), 6.26 (d, J=5.4 Hz, 1H), 4.45 (s, 2H), 3.83 (s, 2H). | Rt = 2.04 min, m/z 441.3 [M+H]⁺ (Method 1) |
| | | 6-(4-(4-(aminomethyl)-2-fluorophenoxy)-1H-pyrrolo[2,3-b]pyridin-3-yl)-N-benzylpyrimidin-4-amine | | |
| 32 | 35p | 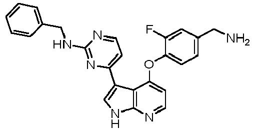 | ¹H NMR (400 MHz, DMSO) δ 8.18 (d, J=5.2 Hz, 1H), 8.12 (d, J=5.4 Hz, 1H), 8.04 (s, 1H), 7.50-7.41 (m, 2H), 7.36-7.19 (m, 8H), 6.31 (d, J=5.2 Hz, 1H), 4.56 (d, J=6.2 Hz, 2H), 3.75 (s, 2H). | Rt = 2.26 min, m/z 441.3 [M+H]⁺ (Method 1) |
| | | 4-(4-(4-(aminomethyl)-2-fluorophenoxy)-1H-pyrrolo[2,3-b]pyridin-3-yl)-N-benzylpyrimidin-2-amine | | |
| 33 | 35q | 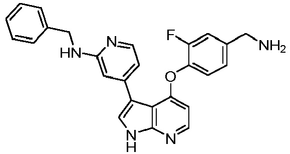 | ¹H NMR (400 MHz, DMSO) δ 12.15 (s, 1H), 8.09 (d, J=5.4 Hz, 1H), 7.88 (d, J=5.4 Hz, 1H), 7.70 (s, 1H), 7.42 (dd, J=2.1, 12.4 Hz, 1H), 7.34-7.23 (m, 6H), 7.20-7.15 (m, 1H), 6.92-6.84 (m, 2H), 6.82 (s, 1H), 6.23 (dd, J=0.9, 5.5 Hz, 1H), 4.44 (d, J=6.2 Hz, 2H), 3.76 (s, 2H). | Rt = 2.04 min, m/z 440.3 [M+H]⁺ (Method 1) |
| | | 4-(4-(4-(aminomethyl)-2-fluorophenoxy)-1H-pyrrolo[2,3-b]pyridin-3-yl)-N-benzylpyridin-2-amine | | |
| 34 | 35r | 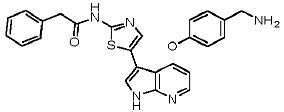 | ¹H NMR (400 MHz, DMSO) δ 12.09 (s, 1H), 8.08 (d, J=5.4 Hz, 1H), 7.65 (d, J=8.1 Hz, 2H), 7.42 (d, J=8.5 Hz, 2H), 7.32 (d, J=4.6 Hz, 6H), 7.14 (d, J=8.4 Hz, 2H), 6.27 (d, J=5.4 Hz, 1H), 3.74 (s, 4H). | Rt = 2.63 min, m/z 456.2 [M+H]⁺ (Method 1) |
| | | N-(5-(4-(4-(aminomethyl)phenoxy)-1H-pyrrolo[2,3-b]pyridin-3-yl)thiazol-2-yl)-2-phenylacetamide | | |
| 35 | 35s | 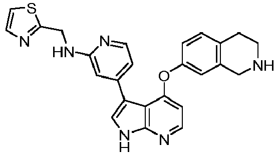 | "¹H NMR (400 MHz, DMSO) δ 12.14 (s, 1H), 8.10 (d, J=5.5 Hz, 1H), 7.89 (d, J=6.0 Hz, 1H), 7.72 (s, 1H), 7.69 (d, J=3.2 Hz, 1H), 7.51 (d, J=3.3 Hz, 1H), 7.28 (t, J=6.2 Hz, 1H), 7.13 (d, J=8.3 Hz, 1H), 6.95 (dd, J=3.2, 8.2 Hz, 1H), 6.93-6.87 (m, 3H), 6.33 (d, J=5.4 Hz, 1H), 4.73 (d, J=6.2 Hz, 2H), 3.81 (s, 2H), 2.94 (t, J=5.8 Hz, 2H), 2.68 (t, J=5.5 Hz, 2H). | Rt = 1.81 min, m/z 455.4 [M+H]⁺ (Method 1) |
| | | 4-(4-((1,2,3,4-tetrahydroisoquinolin- 7-yl)oxy)-1H-pyrrolo[2,3-b]pyridin-3-yl)-N-(thiazol-2-ylmethyl)pyridin-2-amine | | |
| 36 | 35t | 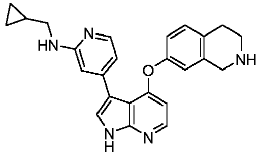 | ¹H NMR (400 MHz, DMSO) δ 12.11 (s, 1H), 8.09 (d, J=5.4 Hz, 1H), 7.86-7.84 (m, 1H), 7.70 (s, 1H), 7.13 (d, J=8.4 Hz, 1H), 6.93 (dd, J=2.5, 8.2 Hz, 1H), 6.88 (d, J=2.4 Hz, 1H), 6.81-6.79 (m, 2H), 6.32 (t, J=5.3 Hz, 2H), 3.81 (s, 2H), 3.04-2.91 (m, 4H), 2.68 (t, J=5.5 Hz, 2H), 1.05-0.97 (m, 1H), 0.37 (ddd, J=4.1, 5.8, 8.0 Hz, 2H), 0.16-0.11 (m, 2H). | Rt = 1.94 min, m/z 412.3 [M+H]⁺ (Method 1) |
| | | N-(cyclopropylmethyl)-4-(4-((1,2,3,4-tetrahydroisoquinolin- 7-yl)oxy)-1H-pyrrolo[2,3-b]pyridin-3-yl)pyridin-2-amine | | |
| 37 | 35u | 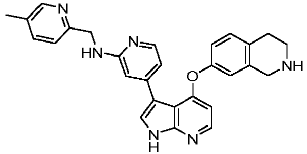 | ¹H NMR (400 MHz, DMSO) δ 12.11 (s, 1H), 8.31 (d, J=1.5 Hz, 1H), 8.09 (d, J=5.4 Hz, 1H), 7.85 (d, J=5.4 Hz, 1H), 7.70 (s, 1H), 7.47 (dd, J=1.9, 8.0 Hz, 1H), 7.15 (dd, J=8.1,14.6 Hz, 2H), 6.97-6.87 (m, 4H), 6.85 (dd, J=1.4, 5.3 Hz, 1H), 6.32 (d, J=5.5 Hz, 1H), 4.47 (d, J=6.0 Hz, 2H), 3.81 (s, 2H), 2.94 (t, J=5.8 Hz, 2H), 2.67 (t, J=5.5 Hz, 2H), 2.24 (s, 3H). | Rt = 3.42 min, m/z 463.4 [M+H]⁺ (Method 3) |
| | | N-((5-methylpyridin-2-yl)methyl)-4-(4-((1,2,3,4-tetrahydroisoquinolin-7-yl)oxy)-1H-pyrrolo[2,3-b]pyridin-3-yl)pyridin-2-amine | | |
| 38 | 35v | 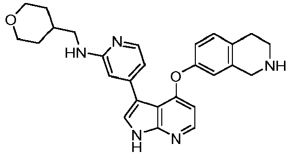 | ¹H NMR (400 MHz, DMSO) δ 12.10 (s, 1H), 8.09 (d, J=5.4 Hz, 1H), 7.85 (d, J=5.4 Hz, 1H), 7.71 (s, 1H), 7.13 (d, J=8.3 Hz, 1H), 6.94 (dd, J=2.5, 8.1 Hz, 1H), 6.88 (d, J=2.3 Hz, 1H), 6.82-6.77 (m, 2H), 6.36-6.33 (m, 2H), 3.83-3.77 (m, 4H), 3.23-3.16 (m, 2H), 3.04-2.91 (m, 4H), 2.68 (t, J=5.4 Hz, 2H), 1.78-1.70 (m, 1H), 1.57-1.54 (m, 2H), 1.15-1.04 (m, 2H). | Rt = 3.26 min, m/z 456.4 [M+H]⁺ (Method 1) |
| | | N-((tetrahydro-2H-pyran-4-yl)methyl)-4-(4-((1,2,3,4-tetrahydroisoquinolin-7-yl)oxy)-1H-pyrrolo[2,3-b]pyridin-3-yl)pyridin-2-amine | | |
| 39 | 35w | 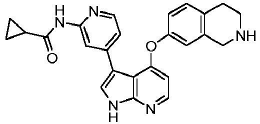 | ¹H NMR (400 MHz, DMSO) δ 12.17 (s, 1H), 10.65 (s, 1H), 8.51 (s, 1H), 8.19 (d, J=5.3 Hz, 1H), 8.11 (d, J=5.5 Hz, 1H), 7.77 (s, 1H), 7.36 (dd, J=1.6, 5.2 Hz, 1H), 7.11 (d, J=8.2 Hz, 1H), 6.92-6.85 (m, 2H), 6.35 (d, J=5.4 Hz, 1H), 3.80 (s, 2H), 2.93 (t, J=5.8 Hz, 2H), 2.66 (t, J=5.6 Hz, 2H), 2.08-1.97 (m, 1H), 0.82-0.78 (m, 4H). | Rt = 1.98 min, m/z 426.4 [M+H]⁺ (Method 1) |
| | | N-(4-(4-((1,2,3,4-tetrahydroisoquinolin-7-yl)oxy)-1H-pyrrolo[2,3-b]pyridin-3-yl)pyridin-2-yl)cyclopropanecarboxamide | | |
| 40 | 35x | 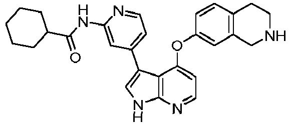 | ¹H NMR (400 MHz, DMSO) 12.24 (s, 1H), 10.22 (s, 1H), 8.52 (s, 1H), 8.25 (s, 0.8H, formic acid), 8.18 (d, J=5.2 Hz, 1H), 8.13 (d, J=5.4 Hz, 1H), 7.80 (s, 1H), 7.35 (dd, J=1.6, 5.2 Hz, 1H), 7.13 (d, J=8.2 Hz, 1H), 6.96-6.91 (m, 2H), 6.38 (d, J=5.4 Hz, 1H), 3.88 (s, 2H), 3.01 (t, J=5.9 Hz, 2H), 2.75-2.67 (m, 2H), 1.77-1.73 (m, 4H), 1.66-1.63 (m, 1H), 1.43-1.34 (m, 2H), 1.26-1.20 (m, 3H). | Rt = 2.61 min, m/z 468.4 [M+H]⁺ (Method 1) |
| | | N-(4-(4-((1,2,3,4-tetrahydroisoquinolin-7-yl)oxy)-1H-pyrrolo[2,3-b]pyridin-3-yl)pyridin-2-yl)cyclohexanecarboxamide | | |
| 41 | 35y | 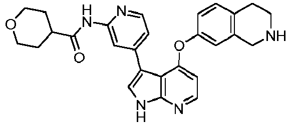 | ¹H NMR (400 MHz, DMSO) δ 12.25 (s, 1H), 10.34 (s, 1H), 8.52 (d, J=0.7 Hz, 1H), 8.25 (s, 1H, formic acid), 8.19 (d, J=5.2 Hz, 1H), 8.13 (d, J=5.5 Hz, 1H), 7.81 (s, 1H), 7.37 (dd, J=1.6, 5.2 Hz, 1H), 7.15 (d, J=8.2 Hz, 1H), 6.97-6.92 (m, 2H), 6.38 (d, J=5.5 Hz, 1H), 3.91-3.87 (m, 4H), 3.36-3.28 (m, 2H), 3.02 (t, J=5.9 Hz, 2H), 2.78-2.70 (m, 3H), 1.68-1.61 (m, 4H). | Rt = 2.09 min, m/z 470.4 [M+H]⁺ (Method 1) |
| | | N-(4-(4-((1,2,3,4-tetrahydroisoquinolin- 7-yl)oxy)-1H-pyrrolo[2,3-b]pyridin-3-yl)pyridin-2-yl)tetrahydro-2H-pyran-4-carboxamide | | |
| 42 | 35z | 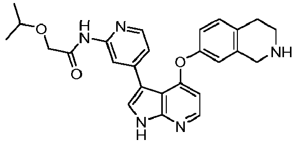 | ¹H NMR (400 MHz, DMSO) δ 12.28 (s, 1H), 9.54 (s, 1H), 8.50 (s, 1H), 8.21 (d, J=5.5 Hz, 1H), 8.13 (d, J=5.5 Hz, 1H), 7.82 (s, 1H), 7.43 (dd, J=1.6, 5.3 Hz, 1H), 7.14 (d, J=8.3 Hz, 1H), 6.96 (dd, J=2.5, 8.2 Hz, 1H), 6.91 (d, J=2.3 Hz, 1H), 6.36 (d, J=5.4 Hz, 1H), 4.08 (s, 2H), 3.87 (s, 2H), 3.74-3.66 (m, 1H), 3.01 (t, J=5.8 Hz, 2H), 2.72 (t, J=5.7 Hz, 2H), 1.17 (d, J=6.1 Hz, 6H). | Rt = 2.56 min, m/z 458.4 [M+H]⁺ (Method 1) |
| | | 2-isopropoxy-N-(4-(4-((1,2,3,4-tetrahydroisoquinolin-7-yl)oxy)-1H-pyrrolo[2,3-b]pyridin-3-yl)pyridin-2-yl)acetamide | | |
| 43 | 35aa | 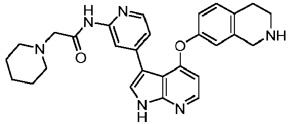 | ¹H NMR (400 MHz, DMSO) δ 12.26 (s, 1H), 9.77 (s, 1H), 8.51 (d, J=0.9 Hz, 1H), 8.20 (d, J=5.7 Hz, 1H), 8.12 (d, J=5.5 Hz, 1H), 7.81 (s, 1H), 7.42 (dd, J=1.6, 5.2 Hz, 1H), 7.11 (d, J=8.2 Hz, 1H), 6.92 (dd, J=2.6, 8.3 Hz, 1H), 6.87 (d, J=2.4 Hz, 1H), 6.36 (d, J=5.4 Hz, 1H), 3.79 (s, 2H), 3.11 (s, 2H), 2.93 (t, J=5.8 Hz, 2H), 2.68-2.64 (m, 2H), 2.48-2.46 (m, 4H), 1.57-1.51 (m, 4H), 1.41 (d, J=5.3 Hz, 2H). | Rt = 1.82 min, m/z 483.4 [M+H]⁺ (Method 1) |
| | | 2-(piperidin-1-yl)-N-(4-(4-((1,2,3,4-tetrahydroisoquinolin- 7-yl)oxy)-1H-pyrrolo[2,3-b]pyridin-3-yl)pyridin-2-yl)acetamide | | |
| 44 | 35bb | 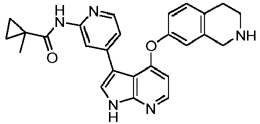 | ¹H NMR (400 MHz, DMSO) δ 12.24 (s, 1H), 9.36 (s, 1H), 8.46 (d, J=0.9 Hz, 1H), 8.21 (d, J=5.2 Hz, 1H), 8.12 (d, J=5.5 Hz, 1H), 7.81 (s, 1H), 7.41 (dd, J=1.6, 5.2 Hz, 1H), 7.12 (d, J=8.2 Hz, 1H), 6.93-6.87 (m, 2H), 6.36 (d, J=5.4 Hz, 1H), 3.82 (s, 2H), 2.94 (t, J=5.8 Hz, 2H), 2.68 (t, J=5.7 Hz, 2H), 1.42 (s, 3H), 1.13-1.09 (m, 2H), 0.68-0.64 (m, 2H). | Rt = 2.30 min, m/z 440.1 [M+H]⁺ (Method 1) |
| | | 1-methyl-N-(4-(4-((1,2,3,4-tetrahydroisoquinolin- 7-yl)oxy)-1H-pyrrolo[2,3-b]pyridin-3-yl)pyridin-2-yl)cyclopropanecarboxamide | | |
| 45 | 35cc | 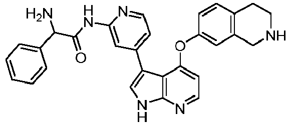 | ¹H NMR (400 MHz, DMSO) δ 12.23 (S, 1H), 10.42 (s, 1H), 8.51 (s, 1H), 8.20 (dd J=5.3, 0.6 Hz, 1H), 8.12 (d J=5.7 Hz, 1H), 7.79 (s, 1H), 7.47-7.42 (m, 2H), 7.39 (dd J=5.2, 1.7Hz, 1H), 7.33-7.22 (m, 3H), 7.08 (d J=8.3Hz, 1H), 6.91-6.84 (m, 2H), 6.35 (d J=5.5Hz, 1H), 4.64 (s, 1H), 3.84 (s, 2H), 3.01 (t J=5.8 Hz, 2H), 2.72 (t J=5.8 Hz, 2H). | Rt = 2.00 min, m/z 491.1 [M+H⁺ (Method 1) |
| | | 2-amino-2-phenyl-N-(4-(4-((1,2,3,4-tetrahydroisoquinolin-7-yl)oxy)-1H-pyrrolo[2,3-b]pyridin-3-yl)pyridin-2-yl)acetamide | | |
| 46 | 35dd | 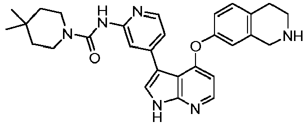 | ¹H NMR (400 MHz, DMSO) δ 12.17 (s, 1H), 8.92 (s, 1H), 8.20 (d, J=0.9 Hz, 1H), 8.11 (d, J=5.5 Hz, 2H), 7.76 (s, 1H), 7.27 (dd, J=1.6,5.2 Hz, 1H), 7.11 (d, J=8.2 Hz, 1H), 6.95-6.88 (m, 2H), 6.34 (d, J=5.4 Hz, 1H), 3.81 (s, 2H), 3.46-3.40 (m, 4H), 2.94 (t, J=5.9 Hz, 2H), 2.70-2.65 (m, 2H), 1.29-1.24 (m, 4H), 0.93 (s, 6H). | Rt = 2.55 min, m/z 497.3 [M+H]⁺ (Method 1) |
| | | 4,4-dimethyl-N-(4-(4-((1,2,3,4-tetrahydroisoquinolin- 7-yl)oxy)-1H-pyrrolo[2,3-b]pyridin-3-yl)pyridin-2-yl)piperidine-1-carboxamide | | |
| 47 | 35ee | 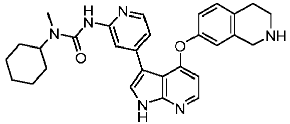 | ¹H NMR (400 MHz, DMSO) 8 12.18 (s, 1H), 8.59 (s, 1H), 8.23 (s, 1H), 8.11 (d, J=5.5 Hz, 2H), 7.76 (s, 1H), 7.27 (dd, J=1.6, 5.3 Hz, 1H), 7.10 (d, J=8.2 Hz, 1H), 6.94-6.88 (m, 2H), 6.35 (d, J=5.4 Hz, 1H), 4.06-3.99 (m, 1H), 3.82 (s, 2H), 2.94 (t, J=5.8 Hz, 2H), 2.81 (s, 3H), 2.69-2.66 (m, 2H), 1.77-1.74 (m, 2H), 1.62-1.28 (m, 7H), 1.14-1.03 (m, 1H). | Rt = 2.56 min, m/z 497.4 [M+H]⁺ (Method 1) |
| | | 1-cyclohexyl-1-methyl-3-(4-(4-((1,2,3,4-tetrahydroisoquinolin-7-yl)oxy)-1H-pyrrolo[2,3-b]pyridin-3-yl)pyridin-2-yl)urea | | |
| 48 | 35ff | 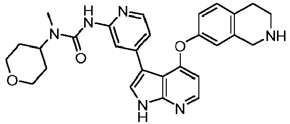 | ¹H NMR (400 MHz, DMSO) 8 12.18 (s, 1H), 8.72 (s, 1H), 8.24 (s, 1H), 8.13-8.10 (m, 2H), 7.77 (s, 1H), 7.28 (dd, J=1.6, 5.2 Hz, 1H), 7.11 (d, J=8.2 Hz, 1H), 6.94-6.88 (m, 2H), 6.35 (d, J=5.4 Hz, 1H), 4.32-4.24 (m, 1H), 3.91 (dd, J=4.4, 11.1 Hz, 2H), 3.81 (s, 2H), 3.42-3.35 (m, 2H), 2.94 (t, J=5.9 Hz, 2H), 2.83 (s, 3H), 2.67 (t, J=5.6 Hz, 2H), 1.76-1.66 (m, 2H), 1.45 (dd, J=2.3, 12.2 Hz, 2H). | Rt = 2.04 min, m/z 521.2 [M+Na]+ (Method 1) |
| | | 1-methyl-1-(tetrahydro-2H-pyran-4-yl)-3-(4-(4-((1,2,3,4-tetrahydroisoquinolin-7-yl)oxy)-1H-pyrrolo[2,3-b]pyridin-3-yl)pyridin-2-yl)urea | | |

### Example 49

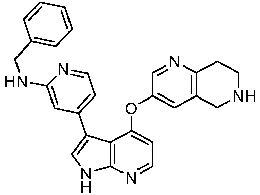

### N-Benzyl-4-(4-((5,6,7,8-tetrahydro-1,6-naphthyridin-3-yl)oxy)-1H-pyrrolo[2,3-b]pyridin-3-yl)pyridin-2-amine

A solution of Intermediate 22j and lithium hydroxide monohydrate (0.015 g, 0.366 mmol) in THF (4 mL), methanol (4 mL) and water (2 mL) was stirred at ambient temperature for 1 h. the reaction mixture was diluted with brine and the product was extracted into ethyl acetate (3 x 50 mL). The combined extracts were dried (Na₂SO₄) and evaporated. The residue was then dissolved in DCM (2 mL) and trifluoroacetic acid (2 mL) and the resulting reaction mixture was stirred at ambient temperature for 2 h. The reaction mixture was diluted with methanol and passed down a 10 g SCX-2 cartridge eluting with methanol and then 2M methanolic ammonia. The solution was evaporated to give a residue which was purified by MDAP (acidic) to yield the product as a white solid (7.5 mg).
LCMS (Method 1): Rt = 1.88 min, m/z 449.3 [M+H]⁺

¹H NMR (400 MHz, DMSO) d 12.18 (s, 1H), 8.27 (d, J=2.8 Hz, 1H), 8.23 (s, 1H), 8.13 (d, J=5.5 Hz, 1H), 7.86 (d, J=5.4 Hz, 1H), 7.72 (d, J=2.4 Hz, 1H), 7.32 (d, J=2.6 Hz, 1H), 7.30 - 7.23 (m, 4H), 7.21 - 7.15 (m, 1H), 6.93 (t, J=6.1 Hz, 1H), 6.84 - 6.78 (m, 2H), 6.40 (d, J=5.4 Hz, 1H), 4.45 (d, J=6.1 Hz, 2H), 3.84 (s, 2H), 3.04 (t, J=5.9 Hz, 2H), 2.79 (t, J=5.8 Hz, 2H).

### Example 50 to 56

The following examples were prepared in a similar manner from the Intermediate (I) indicated using a method similar to that used for Example 49.

| Ex. | I | Structure | NMR | LCMS |
|---|---|---|---|---|
| 50 | 15e | 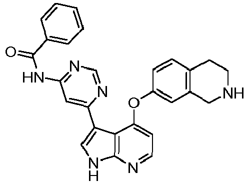 N-(6-(4-((1,2,3,4-tetrahydroisoquinolin- 7-yl)oxy)-1H-pyrrolo[2,3-b]pyridin-3-yl)pyrimidin-4-yl)benzamide | ¹H NMR (400 MHz, DMSO) δ 12.45 (s, 1H), 11.03 (s, 1H), 9.03 (d, J=1.3 Hz, 1H), 8.82 (d, J=1.2 Hz, 1H), 8.17-8.15 (m, 2H), 8.03-7.99 (m, 2H), 7.64-7.60 (m, 1H), 7.55-7.50 (m, 2H), 7.07 (d, J=8.0 Hz, 1H), 6.96-6.92 (m, 2H), 6.48 (d, J=5.5 Hz, 1H), 3.73 (s, 2H), 2.89 (t, J=5.8 Hz, 2H), 2.63 (t, J=5.9 Hz, 2H). | Rt = 2.61 min, m/z 463.3 [M+H]⁺ (Method 1) |
| 51 | 15f | 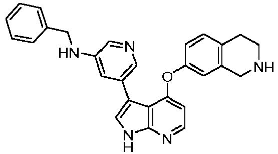 N-benzyl-5-(4-((1,2,3,4-tetrahydroisoquinolin- 7-yl)oxy)-1H-pyrrolo[2,3-b]pyridin-3-yl)pyridin-3-amine | ¹H NMR (400 MHz, DMSO) δ 12.03 (s, 1H), 8.08 (d, J=5.5 Hz, 1H), 8.04 (d, J=1.8 Hz, 1H), 7.83 (d, J=2.6 Hz, 1H), 7.58 (d, J=2.5 Hz, 1H), 7.30-7.28 (m, 2H), 7.28 (s, 2H), 7.24-7.19 (m, 1H), 7.18-7.16 (m, 2H), 7.16-7.13 (m, 1H), 6.97-6.91 (m, 1H), 6.89-6.85 (m, 1H), 6.38 (t, J=6.0 Hz, 1H), 6.30 (d, J=5.4 Hz, 1H), 4.19 (d, J=5.9 Hz, 2H), 3.86 (s, 2H), 3.03-2.93 (m, 2H), 2.76-2.69 (m, 2H). | Rt = 2.12 min, m/z 448.3 [M+H]⁺ (Method 1) |
| 52 | 15g | 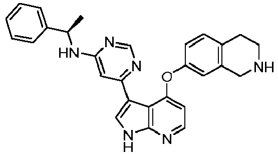 (R)-N-(1-phenylethyl)-6-(4-((1,2,3,4-tetrahydroisoquinolin- 7-yl)oxy)-1H-pyrrolo[2,3-b]pyridin-3-yl)pyrimidin-4-amine | ¹H NMR (400 MHz, DMSO) δ 12.26 (s, 1H), 8.30 (s, 1H), 8.13 (d, J=5.4 Hz, 1H), 7.99 (s, 1H), 7.69 (d, J=8.1 Hz, 1H), 7.24-7.12 (m, 7H), 6.97 (dd, J=2.4, 8.2 Hz, 1H), 6.91-6.88 (m, 1H), 6.47 (d, J=5.3 Hz, 1H), 5.10 (s, 1H), 3.83 (s, 2H), 2.98-2.93 (m, 2H), 2.72-2.66 (m, 2H), 2.54-2.54 (m, 1H), 1.37 (s, 3H). | Rt = 2.24 min, m/z 463.3 [M+H]⁺ (Method 1) |
| 53 | 15i | 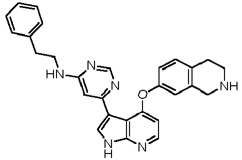 N-phenethyl-6-(4-((1,2,3,4-tetrahydroisoquinolin- 7-yl)oxy)-1H-pyrrolo[2,3-b]pyridin-3-yl)pyrimidin-4-amine | ¹H NMR (400 MHz, DMSO) δ 12.25 (s, 1H), 8.34 (s, 1H), 8.11 (d, J=5.4 Hz, 1H), 8.04 (s, 1H), 7.27-7.07 (m, 9H), 6.96-6.87 (m, 2H), 6.37 (d, J=5.2 Hz, 1H), 3.79 (s, 2H), 3.56-3.38 (m, 2H), 2.93 (t, J=5.8 Hz, 2H), 2.77 (s, 2H), 2.68 (t, J=5.2 Hz, 2H). | Rt = 2.28 min, m/z 463.3 [M+H]⁺ (Method 1) |
| 54 | 15k | 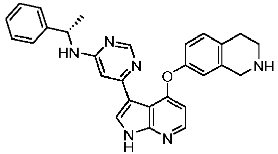 (S)-N-(1-phenylethyl)-6-(4-((1,2,3,4-tetrahydroisoquinolin- 7-yl)oxy)-1H-pyrrolo[2,3-b]pyridin-3-yl)pyrimidin-4-amine | ¹H NMR (400 MHz, DMSO) δ 12.25 (s, 1H), 8.30 (s, 1H), 8.13 (d, J=5.4 Hz, 1H), 7.99 (s, 1H), 7.68 (d, J=7.9 Hz, 1H), 7.25-7.12 (m, 7H), 6.97 (dd, J=2.3, 8.3 Hz, 1H), 6.89 (d, J=2.3 Hz, 1H), 6.46 (d, J=5.4 Hz, 1H), 5.18 (s, 1H), 3.82 (s, 2H), 3.62-3.42 (m, 1H), 2.95 (t, J=5.8 Hz, 2H), 2.71-2.66 (m, 2H), 1.37 (s, 3H). | Rt = 2.27 min, m/z 463.3 [M+H]⁺ (Method 1) |
| 55 | 15l | 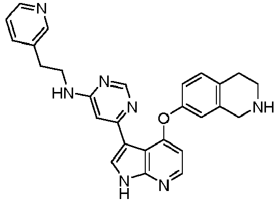 N-(2-(pyridin-3-yl)ethyl)-6-(4-((1,2,3,4-tetrahydroisoquinolin- 7-yl)oxy)-1H-pyrrolo[2,3-b]pyridin-3-yl)pyrimidin-4-amine | ¹H NMR (400 MHz, DMSO) δ 12.24 (s, 1H), 8.47-8.45 (m, 1H), 8.34 (s, 1H), 8.11 (d, J=5.4 Hz, 1H), 8.04 (s, 1H), 7.64 (dt, J=1.8, 7.6 Hz, 1H), 7.22-7.17 (m, 4H), 7.07 (d, J=6.3 Hz, 1H), 6.95-6.87 (m, 2H), 6.37 (d, J=5.4 Hz, 1H), 3.84-3.70 (m, 2H), 3.68-3.39 (m, 2H), 2.97-2.89 (m, 4H), 2.70-2.65 (m, 2H). | Rt = 3.03 min, 464.4 m/z [M+H]⁺ (Method 3) |
| 56 | 22h | 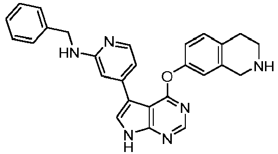 N-benzyl-4-(4-((1,2,3,4-tetrahydroisoquinolin- 7-yl)oxy)-7H-pyrrolo[2,3-d]pyrimidin-5-yl)pyridin-2-amine | ¹H NMR (400 MHz, DMSO) 8 12.51 (s, 1H), 8.30 (s, 1H), 7.92 (d, J=6.1 Hz, 1H), 7.79 (s, 1H), 7.29-7.26 (m, 5H), 7.22-7.15 (m, 1H), 7.11 (d, J=8.3 Hz, 1H), 7.01-6.93 (m, 2H), 6.92-6.90 (m, 3H), 4.46 (d, J=6.1 Hz, 2H), 3.82 (s, 2H), 2.96 (t, J=5.8 Hz, 2H), 2.70 (t, J=5.6 Hz, 2H). | Rt = 2.05 min, m/z 449.1 [M+H]⁺ (Method 2) |

### PHARMACOLOGICAL ACTIVITY OF THE COMPOUNDS OF THE INVENTION.

### In vitro inhibitory activity assay description

The effectiveness of compounds of the present invention to inhibit Rho kinase activity can be determined in a 10µl assay containing 40mM Tris pH7.5, 20mM MgCl₂ 0.1mg/ml BSA, 50µM DTT and 2.5µM peptide substrate (Myelin Basic Protein) using an ADP-Glo kit (Promega). Compounds were dissolved in DMSO such that the final concentration of DMSO was 1% in the assay. All reactions/incubations are performed at 25°C. Compound (2ul) and either Rho kinase 1 or 2 (4µl) were mixed and incubated for 30 mins. Reactions were initiated by addition of ATP (4µl) such that the final concentration of ATP in the assay was 10µM. After a 1 hour incubation 10µl of ADP-Glo Reagent was added and after a further 45 minute incubation 20ul of Kinase Detection Buffer was added and the mixture incubated for a further 30 minutes. The luminescent signal was measured on a luminometer. Controls consisted of assay wells that did not contain compound with background determined using assay wells with no enzyme added. Compounds were tested in dose-response format and the inhibition of kinase activity was calculated at each concentration of compound. To determine the IC₅₀ (concentration of compound required to inhibit 50% of the enzyme activity) data were fit to a plot of % inhibition vs Log₁₀ compound concentration using a sigmoidal fit with a variable slope and fixing the maximum to 100% and the minimum to 0%. To determine the Ki values the Cheng-Prusoff equation was utilized (Ki=IC₅₀/(1+[S]/Km).

Compounds according to the invention showed Ki values lower than 5 µM and for most of the compounds of the invention Ki is even lower that 500 nM.

The results for individual compounds are provided below in Table 2 and are expressed as range of activity.

**Table 2**

| Example | Activity ROCK 1 | Activity ROCK 2 |
|---|---|---|
| 1 | +++ | +++ |
| 2 | + | + |
| 3 | + | + |
| 4 | +++ | +++ |
| 5 | +++ | +++ |
| 6 | ++ | +++ |
| 7 | +++ | +++ |
| 8 | +++ | +++ |
| 9 | ++ | +++ |
| 10 | +++ | +++ |
| 11 | +++ | +++ |
| 12 | ++ | +++ |
| 13 | ++ | +++ |
| 14 | +++ | +++ |
| 15 | ++ | +++ |
| 16 | +++ | +++ |
| 17 | +++ | +++ |
| 18 | +++ | +++ |
| 19 | +++ | +++ |
| 20 | ++ | +++ |
| 21 | +++ | +++ |
| 22 | +++ | +++ |
| 23 | ++ | +++ |
| 24 | ++ | +++ |
| 25 | +++ | +++ |
| 26 | +++ | +++ |
| 27 | + | + |
| 28 | + | + |
| 29 | +++ | +++ |
| 30 | +++ | +++ |
| 31 | +++ | +++ |
| 32 | ++ | +++ |
| 33 | ++ | +++ |
| 34 | ++ | +++ |
| 35 | +++ | +++ |
| 36 | +++ | +++ |
| 37 | ++ | +++ |
| 38 | +++ | +++ |
| 39 | ++ | +++ |
| 40 | +++ | +++ |
| 41 | +++ | +++ |
| 42 | +++ | +++ |
| 43 | +++ | +++ |
| 44 | ++ | +++ |
| 45 | +++ | +++ |
| 46 | ++ | +++ |
| 47 | +++ | +++ |
| 48 | ++ | +++ |
| 49 | + | + |
| 50 | ++ | +++ |
| 51 | ++ | +++ |
| 52 | +++ | +++ |
| 53 | +++ | +++ |
| 54 | ++ | +++ |
| 55 | +++ | +++ |
| 56 | ++ | +++ |

wherein the compounds are classified in term of potency with respect to their inhibitory activity on ROCK-I and ROCK-II isoforms according to the following classification criterion:
+ + + : Ki < 3nM
+ + : Ki in the range 3nM - 30nM
+ : Ki > 30nM

## Claims

1. Compounds of formula (I) 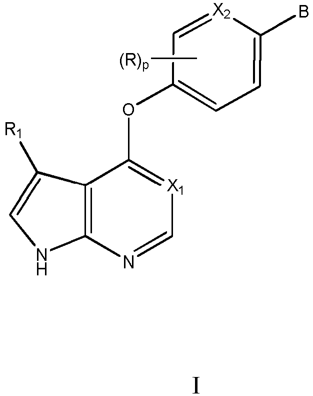 wherein
**X₁**, and **X₂** are in each occurrence independently a CH group or a nitrogen atom. p is zero or an integer from 1 to 3;
each R is independently H or halogen;
**R₁** is a group of formula K 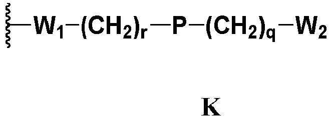
wherein r is 0 or an integer from 1 to 4; q is 0 or an integer from 1 to 4;
W₁ is an arylene or heteroarylene divalent group selected from A1-A11 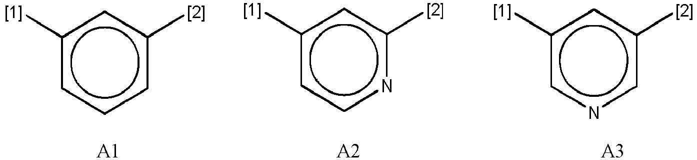 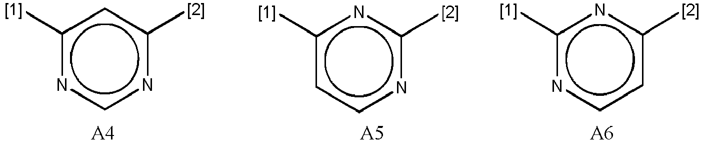 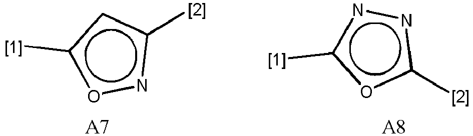 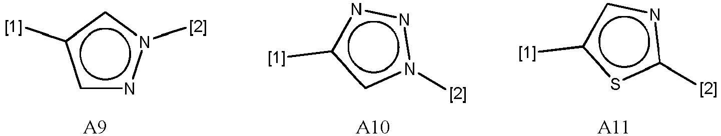
wherein [1] is the point of attachment of W1 to the rest of the molecule and [2] is the point of attachment to -(CH₂)r-;
**P** is absent or is a divalent group selected from O, S, SO, SO₂, CO, NR₆, N(R₆)(CH₂)nSO₂, N(R₆)COO, N(R₆)(CH₂)ₙC(O), N(R₆)(CH₂)ₙO, SO₂N(R₆), OC(O)N(R₆) and C(O)N(R₆), N(R₆)C(O) N(R₆);
**W₂** is H or selected from (C₁-C₆) alkyl, (C₃-C₈)cycloalkyl, (C₃-C₈)heterocycloalkyl, aryl, aryl(C₁-C₆)alkyl and heteroaryl; optionally substituted by one or more substituents selected independently from halogen atoms, -OH, oxo (=O), -SH, -NO₂, -CN, -CON(R₆)₂, -C(O)R₆, -NR₆ C(O)CH₃, -NH₂, -NHCOR₆, -CO₂R₆, -SO₂N(R₆)₂, -NR₆ SO₂CH₃, (C₁-C₆)alkyl, (C₁-C₆)haloalkyl, (C₁-C₁₀)alkoxy, (C₃-C₈)cycloalkyl and (C₃-C₆)cycloalkylcarbonyl;
n is at each occurrence independently 0 or an integer from 1 to 3;
**R₆** is at each occurrence independently H or selected from (C₁-C₆)alkyl, (C₁-C₆)haloalkyl, (C₂-C₆)alkynyl, (C₂-C₆)alkenyl, (C₃-C₈)cycloalkyl, heteroaryl and aryl optionally substituted by one or more substituents selected from halogen atoms, -OH, oxo (=O), -SH, -NO₂, -CN, -CONH₂, -COOH, (C₁-C₁₀)alkyl and (C₁-C₁₀)alkoxy;
B is a group of formula R₂R₃N-(C₁-C₆) alkyl, optionally substituted by one or more group selected from hydroxyl, (C₁-C₆) alkyl and (C₁-C₆) hydroxyalkyl;
wherein
R₂ and R₃, the same or different, are H or selected from (C₁-C₆) alkyl, (C₁-C₆) hydroxyalkyl,
or
B is a group of formula R₄R₃N-(C₁-C₆) alkyl, wherein
R₃ is H, or is selected from (C₁-C₆) alkyl, (C₁-C₆) hydroxyalkyl and R₄ is a divalent alkyl group -(CH₂)ₛ-, wherein s is an integer from 1 to 3, said divalent alkyl group being connected the carbon atom in ortho position on the adjacent ring to form a heterocyclic ring, preferably a 4 to 10 membered heterocyclic ring, fused with the adjacent ring; said heterocyclic ring being in its turn further optionally substituted with one or more group selected from (C₁-C₆) alkyl, (C₁-C₆) hydroxyalkyl;
and pharmaceutically acceptable salt and solvates thereof.

2. A compound according to Claim 1 wherein each of X₁ and X₂ is a CH ; represented by the formula IA: 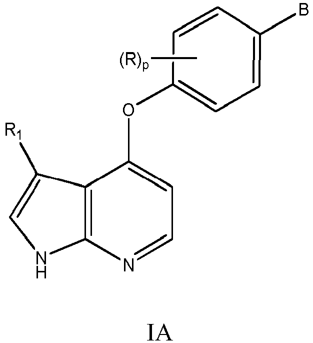 wherein B, R, R₁ and p are as defined in claim 1.

3. Compounds according to claim 1 wherein
B is a heterocyclic ring fused with the adjacent ring forming a bicyclic group selected from I1-I6 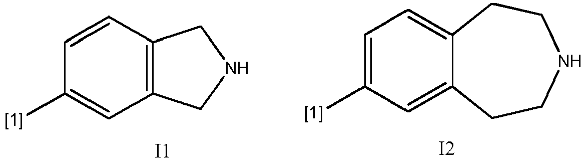 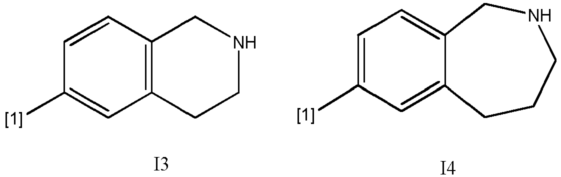 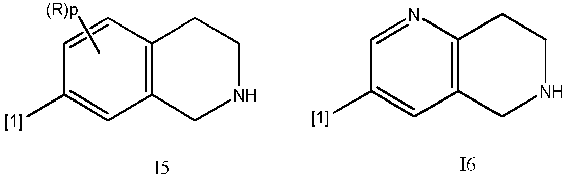
said heterocyclic ring being in its turn further optionally substituted with one or more group selected from (C₁-C₆) alkyl, (C₁-C₆) hydroxyalkyl;
and pharmaceutically acceptable salts and solvates thereof.

4. Compounds according to claim 3 wherein
B is a heterocyclic ring fused with the adjacent ring forming a bicyclic group (I5) represented by the formula IB 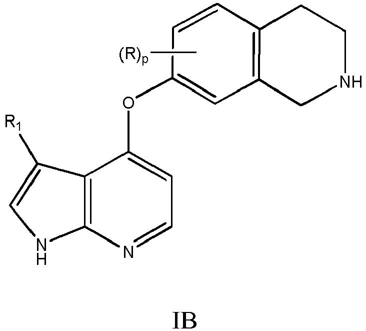
wherein R₁, R and p are as defined in claim 3,
and pharmaceutically acceptable salts and solvates thereof.

5. A compound according to claim 4 wherein
**R₁** is a group of formula **K** 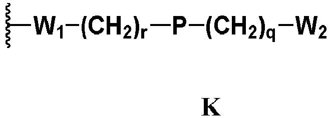
**r** is 0 or an integer from 1 to 4; **q** is 0 or an integer from 1 to 4;
W₁ is an arylene or heteroarylene divalent group selected from A2, A4 or A9; **P** is absent or is a divalent group selected from the O, S, SO, SO₂, CO, NR₆, N(R₆)(CH₂)nSO₂, N(R₆)COO, N(R₆)(CH₂)ₙC(O), N(R₆)(CH₂)ₙO, SO₂N(R₆), OC(O)N(R₆), C(O)N(R₆), and N(R₆)C(O) N(R₆);
**W₂** is H or selected from, (C₁-C₆) alkyl, (C₃-C₈)cycloalkyl, (C₃-C₈)heterocycloalkyl, aryl, aryl(C₁-C₆)alkyl and heteroaryl, optionally substituted by one or more substituents selected independently from halogen atoms, -OH, oxo (=O), -SH, -NO₂, -CN, -CON(R₆)₂, -C(O)R₆, -NR₆ C(O)CH₃, - NH₂, -NHCOR₆, -CO₂R₆, -SO₂N(R₆)₂, -NR₆ SO₂CH₃, (C₁-C₆)alkyl, (C₁-C₆)haloalkyl (C₁-C₁₀)alkoxy, (C₃-C₈)cycloalkyl, and (C₃-C₆)cycloalkylcarbonyl, (C₃-C₆)cycloalkyl-aminocarbonyl;
n is at each occurrence independently 0 or an integer from 1 to 3;
**R₆** is at each occurrence independently H or (C₁-C₆)alkyl;
and pharmaceutically acceptable salts and solvates thereof.

6. A compound according to claim 1, represented by the formula IC, 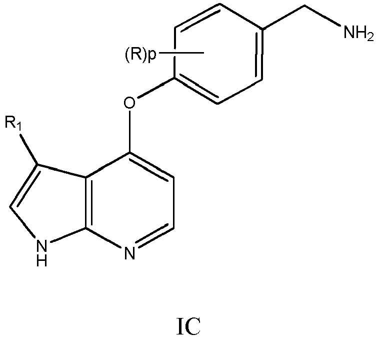 wherein R, R₁ and p are as defined in claim 1,
and pharmaceutically acceptable salts and solvates thereof.

7. A compound according to claim 6 wherein
each **R**, is halogen;
**R₁** is a group of formula **K** 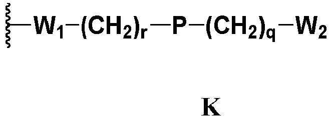
wherein **r** is 0; **q** is 0 or 1;
W₁ is an arylene or heteroarylene divalent group selected from A2, A4 or A9 **P** is absent or is selected from the divalent groups consisting of NR₆, N(R₆)(CH₂),SO₂, and N(R₆)(CH₂)ₙC(O);
**W₂** is selected from, (C₃-C₈)cycloalkyl, (C₃-C₈)heterocycloalkyl, aryl and heteroaryl, optionally substituted by one or more (C₁-C₆)alkyl;
n is at each occurrence independently 0 or an integer from 1 to 3;
**R₆** is at each occurrence independently H or (C₁-C₆)alkyl,
and pharmaceutically acceptable salts and solvates thereof.

8. A compound according to claim 1 selected from:
7-((3-(pyridin-4-yl)-1H-pyrrolo[2,3-b]pyridin-4-yl)oxy)-1,2,3,4-tetrahydroisoquinoline;
(4-((3-(5-(benzyloxy)pyridin-3-yl)-1H-pyrrolo[2,3-b]pyridin-4-yl)oxy)phenyl)methanamine;
(4-((3-(3-(benzyloxy)phenyl)-1H-pyrrolo[2,3-b]pyridin-4-yl)oxy)phenyl)methanamine;
6-(4-(4-(aminomethyl)-2,6-difluorophenoxy)-1H-pyrrolo[2,3-b]pyridin-3-yl)-N-(2-tosylethyl)pyrimidin-4-amine;
4-(4-(4-(aminomethyl)-2,6-difluorophenoxy)-7H-pyrrolo[2,3-d]pyrimidin-5-yl)-N-benzylpyridin-2-amine;
N-benzyl-4-(4-((6-fluoro-1,2,3,4-tetrahydroisoquinolin-7-yl)oxy)-7H-pyrrolo[2,3-d]pyrimidin-5-yl)pyridin-2-amine;
4-(4-(4-(aminomethyl)-2,6-difluorophenoxy)-1H-pyrrolo[2,3-b]pyridin-3-yl)-N-(pyridin-3-ylmethyl)pyridin-2-amine;
4-(4-(4-(aminomethyl)-2,6-difluorophenoxy)-1H-pyrrolo[2,3-b]pyridin-3-yl)-N-(cyclopropylmethyl)pyridin-2-amine;
N-benzyl-5-(4-((1,2,3,4-tetrahydroisoquinolin-7-yl)oxy)-1H-pyrrolo[2,3-b]pyridin-3 -yl)isoxazol-3-amine;
N-cyclopropyl-1-(4-(4-((1,2,3,4-tetrahydroisoquinolin-7-yl)oxy)-1H-pyrrolo[2,3-b]pyridin-3-yl)pyridin-2-yl)pyrrolidine-3-carboxamide;
N-(4-(4-(4-(aminomethyl)-2,6-difluorophenoxy)-1H-pyrrolo[2,3-b]pyridin-3-yl)pyridin-2-yl)cyclopropanecarboxamide;
N-benzyl-2-(4-(4-((1,2,3,4-tetrahydroisoquinolin-7-yl)oxy)-1H-pyrrolo[2,3-b]pyridin-3-yl)-1H-1,2,3-triazol-1-yl)acetamide;
N-benzyl-5-(4-((1,2,3,4-tetrahydroisoquinolin-7-yl)oxy)-1H-pyrrolo[2,3-b]pyridin-3 -yl)isoxazole-3-carboxamide;
2-phenyl-N-(5-(4-((1,2,3,4-tetrahydroisoquinolin-7-yl)oxy)-1H-pyrrolo[2,3-b]pyridin-3-yl)-1,3,4-oxadiazol-2-yl)acetamide;
4-(4-(4-(aminomethyl)phenoxy)-1H-pyrrolo[2,3-b]pyridin-3-yl)-N-benzylpyridin-2-amine;
N-(pyridin-3-ylmethyl)-4-(4-((1,2,3,4-tetrahydroisoquinolin-7-yl)oxy)-1H-pyrrolo[2,3-b]pyridin-3-yl)pyridin-2-amine;
4-(4-(4-((1,2,3,4-tetrahydroisoquinolin-7-yl)oxy)-1H-pyrrolo[2,3-b]pyridin-3-yl)pyridin-2-yl)morpholine;
(S)-N-((tetrahydrofuran-2-yl)methyl)-4-(4-((1,2,3,4-tetrahydroisoquinolin-7-yl)oxy)-1H-pyrrolo[2,3-b]pyridin-3-yl)pyridin-2-amine;
(R)-N-((tetrahydrofuran-2-yl)methyl)-4-(4-((1,2,3,4-tetrahydroisoquinolin-7-yl)oxy)-1H-pyrrolo[2,3-b]pyridin-3-yl)pyridin-2-amine;
N-(2-methoxyethyl)-4-(4-((1,2,3,4-tetrahydroisoquinolin-7-yl)oxy)-1H-pyrrolo[2,3-b]pyridin-3-yl)pyridin-2-amine;
7-((3-(1-methyl-1H-pyrazol-4-yl)-1H-pyrrolo[2,3-b]pyridin-4-yl)oxy)-1,2,3,4-tetrahydroisoquinoline;
(4-((3-(1-benzyl-1H-pyrazol-4-yl)-1H-pyrrolo[2,3-b]pyridin-4-yl)oxy)-3-fluorophenyl)methanamine;
(3-fluoro-4-((3-(1-(pyridin-4-ylmethyl)-1H-pyrazol-4-yl)-1H-pyrrolo[2,3-b]pyridin-4-yl)oxy)phenyl)methanamine;
(3-fluoro-4-((3-(1-((tetrahydro-2H-pyran-4-yl)methyl)-1H-pyrazol-4-yl)-1H-pyrrolo[2,3-b]pyridin-4-yl)oxy)phenyl)methanamine;
N-benzyl-6-(4-((1,2,3,4-tetrahydroisoquinolin-7-yl)oxy)-1H-pyrrolo[2,3-b]pyridin-3 -yl)pyrimidin-4-amine;
N-benzyl-4-(4-((1,2,3,4-tetrahydroisoquinolin-7-yl)oxy)-1H-pyrrolo[2,3-b]pyridin-3 -yl)pyrimidin-2-amine;
N-benzyl-6-(4-((1,2,3,4-tetrahydroisoquinolin-7-yl)oxy)-1H-pyrrolo[2,3-b]pyridin-3-yl)pyridin-2-amine;
N-benzyl-2-(4-((1,2,3,4-tetrahydroisoquinolin-7-yl)oxy)-1H-pyrrolo[2,3-b]pyridin-3 -yl)pyrimidin-4-amine;
N-(2-(phenylsulfonyl)ethyl)-6-(4-((1,2,3,4-tetrahydroisoquinolin-7-yl)oxy)-1H-pyrrolo[2,3-b]pyridin-3-yl)pyrimidin-4-amine;
6-(4-((1,2,3,4-tetrahydroisoquinolin-7-yl)oxy)-1H-pyrrolo[2,3-b]pyridin-3-yl)-N-(thiazol-2-ylmethyl)pyrimidin-4-amine;
6-(4-(4-(aminomethyl)-2-fluorophenoxy)-1H-pyrrolo[2,3-b]pyridin-3-yl)-N-benzylpyrimidin-4-amine;
4-(4-(4-(aminomethyl)-2-fluorophenoxy)-1H-pyrrolo[2,3-b]pyridin-3-yl)-N-benzylpyrimidin-2-amine;
4-(4-(4-(aminomethyl)-2-fluorophenoxy)-1H-pyrrolo[2,3-b]pyridin-3-yl)-N-benzylpyridin-2-amine;
N-(5-(4-(4-(aminomethyl)phenoxy)-1H-pyrrolo[2,3-b]pyridin-3-yl)thiazol-2-yl)-2-phenylacetamide;
4-(4-((1,2,3,4-tetrahydroisoquinolin-7-yl)oxy)-1H-pyrrolo[2,3-b]pyridin-3-yl)-N-(thiazol-2-ylmethyl)pyridin-2-amine;
N-(cyclopropylmethyl)-4-(4-((1,2,3,4-tetrahydroisoquinolin-7-yl)oxy)-1H-pyrrolo[2,3-b]pyridin-3-yl)pyridin-2-amine;
N-((5-methylpyridin-2-yl)methyl)-4-(4-((1,2,3,4-tetrahydroisoquinolin-7-yl)oxy)-1H-pyrrolo[2,3-b]pyridin-3-yl)pyridin-2-amine;
N-((tetrahydro-2H-pyran-4-yl)methyl)-4-(4-((1,2,3,4-tetrahydroisoquinolin-7-yl)oxy)-1H-pyrrolo[2,3-b]pyridin-3-yl)pyridin-2-amine;
N-(4-(4-((1,2,3,4-tetrahydroisoquinolin-7-yl)oxy)-1H-pyrrolo[2,3-b]pyridin-3-yl)pyridin-2-yl)cyclopropanecarboxamide;
N-(4-(4-((1,2,3,4-tetrahydroisoquinolin-7-yl)oxy)-1H-pyrrolo[2,3-b]pyridin-3-yl)pyridin-2-yl)cyclohexanecarboxamide;
N-(4-(4-((1,2,3,4-tetrahydroisoquinolin-7-yl)oxy)-1H-pyrrolo[2,3-b]pyridin-3-yl)pyridin-2-yl)tetrahydro-2H-pyran-4-carboxamide;
2-isopropoxy-N-(4-(4-((1,2,3,4-tetrahydroisoquinolin-7-yl)oxy)-1H-pyrrolo[2,3-b]pyridin-3-yl)pyridin-2-yl)acetamide;
2-(piperidin-1-yl)-N-(4-(4-((1,2,3,4-tetrahydroisoquinolin-7-yl)oxy)-1H-pyrrolo[2,3-b]pyridin-3-yl)pyridin-2-yl)acetamide;
1-methyl-N-(4-(4-((1,2,3,4-tetrahydroisoquinolin-7-yl)oxy)-1H-pyrrolo[2,3-b]pyridin-3-yl)pyridin-2-yl)cyclopropanecarboxamide;
2-amino-2-phenyl-N-(4-(4-((1,2,3,4-tetrahydroisoquinolin-7-yl)oxy)-1H-pyrrolo[2,3-b]pyridin-3-yl)pyridin-2-yl)acetamide;
4,4-dimethyl-N-(4-(4-((1,2,3,4-tetrahydroisoquinolin-7-yl)oxy)-1H-pyrrolo[2,3-b]pyridin-3-yl)pyridin-2-yl)piperidine-1-carboxamide;
1-cyclohexyl-1-methyl-3-(4-(4-((1,2,3,4-tetrahydroisoquinolin-7-yl)oxy)-1H-pyrrolo[2,3-b]pyridin-3-yl)pyridin-2-yl)urea;
1-methyl-1-(tetrahydro-2H-pyran-4-yl)-3-(4-(4-((1,2,3,4-tetrahydroisoquinolin-7-yl)oxy)-1H-pyrrolo[2,3-b]pyridin-3-yl)pyridin-2-yl)urea;
N-benzyl-4-(4-((5,6,7,8-tetrahydro-1,6-naphthyridin-3-yl)oxy)-1H-pyrrolo[2,3-b]pyridin-3-yl)pyridin-2-amine;
N-(6-(4-((1,2,3,4-tetrahydroisoquinolin-7-yl)oxy)-1H-pyrrolo[2,3-b]pyridin-3-yl)pyrimidin-4-yl)benzamide;
N-benzyl-5-(4-((1,2,3,4-tetrahydroisoquinolin-7-yl)oxy)-1H-pyrrolo[2,3-b]pyridin-3-yl)pyridin-3-amine;
(R)-N-(1-phenylethyl)-6-(4-((1,2,3,4-tetrahydroisoquinolin-7-yl)oxy)-1H-pyrrolo[2,3-b]pyridin-3-yl)pyrimidin-4-amine;
N-phenethyl-6-(4-((1,2,3,4-tetrahydroisoquinolin-7-yl)oxy)-1H-pyrrolo[2,3-b]pyridin-3-yl)pyrimidin-4-amine;
(S)-N-(1-phenylethyl)-6-(4-((1,2,3,4-tetrahydroisoquinolin-7-yl)oxy)-1H-pyrrolo[2,3-b]pyridin-3-yl)pyrimidin-4-amine;
N-(2-(pyridin-3-yl)ethyl)-6-(4-((1,2,3,4-tetrahydroisoquinolin-7-yl)oxy)-1H-pyrrolo[2,3-b]pyridin-3-yl)pyrimidin-4-amine;
N-benzyl-4-(4-((1,2,3,4-tetrahydroisoquinolin-7-yl)oxy)-7H-pyrrolo[2,3-d]pyrimidin-5-yl)pyridin-2-amine;
or pharmaceutically acceptable salts and solvates thereof.

9. A pharmaceutical composition comprising a compound as defined in any one of claims 1 to 8, or a pharmaceutically acceptable salt thereof, optionally in combination with at least one further active ingredient, in admixture with at least one pharmaceutically acceptable carrier or excipient.

10. A pharmaceutical composition according to claim 9 suitable to be administered by inhalation, such as inhalable powders, propellant-containing metering aerosols or propellant-free inhalable formulations.

11. A compound according to any one of claims 1 to 8 or a composition according to claim 9-10 for use as a medicament.

12. A compound or a composition for use according to claim 11 for use in the prevention and /or treatment of pulmonary disease selected from the group consisting of asthma, chronic obstructive pulmonary disease COPD, idiopathic pulmonary fibrosis (IPF), pulmonary hypertension (PH) and specifically Pulmonary Arterial Hypertension (PAH).

13. A combination of a compound as defined in any one of the claims 1 to 8 with one or more active ingredients selected from the classes consisting of organic nitrates and NO donors; inhaled NO; stimulator of soluble guanylate cyclase (sGC); prostaciclin analogue PGI2 and agonist of prostacyclin receptors; compounds that inhibit the degradation of cyclic guanosine monophosphate (cGMP) and/or cyclic adenosine monophosphate (cAMP); human neutrophilic elastase inhibitors; compounds inhibiting the signal transduction cascade; active substances for lowering blood pressure; neutral endopeptidase inhibitor; osmotic agents; ENaC blockers; anti-inflammatories including corticosteroids and antagonists of chemokine receptors; bronchodilators; antihistamine drugs; anti-tussive drugs; antibiotics and DNase drug substance and selective cleavage agents; agents that inhibit ALK5 and/or ALK4 phosphorylation of Smad2 and Smad3; tryptophan hydroylase 1 (TPH1) inhibitors and multi-kinase inhibitors.

14. A device comprising the pharmaceutical composition according to claim 9, which are selected from a single- or multi-dose dry powder inhaler, a metered dose inhaler or a soft mist nebulizer.

## Patentansprüche

1. Verbindungen der Formel (I) 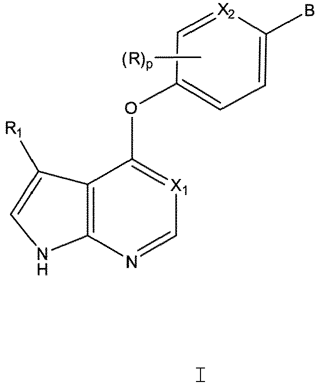 wobei
**X₁** und **X₂** bei jedem Auftreten unabhängig voneinander eine CH-Gruppe oder ein Stickstoffatom sind,
p Null oder eine ganze Zahl von 1 bis 3 ist;
jedes **R** unabhängig voneinander H oder Halogen ist;
**R₁** eine Gruppe der Formel **K** ist 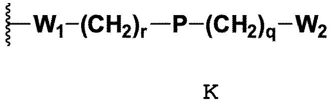
wobei **r** 0 oder eine ganze Zahl von 1 bis 4 ist; **q** 0 oder eine ganze Zahl von 1 bis 4 ist;
W₁ eine zweiwertige Arylen- oder Heteroarylengruppe ist, ausgewählt aus A1-A11 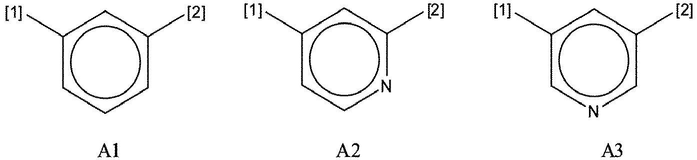 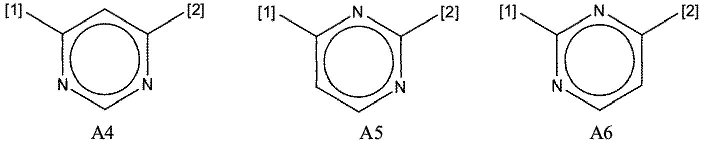 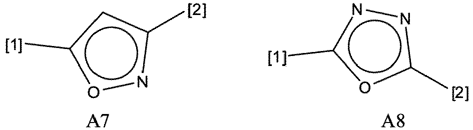 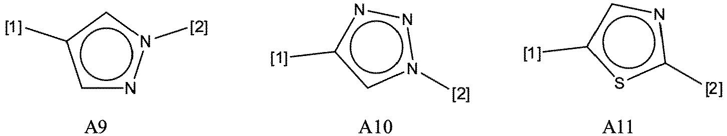
wobei [1] der Punkt der Bindung von W₁ an den Rest des Moleküls und [2] der Punkt der Bindung an -(CH₂)r- ist;
**P** abwesend ist oder eine zweiwertige Gruppe ist, ausgewählt aus O, S, SO, SO₂, CO, NR₆, N(R₆) (CH₂)ₙSO₂, N(R₆)COO, N(R₆)(CH₂)ₙC(O), N(R₆)(CH₂)ₙO, SO₂N(R₆), OC(O)N(R₆) und C(O)N(R₆), N(R₆)C(O)N(R₆);
**W₂** H ist oder ausgewählt ist aus (C₁-C₆)Alkyl, (C₃-C₈) Cycloalkyl, (C₃-C₈)Heterocycloalkyl, Aryl, Aryl (C₁-C₆) alkyl und Heteroaryl; gegebenenfalls substituiert durch einen oder mehrere Substituenten, die unabhängig voneinander ausgewählt sind aus Halogenatomen, -OH, Oxo (=O), -SH, -NO₂, -CN, - CON(R₆)₂, -C(O)R₆, -NR₆C(O)CH₃, -NH₂, -NHCOR₆, -CO₂R₆, - SO₂N(R₆)₂, -NR₆ SO₂CH₃, (C₁-C₆)Alkyl, (C₁-C₆)Halogenalkyl, (C₁-C₁₀)Alkoxy, (C₃-C₈)Cycloalkyl und (C₃-C₆)Cycloalkylcarbonyl;
n bei jedem Auftreten unabhängig 0 oder eine ganze Zahl von 1 bis 3 ist;
**R₆** bei jedem Auftreten unabhängig H ist oder ausgewählt ist aus (C₁-C₆)Alkyl, (C₁-C₆)Halogenalkyl, (C₂-C₆)Alkinyl, (C₂-C₆)Alkenyl, (C₃-C₈)Cycloalkyl, Heteroaryl und Aryl, gegebenenfalls substituiert durch einen oder mehrere Substituenten, ausgewählt aus Halogenatomen, -OH, Oxo (=O), -SH, -NO₂, -CN, -CONH₂, -COOH, (C₁-C₁₀)Alkyl und (C₁-C₁₀) Alkoxy;
B eine Gruppe der Formel R₂R₃N-(C₁-C₆)Alkyl ist, gegebenenfalls substituiert durch eine oder mehrere Gruppen, ausgewählt aus Hydroxyl, (C₁-C₆)Alkyl und (C₁-C₆)Hydroxyalkyl;
wobei
R₂ und R₃, die gleich oder verschieden sind, H sind oder ausgewählt sind aus (C₁-C₆)Alkyl, (C₁-C₆)Hydroxyalkyl,
oder
B eine Gruppe der Formel R₄R₃N-(C₁-C₆)Alkyl ist, wobei
R₃ H ist oder ausgewählt ist aus (C₁-C₆)Alkyl, (C₁-C₆)Hydroxyalkyl und R₄ eine zweiwertige Alkylgruppe -(CH₂)ₛ-ist, wobei s eine ganze Zahl von 1 bis 3 ist, wobei die zweiwertige Alkylgruppe mit dem Kohlenstoffatom in ortho-Position am benachbarten Ring verbunden ist, um einen heterozyklischen Ring, vorzugsweise einen 4- bis 10-gliedrigen heterozyklischen Ring, der mit dem benachbarten Ring kondensiert ist, zu bilden; wobei der heterozyklische Ring seinerseits weiter gegebenenfalls mit einer oder mehreren Gruppen, ausgewählt aus (C₁-C₆)Alkyl, (C₁-C₆)Hydroxyalkyl, substituiert ist;
und pharmazeutisch akzeptable Salze und Solvate davon.

2. Verbindung nach Anspruch 1, wobei jedes von X₁ und X₂ ein CH ist, das durch die Formel IA dargestellt wird: 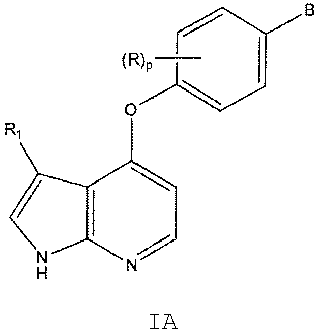 wobei B, R, R₁ und p wie in Anspruch 1 definiert sind.

3. Verbindungen nach Anspruch 1, wobei
B ein heterocyclischer Ring ist, der mit dem benachbarten Ring kondensiert ist und eine bicyclische Gruppe, ausgewählt aus I1-I6, bildet 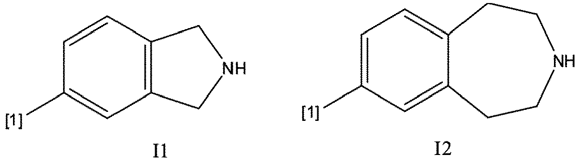 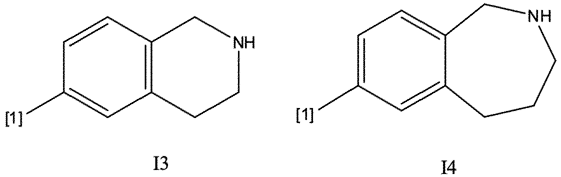 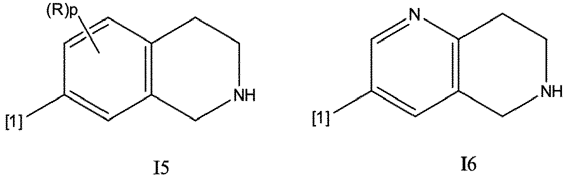
wobei der heterocyclische Ring seinerseits gegebenenfalls mit einer oder mehreren Gruppen, ausgewählt aus (C₁-C₆)Alkyl, (C₁-C₆)Hydroxyalkyl, substituiert ist;
und pharmazeutisch akzeptable Salze und Solvate davon.

4. Verbindungen nach Anspruch 3, wobei
B ein heterocyclischer Ring ist, der mit dem benachbarten Ring unter Bildung einer bicyclischen Gruppe (I5) kondensiert ist, dargestellt durch die Formel IB 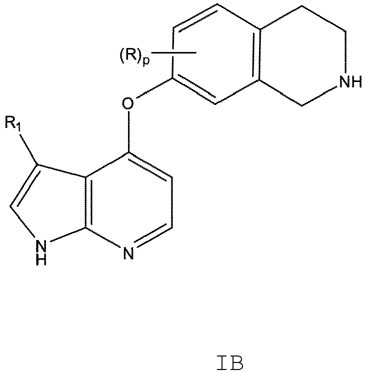 wobei R₁, R und p wie in Anspruch 3 definiert sind, und pharmazeutisch akzeptable Salze und Solvate davon.

5. Verbindung nach Anspruch 4, wobei
**R₁** eine Gruppe der Formel **K** ist 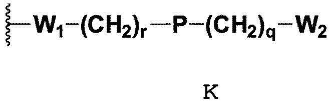
**r** 0 oder eine ganze Zahl von 1 bis 4 ist; **q** 0 oder eine ganze Zahl von 1 bis 4 ist;
W₁ eine zweiwertige Arylen- oder Heteroarylengruppe ist, ausgewählt aus A2, A4 oder A9;
**P** abwesend ist oder eine zweiwertige Gruppe ist, ausgewählt aus O, S, SO, SO₂, CO, NR₆, N(R₆) (CH₂)ₙSO₂, N(R₆)COO, N(R₆)(CH₂)ₙC(O), N(R₆)(CH₂)ₙO, SO₂N(R₆), OC(O)N(R₆), C(O)N(R₆), und N(R₆)C(O)N(R₆);
**W₂** H ist oder ausgewählt ist aus (C₁-C₆) -Alkyl, (C₃-C₈)-Cycloalkyl, (C₃-C₈)-Heterocycloalkyl, Aryl, Aryl (C₁-C₆) -Alkyl und Heteroaryl, gegebenenfalls substituiert durch einen oder mehrere Substituenten, unabhängig ausgewählt aus Halogenatomen, -OH, Oxo (=O), -SH, -NO₂, -CN, -CON(R₆)₂, - C(O)R₆, -NR₆C(O)CH₃, -NH₂, -NHCOR₆, -CO₂R₆, -SO₂N(R₆)₂, -NR₆ SO₂CH₃, (C₁-C₆) -Alkyl, (C₁-C₆)-Halogenalkyl (C₁-C₁₀)-Alkoxy, (C₃-C₈)-Cycloalkyl und (C₃-C₆)-Cycloalkyl-carbonyl, (C₃-C₆)-Cycloalkyl-aminocarbonyl;
n bei jedem Auftreten unabhängig 0 oder eine ganze Zahl von 1 bis 3 ist;
**R₆** bei jedem Auftreten unabhängig H oder (C₁-C₆)Alkyl ist;
und pharmazeutisch akzeptable Salze und Solvate davon.

6. Verbindung nach Anspruch 1, dargestellt durch die Formel IC, 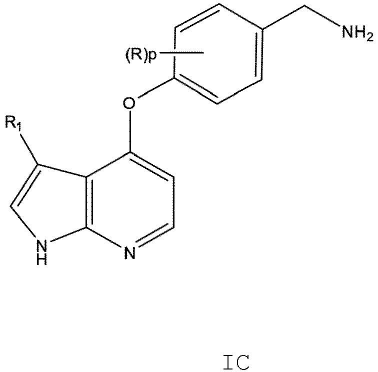 wobei R, R₁ und p wie in Anspruch 1 definiert sind,
und pharmazeutisch akzeptable Salze und Solvate davon.

7. Verbindung nach Anspruch 6, wobei
jedes **R** Halogen ist;
**R₁** eine Gruppe der Formel **K** ist 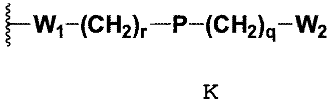
wobei **r** 0 ist; **q** 0 oder 1 ist;
W₁ eine zweiwertige Arylen- oder Heteroarylengruppe ist, ausgewählt aus A2, A4 oder A9
**P** abwesend ist oder ausgewählt ist aus den zweiwertigen Gruppen, bestehend aus NR₆, N(R₆)(CH₂)ₙSO₂, und
N(R₆)(CH₂)ₙC(O);
**W₂** ausgewählt ist aus (C₃-C₈)Cycloalkyl, (C₃-C₈)Heterocycloalkyl, Aryl und Heteroaryl, gegebenenfalls substituiert durch ein oder mehrere (C₁-C₆)Alkyl;
n bei jedem Auftreten unabhängig 0 oder eine ganze Zahl von 1 bis 3 ist;
**R₆** bei jedem Auftreten unabhängig H oder (C₁-C₆)Alkyl ist,
und pharmazeutisch akzeptable Salze und Solvate davon.

8. Verbindung nach Anspruch 1, ausgewählt aus:
7-((3-(Pyridin-4-yl)-1H-pyrrolo[2,3-b]pyridin-4-yl)oxy)-1,2,3,4-tetrahydroisochinolin;
(4-((3-(5-(benzyloxy)pyridin-3-yl)-1H-pyrrolo[2,3-b]pyridin-4-yl)oxy)phenyl)methanamin;
(4-((3-(3-(benzyloxy)phenyl)-1H-pyrrolo[2,3-b]pyridin-4-yl)oxy)phenyl)methanamin;
6-(4-(4-(Aminomethyl)-2,6-difluorphenoxy)-1H-pyrrolo[2,3-b]pyridin-3-yl)-N-(2-tosylethyl)pyrimidin-4-amin;
4-(4-(4-(Aminomethyl)-2,6-difluorphenoxy)-7H-pyrrolo[2,3-d]pyrimidin-5-yl)-N-benzylpyridin-2-amin;
N-Benzyl-4-(4-((6-Fluor-1,2,3,4-tetrahydroisochinolin-7-yl)oxy)-7H-pyrrolo[2,3-d]pyrimidin-5-yl)pyridin-2-amin;
4-(4-(4-(Aminomethyl)-2,6-difluorphenoxy)-1H-pyrrolo[2,3-b]pyridin-3-yl)-N-(pyridin-3-ylmethyl)pyridin-2-amin;
4-(4-(4-(Aminomethyl)-2,6-difluorphenoxy)-1H-pyrrolo[2,3-b]pyridin-3-yl)-N-(cyclopropylmethyl)pyridin-2-amin;
N-Benzyl-5-(4-((1,2,3,4-Tetrahydroisochinolin-7-yl)oxy)-1H-pyrrolo[2,3-b]pyridin-3-yl)isoxazol-3-amin;
N-Cyclopropyl-1-(4-(4-((1,2,3,4-tetrahydroisochinolin-7-yl)oxy)-1H-pyrrolo[2,3-b]pyridin-3-yl)pyridin-2-yl)pyrrolidin-3-carboxamid;
N-(4-(4-(4-(Aminomethyl)-2,6-difluorophenoxy)-1H-pyrrolo[2,3-b]pyridin-3-yl)pyridin-2-yl)cyclopropancarboxamid;
N-benzyl-2-(4-(4-((1,2,3,4-tetrahydroisochinolin-7-yl)oxy)-1H-pyrrolo[2,3-b]pyridin-3-yl)-1H-1,2,3-triazol-1-yl)acetamid;
N-Benzyl-5-(4-((1,2,3,4-Tetrahydroisochinolin-7-yl)oxy)-1H-pyrrolo[2,3-b]pyridin-3-yl)isoxazol-3-carboxamid;
2-Phenyl-N-(5-(4-((1,2,3,4-tetrahydroisochinolin-7-yl)oxy)-1H-pyrrolo[2,3-b]pyridin-3-yl)-1,3,4-oxadiazol-2-yl)acetamid;
4-(4-(4-(Aminomethyl)phenoxy)-1H-pyrrolo[2,3-b]pyridin-3-yl)-N-benzylpyridin-2-amin;
N-(Pyridin-3-ylmethyl)-4-(4-((1,2,3,4-tetrahydroisochinolin-7-yl)oxy)-1H-pyrrolo[2,3-b]pyridin-3-yl)pyridin-2-amin;
4-(4-(4-((1,2,3,4-Tetrahydroisochinolin-7-yl)oxy)-1H-pyrrolo[2,3-b]pyridin-3-yl)pyridin-2-yl)morpholin;
(S)-N-((Tetrahydrofuran-2-yl)methyl)-4-(4-((1,2,3,4-tetrahydroisochinolin-7-yl)oxy)-1H-pyrrolo[2,3-b]pyridin-3-yl)pyridin-2-amin;
(R)-N-((Tetrahydrofuran-2-yl)methyl)-4-(4-((1,2,3,4-tetrahydroisochinolin-7-yl)oxy)-1H-pyrrolo[2,3-b]pyridin-3-yl)pyridin-2-amin;
N-(2-Methoxyethyl)-4-(4-((1,2,3,4-tetrahydroisochinolin-7-yl)oxy)-1H-pyrrolo[2,3-b]pyridin-3-yl)pyridin-2-amin;
7-((3-(1-Met-hyl-1H-pyrazol-4-yl)-1H-pyrrolo[2,3-b]pyridin-4-yl)oxy)-1,2,3,4-tetrahydroisochinolin;
(4-((3-(1-Benzyl-1H-pyrazo1-4-yl)-1H-pyrrolo[2,3-b]pyridin-4-yl)oxy)-3-fluorphenyl)methanamin;
(3-Fluor-4-((3-(1-(Pyridin-4-ylmethyl)-1H-pyrazol-4-yl)-1H-pyrrolo[2,3-b]pyridin-4-yl)oxy)phenyl)methanamin;
(3-Fluor-4-((3-(1-((tetrahydro-2H-pyran-4-yl)methyl)-1H-pyrazol-4-yl)-1H-pyrrolo[2,3-b]pyridin-4-yl)oxy)phenyl)methanamin;
N-Benzyl-6-(4-((1,2,3,4-tetrahydroisochinolin-7-yl)oxy)-1H-pyrrolo[2,3-b]pyridin-3-yl)pyrimidin-4-amin;
N-Benzyl-4-(4-((1,2,3,4-tetrahydroisochinolin-7-yl)oxy)-1H-pyrrolo[2,3-b]pyridin-3-yl)pyrimidin-2-amin;
N-Benzyl-6-(4-((1,2,3,4-tetrahydroisochinolin-7-yl)oxy)-1H-pyrrolo[2,3-b]pyridin-3-yl)pyridin-2-amin;
N-Benzyl-2-(4-((1,2,3,4-tetrahydroisochinolin-7-yl)oxy)-1H-pyrrolo[2,3-b]pyridin-3-yl)pyrimidin-4-amin;
N-(2-(Phenylsulfonyl)ethyl)-6-(4-((1,2,3,4-tetrahydroisochinolin-7-yl)oxy)-1H-pyrrolo[2,3-b]pyridin-3-yl)pyrimidin-4-amin;
6-(4-((1,2,3,4-Tet-rahydroisochinolin-7-yl)oxy)-1H-pyrrolo[2,3-b]pyridin-3-yl)-N-(thiazol-2-ylmethyl)pyrimidin-4-amin;
6-(4-(4-(Aminomethyl)-2-fluorphenoxy)-1H-pyrrolo[2,3-b]pyridin-3-yl)-N-benzylpyrimidin-4-amin;
4-(4-(4-(Aminomethyl)-2-fluorphenoxy)-1H-pyrrolo[2,3-b]pyridin-3-yl)-N-benzylpyrimidin-2-amin;
4-(4-(4-(Aminomethyl)-2-fluorphenoxy)-1H-pyrrolo[2,3-b]pyridin-3-yl)-N-benzylpyridin-2-amin;
N-(5-(4-(4-(Aminomethyl)phenoxy)-1H-pyrrolo[2,3-b]pyridin-3-yl)thiazol-2-yl)-2-phenylacetamid;
4-(4-((1,2,3,4-Tetrahydroisochinolin-7-yl)oxy)-1H-pyrrolo[2,3-b]pyridin-3-yl)-N-(thiazol-2-ylmethyl)pyridin-2-amin;
N-(Cyclopropylmethyl)-4-(4-((1,2,3,4-tetrahydroisochinolin-7-yl)oxy)-1H-pyrrolo[2,3-b]pyridin-3-yl)pyridin-2-amin;
N-((5-Methylpyridin-2-yl)methyl)-4-(4-((1,2,3,4-tetrahydroisochinolin-7-yl)oxy)-1H-pyrrolo[2,3-b]pyridin-3-yl)pyridin-2-amin;
N-((Tetrahydro-2H-pyran-4-yl)methyl)-4-(4-((1,2,3,4-tetrahydroisochinolin-7-yl)oxy)-1H-pyrrolo[2,3-b]pyridin-3-yl)pyridin-2-amin;
N-(4-(4-((1,2,3,4-Tetrahydroisochinolin-7-yl)oxy)-1H-pyrrolo[2,3-b]pyridin-3-yl)pyridin-2-yl)cyclopropancarboxamid;
N-(4-(4-((1,2,3,4-Tetrahydroisochinolin-7-yl)oxy)-1H-pyrrolo[2,3-b]pyridin-3-yl)pyridin-2-yl)cyclohexancarboxamid;
N-(4-(4-((1,2,3,4-Tetrahydroisochinolin-7-yl)oxy)-1H-pyrrolo[2,3-b]pyridin-3-yl)pyridin-2-yl)tetrahydro-2H-pyran-4-carboxamid;
2-Isopropoxy-N-(4-(4-((1,2,3,4-tetrahydroisochinolin-7-yl)oxy)-1H-pyrrolo[2,3-b]pyridin-3-yl)pyridin-2-yl)acetamid;
2-(Piperidin-1-yl)-N-(4-(4-((1,2,3,4-tetrahydroisochinolin-7-yl)oxy)-1H-pyrrolo[2,3-b]pyridin-3-yl)pyridin-2-yl)acetamid;
1-Methyl-N-(4-(4-((1,2,3,4-tetrahydroisochinolin-7-yl)oxy)-1H-pyrrolo[2,3-b]pyridin-3-yl)pyridin-2-yl)cyclopropancarboxamid;
2-Amino-2-phenyl-N-(4-(4-((1,2,3,4-tetrahydroisochinolin-7-yl)oxy)-1H-pyrrolo[2,3-b]pyridin-3-yl)pyridin-2-yl)acetamid;
4,4-Dimethyl-N-(4-(4-((1,2,3,4-tetrahydroisochinolin-7-yl)oxy)-1H-pyrrolo[2,3-b]pyridin-3-yl)pyridin-2-yl)piperidin-1-carboxamid;
1-Cyclohexyl-1-methyl-3-(4-(4-((1,2,3,4-tetrahydroisochinolin-7-yl)oxy)-1H-pyrrolo[2,3-b]pyridin-3-yl)pyridin-2-yl)harnstoff;
1-Methyl-1-(tetrahydro-2H-pyran-4-yl)-3-(4-(4-((1,2,3,4-tetrahydroisochinolin-7-yl)oxy)-1H-pyrrolo[2,3-blpyridin-3-yl)pyridin-2-yl)harnstoff;
N-Benzyl-4-(4-((5,6,7,8-tetrahydro-1,6-naphthyridin-3-yl)oxy)-1H-pyrrolo[2,3-b]pyridin-3-yl)pyridin-2-amin;
N-(6-(4-((1,2,3,4-Tetrahydroisochinolin-7-yl)oxy)-1H-pyrrolo[2,3-b]pyridin-3-yl)pyrimidin-4-yl)benzamid;
N-Benzyl-5-(4-((1,2,3,4-tetrahydroisochinolin-7-yl)oxy)-1H-pyrrolo[2,3-b]pyridin-3-yl)pyridin-3-amin;
(R)-N-(1-Phenylethyl)-6-(4-((1,2,3,4-tetrahydroisochinolin-7-yl)oxy)-1H-pyrrolo[2,3-b]pyridin-3-yl)pyrimidin-4-amin;
N-Phenethyl-6-(4-((1,2,3,4-tetrahydroisochinolin-7-yl)oxy)-1H-pyrrolo[2,3-b]pyridin-3-yl)pyrimidin-4-amin;
(S)-N-(1-Phenylethyl)-6-(4-((1,2,3,4-tetrahydroisochinolin-7-yl)oxy)-1H-pyrrolo[2,3-b]pyridin-3-yl)pyrimidin-4-amin;
N-(2-(Pyridin-3-yl)ethyl)-6-(4-((1,2,3,4-tetrahydroisochinolin-7-yl)oxy)-1H-pyrrolo[2,3-b]pyridin-3-yl)pyrimidin-4-amin;
N-Benzyl-4-(4-((1,2,3,4-tetrahydroisochinolin-7-yl)oxy)-7H-pyrrolo[2,3-d]pyrimidin-5-yl)pyridin-2-amin;
oder pharmazeutisch akzeptable Salze und Solvate davon.

9. Pharmazeutische Zusammensetzung, umfassend eine Verbindung, wie sie in einem der Ansprüche 1 bis 8 definiert ist, oder ein pharmazeutisch akzeptables Salz davon, gegebenenfalls in Kombination mit mindestens einem weiteren Wirkstoff, im Gemisch mit mindestens einem pharmazeutisch akzeptablen Träger oder Hilfsstoff.

10. Pharmazeutische Zusammensetzung nach Anspruch 9, die zur Verabreichung durch Inhalation geeignet ist, wie inhalierbare Pulver, treibmittelhaltige Dosieraerosole oder treibmittelfreie inhalierbare Formulierungen.

11. Verbindung nach einem der Ansprüche 1 bis 8 oder Zusammensetzung nach Ansprüchen 9-10 zur Verwendung als Arzneimittel.

12. Verbindung oder Zusammensetzung zur Verwendung nach Anspruch 11 zur Verwendung bei der Vorbeugung und/oder Behandlung von Lungenerkrankungen, ausgewählt aus der Gruppe bestehend aus Asthma, chronisch obstruktiver Lungenerkrankung COPD, idiopathischer Lungenfibrose (IPF), Lungenhochdruck (PH) und insbesondere pulmonaler arterieller Hypertonie (PAH).

13. Kombination einer Verbindung, wie sie in einem der Ansprüche 1 bis 8 definiert ist, mit einem oder mehreren Wirkstoffen, ausgewählt aus den Klassen bestehend aus organischen Nitraten und NO-Donatoren; inhaliertem NO; Stimulator der löslichen Guanylatzyklase (sGC); Prostaciclin-Analog PGI2 und Agonist von Prostacyclin-Rezeptoren; Verbindungen, die den Abbau von zyklischem Guanosinmonophosphat (cGMP) und/oder zyklischem Adenosinmonophosphat (cAMP) hemmen; Inhibitoren der menschlichen neutrophilen Elastase; Verbindungen, die die Signaltransduktionskaskade hemmen; Wirkstoffen zur Senkung des Blutdrucks; neutralen Endopeptidase-Inhibitoren; osmotischen Wirkstoffen; ENaC-Blockern; Entzündungshemmern einschließlich Kortikosteroiden und Antagonisten von Chemokinrezeptoren; Bronchodilatatoren; Antihistaminika; Antitussiva; Antibiotika und DNase-Wirkstoffen und selektiven Spaltmitteln; Wirkstoffen, die die ALK5- und/oder ALK4-Phosphorylierung von Smad2 und Smad3 hemmen; Tryptophanhydroylase 1 (TPH1)-Inhibitoren und Multi-Kinase-Inhibitoren.

14. Vorrichtung, die die pharmazeutische Zusammensetzung nach Anspruch 9 enthält, ausgewählt aus einem Ein- oder Mehrfachdosis-Trockenpulverinhalator, einem Dosierinhalator oder einem Weichnebelvernebler.

## Revendications

1. Composés répondant à la formule (I) 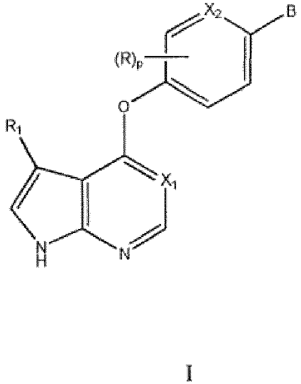 dans laquelle :
**X₁** et **X₂** représentent, à chaque occurrence, de manière indépendante, un groupe CH ou un atome d'azote ;
**p** est égal à zéro ou représente un entier de 1 à 3 ;
chaque radical **R** représente, de manière indépendante, un atome d'hydrogène ou un atome d'halogène ;
**R₁** représente un groupe qui répond à la formule **K** 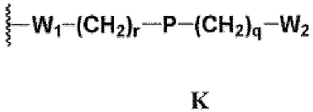
dans laquelle **r** est égal à 0 ou représente un entier de 1 à 4 ; **q** est égal à 0 ou représente un entier de 1 à 4 ;
**W₁** représente un groupe arylène ou hétéroarylène divalent qui est choisi parmi les formules A1-A11 : 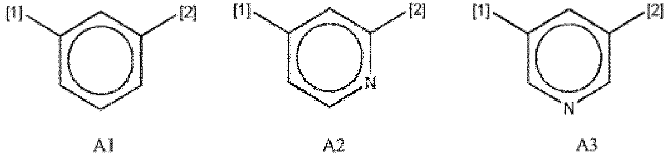 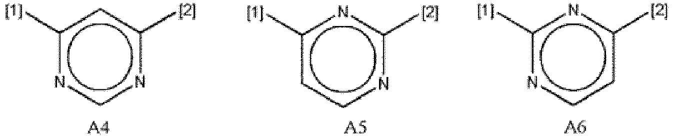 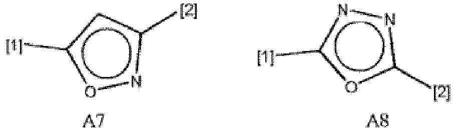 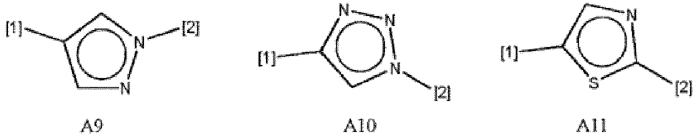
dans lesquelles [1] représente le point de fixation de **W₁** au reste de la molécule et [2] représente le point de fixation au groupe -(CH₂)ᵣ- ;
**P** est absent ou représente un groupe divalent qui est choisi parmi un atome d'oxygène, un atome de soufre, un groupe SO, un groupe SO₂, un groupe CO, un groupe NR₆, un groupe N(R₆)(CH₂)ₙSO₂, un groupe N(R₆)COO, un groupe N(R₆)(CH₂)ₙC(O), un groupe N(R₆)(CH₂)ₙ(O), un groupe SO₂N(R₆), un groupe OC(O)N(R₆) et un groupe C(O)N(R₆), un groupe N(R₆)C(O)N(R₆) ;
**W₂** représente un atome d'hydrogène ou est choisi parmi un groupe alkyle en C₁-C₆, un groupe cycloalkyle en C₃-C₈, un groupe hétérocycloalkyle en C₃-C₈, un groupe aryle, un groupe aryl(alkyle en C₁-C₆) et un groupe hétéroaryle, substitués de manière facultative par un ou plusieurs substituants qui sont choisis de manière indépendante parmi des atomes d'halogène, un groupe -OH, un groupe oxo (=O), un groupe -SH, un groupe -NO₂, un groupe -CN, un groupe -CON(R₆)₂, un groupe -C(O)R₆, un groupe NR₆C(O)CH₃, un groupe -NH₂, un groupe -NHCOR₆, un groupe -CO₂R₆, un groupe -SO₂N(R₆)₂, un groupe -NR₆ SO₂CH₃, un groupe alkyle en C₁-C₆, un groupe halogénoalkyle en C₁-C₆, un groupe alcoxy en C₁-C₁₀, un groupe cycloalkyle en C₃-C₈ et un groupe cycloalkyl(en C₃-C₆)carbonyle ;
n, à chaque occurrence, de manière indépendante, est égal à 0 ou représente un entier de 1 à 3 ;
**R₆**, à chaque occurrence, représente, de manière indépendante, un atome d'hydrogène ou est choisi parmi un groupe alkyle en C₁-C₆, un groupe halogénoalkyle en C₁-C₆, un groupe alcynyle en C₂-C₆, un groupe alcényle en C₂-C₆, un groupe cycloalkyle en C₃-C₈, un groupe hétéroaryle et un groupe aryle, substitués de manière facultative par un ou plusieurs substituants qui sont choisis parmi des atomes d'halogène, un groupe -OH, un groupe oxo (=O), un groupe -SH, un groupe -NO₂, un groupe -CN, un groupe -CONH₂, un groupe -COOH, un groupe alkyle en C₁-C₁₀ et un groupe alcoxy en C₁-C₁₀ ;
**B** représente un groupe qui répond à la formule R₂R₃N-alkyle en C₁-C₆, substitué de manière facultative par un ou plusieurs groupes qui sont choisis parmi un groupe hydroxyle, un groupe alkyle en C₁-C₆ et un groupe hydroxyalkyle en C₁-C₆ ;
dans laquelle
**R₂** et **R₃**, qui sont identiques ou différents, représentent un atome d'hydrogène ou sont choisis parmi un groupe alkyle en C₁-C₆, un groupe hydroxyalkyle en C₁-C₆ ;
ou
**B** représente un groupe qui répond à la formule R₄R₃N-alkyle en C₁-C₆, dans laquelle :
**R₃** représente un atome d'hydrogène ou est choisi parmi un groupe alkyle en C₁-C₆, un groupe hydroxyalkyle en C₁-C₆ et **R₄** représente un groupe alkyle divalent -(CH₂)ₛ-, dans lequel s représente un entier de 1 à 3, le groupe alkyle divalent en question étant relié à l'atome de carbone en position ortho sur le noyau adjacent afin de former un noyau hétérocyclique, de préférence un noyau hétérocyclique de 4 à 10 membres, condensé au noyau adjacent ; ledit noyau hétérocyclique étant, à son tour, substitué en outre de manière facultative avec un ou plusieurs groupes qui sont choisis parmi un groupe alkyle en C₁-C₆, un groupe hydroxyalkyle en C₁-C₆ ;
ainsi que leurs sels et leurs solvates pharmaceutiquement acceptables.

2. Composé selon la revendication 1, dans lequel chacun des radicaux **X₁** et **X₂** représente un groupe CH, qui répond à la formule IA : 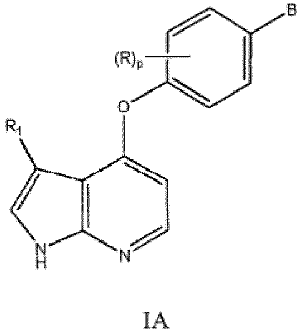 dans laquelle **B**, **R**, **R₁** et p sont tels qu'ils ont été définis à la revendication 1.

3. Composés selon la revendication 1, dans lesquels :
**B** représente un noyau hétérocyclique condensé avec le noyau adjacent pour former un groupe bicyclique qui est choisi parmi I1-I6 : 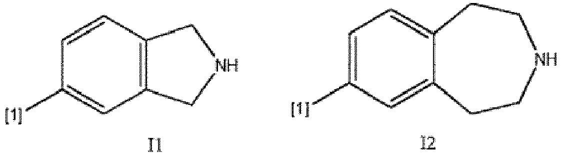 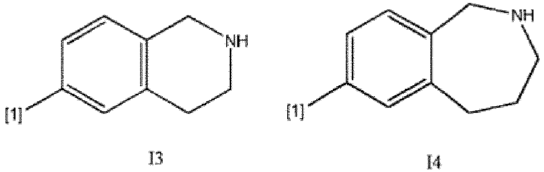 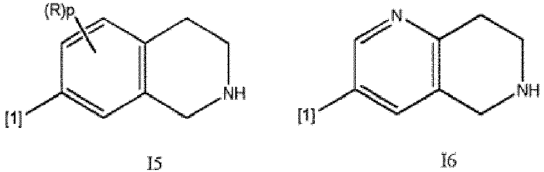
noyau hétérocyclique en question étant, à son tour, substitué en outre de manière facultative avec un ou plusieurs groupes qui sont choisis parmi un groupe alkyle en C₁-C₆, un groupe hydroxyalkyle en C₁-C₆ ; ainsi que leurs sels et leurs solvates pharmaceutiquement acceptables.

4. Composés selon la revendication 3, dans lesquels :
**B** représente un noyau hétérocyclique condensé avec le noyau adjacent pour former un groupe bicyclique (I5) qui répond à la formule IB : 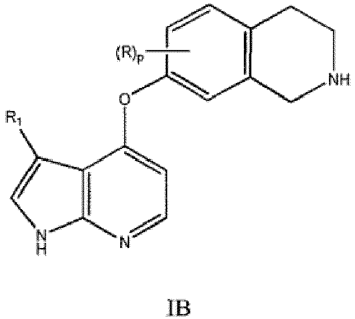
dans laquelle **R₁**, **R** et **p** sont tels qu'ils ont été définis à la revendication 3;
ainsi que leurs sels et leurs solvates pharmaceutiquement acceptables.

5. Composé selon la revendication 4, dans lequel :
**R₁** représente un groupe qui répond à la formule **K** 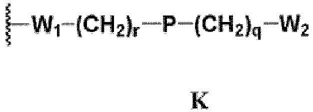
dans laquelle **r** est égal à 0 ou représente un entier de 1 à 4 ; **q** est égal à 0 ou représente un entier de 1 à 4 ;
**W₁** représente un groupe arylène ou hétéroarylène divalent qui est choisi parmi les formules A2, A4 ou A9 ;
**P** est absent ou représente un groupe divalent qui est choisi parmi un atome d'oxygène, un atome de soufre, un groupe SO, un groupe SO₂, un groupe CO, un groupe NR₆, un groupe N(R₆)(CH₂)ₙSO₂, un groupe N(R₆)COO, un groupe N(R₆)(CH₂)ₙC(O), un groupe N(R₆)(CH₂)ₙO, un groupe SO₂N(R₆), un groupe OC(O)N(R₆), un groupe C(O)N(R₆) et un groupe N(R₆)C(O)N(R₆) ;
**W₂** représente un atome d'hydrogène ou est choisi parmi un groupe alkyle en C₁-C₆, un groupe cycloalkyle en C₃-C₈, un groupe hétérocycloalkyle en C₃-C₈, un groupe aryle, un groupe aryl(alkyle en C₁-C₆) et un groupe hétéroaryle, substitués de manière facultative par un ou plusieurs substituants qui sont choisis de manière indépendante parmi des atomes d'halogène, un groupe -OH, un groupe oxo (=O), un groupe -SH, un groupe -NO₂, un groupe -CN, un groupe -CON(R₆)₂, un groupe -C(O)R₆,
un groupe NR₆C(O)CH₃, un groupe -NH₂, un groupe -NHCOR₆, un groupe -CO₂R₆, un groupe -SO₂N(R₆)₂, un groupe -NR₆ SO₂CH₃, un groupe alkyle en C₁-C₆, un groupe halogénoalkyle en C₁-C₆, un groupe alcoxy en C₁-C₁₀, un groupe cycloalkyle en C₃-C₈, et un groupe cycloalkyl(en C₃-C₆)carbonyle, un groupe cycloalkyl(en C₃-C₆)aminocarbonyle ;
n, à chaque occurrence, de manière indépendante, est égal à 0 ou représente un entier de 1 à 3 ;
**R₆**, à chaque occurrence, représente, de manière indépendante, un atome d'hydrogène ou un groupe alkyle en C₁-C₆ ;
ainsi que ses sels et ses solvates pharmaceutiquement acceptables.

6. Composé selon la revendication 1, qui répond à la formule IC : 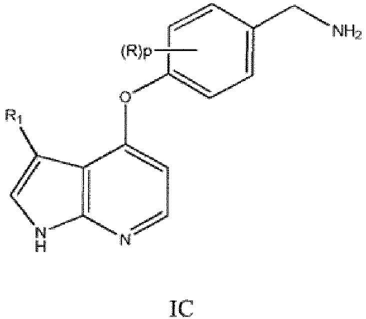
dans laquelle **R**, **R₁** et **p** sont tels qu'ils ont été définis à la revendication 1 ;
ainsi que ses sels et ses solvates pharmaceutiquement acceptables.

7. Composé selon la revendication 6, dans lequel :
chaque radical **R** représente un atome d'halogène ;
**R₁** représente un groupe qui répond à la formule **K** 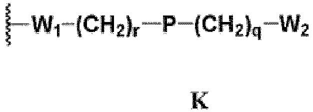
dans laquelle r est égal à 0 ; **q** est égal à 0 ou 1 ;
**W₁** représente un groupe arylène ou hétéroarylène divalent qui est choisi parmi les formules A2, A4 ou A9 ;
**P** est absent ou est choisi à partir des groupes divalents qui sont constitués par un groupe NR₆, un groupe N(R₆)(CH₂)ₙSO₂, et un groupe N(R₆)(CH₂)ₙC(O) ;
**W₂** est choisi parmi un groupe cycloalkyle en C₃-C₈, un groupe hétérocycloalkyle en C₃-C₈, un groupe aryle et un groupe hétéroaryle,
substitués de manière facultative par un ou plusieurs groupes alkyle en C₁-C₆ ;
n, à chaque occurrence, de manière indépendante, est égal à 0 ou représente un entier de 1 à 3 ;
**R₆** représente, à chaque occurrence, de manière indépendante, un atome d'hydrogène ou un groupe alkyle en C₁-C₆ ;
ainsi que ses sels et ses solvates pharmaceutiquement acceptables.

8. Composé selon la revendication 1, qui est choisi parmi :
la 7-((3-(pyridin-4-yl)-1H-pyrrolo[2,3-b]pyridin-4-yl)oxy)-1,2,3,4-tétrahydro-isoquinoléine ;
la (4-((3-(5-(benzyloxy)pyridin-3-yl)-1H-pyrrolo[2,3-b]pyridin-4-yl)oxy)phényl)-méthanamine ;
la (4-((3-(3-(benzyloxy)phényl)-1H-pyrrolo[2,3-b]pyridin-4-yl)oxy)phényl)-méthanamine ;
la 6-(4-(4-(aminométhyl)-2,6-difluorophénoxy)-1H-pyrrolo[2,3-b]pyridin-3-yl)-N-(2-tosyléthyl)pyrimidin-4-amine ;
la 4-(4-(4-(aminométhyl)-2,6-difluorophénoxy)-7H-pyrrolo[2,3-d]pyrimidin-5-yl)-N-benzylpyridin-2-amine ;
la N-benzyl-4-(4-((6-fluoro-1,2,3,4-tétrahydroisoquinolin-7-yl)oxy)-7H-pyrrolo[2,3-d]pyrimidin-5-yl)pyridin-2-amine ;
la 4-(4-(4-(aminométhyl)-2,6-difluorophénoxy)-1H-pyrrolo[2,3-b]pyridin-3-yl)-N-(pyridin-3-ylméthyl)pyridin-2-amine ;
la 4-(4-(4-(aminométhyl)-2,6-difluorophénoxy)-1H-pyrrolo[2,3-b]pyridin-3-yl)-N-(cyclopropylméthyl)pyridin-2-amine ;
la N-benzyl-5-(4-((1,2,3,4-tétrahydroisoquinolin-7-yl)oxy)-1H-pyrrolo[2,3-b]pyridin-3-yl)isoxazol-3-amine ;
le N-cyclopropyl-1-(4-(4-((1,2,3,4-tétrahydroisoquinolin-7-yl)oxy)-1H-pyrrolo[2,3-b]pyridin-3-yl)pyridin-2-yl)pyrrolidine-3-carboxamide ;
le N-(4-(4-(4-(aminométhyl)-2,6-difluorophénoxy)-1H-pyrrolo[2,3-b]pyridin-3-yl)pyridin-2-yl)cyclopropanecarboxamide ;
le N-benzyl-2-(4-(4-((1,2,3,4-tétrahydroisoquinolin-7-yl)oxy)-1H-pyrrolo[2,3-b]pyridin-3-yl)-1H-1,2,3-triazol-1-yl)acétamide ;
le N-benzyl-5-(4-((1,2,3,4-tétrahydroisoquinolin-7-yl)oxy)-1H-pyrrolo[2,3-bipyridin-3-yl)isoxazole-3-carboxamide ;
le 2-phényl-N-(5-(4-((1 ,2,3,4-tétrahydroisoquinolin-7-yl)oxy)-1H-pyrrolo[2,3-b]pyridin-3-yl)-1,3,4-oxadiazol-2-yl)acétamide ;
la 4-(4-(4-(aminométhyl)phénoxy)-1H-pyrrolo[2,3-b]pyridin-3-yl)-N-benzylpyridin-2-amine ;
la N-(pyridin-3-ylméthyl)-4-(4-((1,2,3,4-tétrahydroisoquinolin-7-yl)oxy)-1H-pyrrolo[2,3-b]pyridin-3-yl)pyridin-2-amine ;
la 4-(4-(4-((1,2,3,4-tétrahydroisoquinolin-7-yl)oxy)-1H-pyrrolo[2,3-b]pyridin-3-yl)pyridin-2-yl)morpholine ;
la (S)-N-((tétrahydrofuran-2-yl)méthyl)-4-(4-((1,2,3,4-tétrahydroisoquinolin-7-yl)oxy)-1H-pyrrolo[2,3-b]pyridin-3-yl)pyridin-2-amine ;
la (R)-N-((tétrahydrofuran-2-yl)méthyl)-4-(4-((1,2,3,4-tétrahydro-isoquinolin-7-yl)oxy)-1H-pyrrolo[2,3-b]pyridin-3-yl)pyridin-2-amine ;
la N-(2-méthoxyéthyl)-4-(4-(1,2,3,4-tétrahydroisoquinolin-7-yl)oxy)-1H-pyrrolo[2,3-b]pyridin-3-yl)pyridin-2-amine ;
la 7-((3-(1-méthyl-1H-pyrazol-4-yl)-1 H-pyrrolo[2,3-b]pyridin-4-yl)oxy)-1,2,3,4-tétrahydroisoquinoléine ;
la (4-((3-(1-benzyl-1H-pyrazol-4-yl)-1H-pyrrolo[2,3-b]pyridin-4-yl)oxy)-3-fluorophényl)méthanamine ;
la (3-fluoro-4-((3-(1-(pyridin-4-ylméthyl)-1H-pyrazol-4-yl)-1 H-pyrrolo[2,3-b]pyridin-4-yl)oxy)phényl)méthanamine ;
la (3-fluoro-4-((3-(1-((tétrahydro-2H-pyran-4-yl)méthyl)-1H-pyrazol-4-yl)-1H-pyrrolo[2,3-b]pyridin-4-yl)oxy)phényl)méthanamine ;
la N-benzyl-6-(4-((1,2,3,4-tétrahydroisoquinolin-7-yl)oxy)-1H-pyrrolo[2,3-b]pyridin-3-yl)pyrimidin-4-amine ;
la N-benzyl-4-(4-((1,2,3,4-tétrahydroisoquinolin-7-yl)oxy)-1H-pyrrolo[2,3-b]pyridin-3-yl)pyrimidin-2-amine ;
la N-benzyl-6-(4-((1,2,3,4-tétrahydroisoquinolin-7-yl)oxy)-1H-pyrrolo[2,3-b]pyridin-3-yl)pyridin-2-amine ;
la N-benzyl-2-(4-((1,2,3,4-tétrahydroisoquinolin-7-yl)oxy)-1H-pyrrolo[2,3-b]pyridin-3-yl)pyrimidin-4-amine ;
la N-(2-(phénylsulfonyl)éthyl)-6-(4-((1,2,3,4-tétrahydroisoquinolin-7-yl)oxy)-1H-pyrrolo[2,3-b]pyridin-3-yl)pyrimidin-4-amine ;
la 6-(4-((1,2,3,4-tétrahydroisoquinolin-7-yl)oxy)-1H-pyrrolo[2,3-b]pyridin-3-yl)-N-(thiazol-2-ylméthyl)pyrimidin-4-amine ;
la 6-(4-(4-(aminométhyl)-2-fluorophénoxy)-1H-pyrrolo[2,3-b]pyridin-3-yl)-N-benzylpyrimidin-4-amine ;
la 4-(4-(4-(aminométhyl)-2-fluorophénoxy)-1H-pyrrolo[2,3-bipyridin-3-yl)-N-benzylpyrimidin-2-amine ;
la 4-(4-(4-(aminométhyl)-2-fluorophénoxy)-1H-pyrrolo[2,3-b]pyridin-3-yl)-N-benzylpyridin-2-amine ;
le N-(5-(4-(4-(aminométhyl)phénoxy)-1H-pyrrolo[2,3-b]pyridin-3-yl)thiazol-2-yl)-2-phénylacétamide ;
la 4-(4-((1,2,3,4-tétrahydroisoquinolin-7-yl)oxy)-1H-pyrrolo[2,3-b]pyridin-3-yl)-N-(thiazol-2-ylméthyl)pyridin-2-amine ;
la N-(cyclopropylméthyl)-4-(4-((1,2,3,4-tétrahydroisoquinolin-7-yl)oxy)-1H-pyrrolo[2,3-b]pyridin-3-yl)pyridin-2-amine ;
la N-((5-méthylpyridin-2-yl)méthyl)-4-(4-((1,2,3,4-tétrahydroisoquinolin-7-yl)oxy)-1H-pyrrolo[2,3-b]pyridin-3-yl)pyridin-2-amine ;
la N-((tétrahydro-2H-pyran-4-yl)méthyl)-4-(4-((1,2,3,4-tétrahydro-isoquinolin-7-yl)oxy)-1H-pyrrolo[2,3-b]pyridin-3-yl)pyridin-2-amine ;
le N-(4-(4-((1,2,3,4-tétrahydroisoquinolin-7-yl)oxy)-1H-pyrrolo[2,3-b]pyridin-3-yl)pyridin-2-yl)cyclopropanecarboxamide ;
le N-(4-(4-((1,2,3,4-tétrahydroisoquinolin-7-yl)oxy)-1H-pyrrolo[2,3-b]pyridin-3-yl)pyridin-2-yl)cyclohexanecarboxamide ;
le N-(4-(4-((1,2,3,4-tétrahydroisoquinolin-7-yl)oxy)-1H-pyrrolo[2,3-b]pyridin-3-yl)pyridin-2-yl)tétrahydro-2H-pyran-4-carboxamide ;
le 2-isopropoxy-N-(4-(4-((1 ,2,3,4-tétrahydroisoquinolin-7-yl)oxy)-1 H-pyrrolo[2,3b]pyridin-3-yl)pyridin-2-yl)acétamide ;
le 2-(pipéridin-1-yl)-N-(4-(4-((1 ,2,3,4-tétrahydroisoquinolin-7-yl)oxy)-1H-pyrrolo[2,3-b]pyridin-3-yl)pyridin-2-yl)acétamide ;
le 1-méthyl-N-(4-(4-((1,2,3,4-tétrahydroisoquinolin-7-yl)oxy)-1H-pyrrolo[2,3-b]pyridin-3-yl)pyridin-2-yl)cyclopropanecarboxamide ;
le 2-amino-2-phényl-N-(4-(4-((1 ,2,3,4-tétrahydroisoquinolin-7-yl)oxy)-1H-pyrrolo[2,3-b]pyridin-3-yl)pyridin-2-yl)acétamide ;
le 4,4-diméthyl-N-(4-(4-((1 ,2,3,4-tétrahydroisoquinolin-7-yl)oxy)-1H-pyrrolo[2,3-b]pyridin-3-yl)pyridin-2-yl)pipéridine-1-carboxamide ;
la 1-cyclohexyl-1-méthyl-3-(4-(4-((1,2,3,4-tétrahydroisoquinolin-7-yl)oxy)-1H-pyrrolo[2,3-b]pyridin-3-yl)pyridin-2-yl)urée ;
la 1-méthyl-1-(tétrahydro-2H-pyran-4-yl)-3-(4-(4-((1,2,3,4-tétrahydroisoquinolin-7-yl)oxy)-1H-pyrrolo[2,3-b]pyridin-3-yl)pyridin-2-yl)urée ;
la N-benzyl-4-(4-((5,6,7,8-tétrahydro-1,6-naphtyridin-3-yl)oxy)-1H-pyrrolo[2,3-b]-pyridin-3-yl)pyridin-2-amine ;
le N-(6-(4-((1,2,3,4-tétrahydroisoquinolin-7-yl)oxy)-1H-pyrrolo[2,3-b]pyridin-3-yl)pyrimidin-4-yl)benzamide ;
la N-benzyl-5-(4-((1,2,3,4-tétrahydroisoquinolin-7-yl)oxy)-1H-pyrrolo[2,3-b]pyridin-3-yl)pyridin-3-amine ;
la (R)-N-(1-phényléthyl)-6-(4-((1,2,3,4-tétrahydroisoquinolin-7-yl)oxy)-1H-pyrrolo[2,3-b]pyridin-3-yl)pyrimidin-4-amine ;
la N-phénéthyl-6-(4-((1,2,3,4-tétrahydroisoquinolin-7-yl)oxy)-1H-pyrrolo[2,3-b]pyridin-3-yl)pyrimidin-4-amine ;
la (S)-N-(1-phényléthyl)-6-(4-((1,2,3,4-tétrahydroisoquinolin-7-yl)oxy)-1H-pyrrolo[2,3-b]pyridin-3-yl)pyrimidin-4-amine ;
la N-(2-(pyridin-3-yl)éthyl)-6-(4-((1,2,3,4-tétrahydroisoquinolin-7-yl)oxy)-1H-pyrrolo[2,3-b]pyridin-3-yl)pyrimidin-4-amine ;
la N-benzyl-4-(4-((1,2,3,4-tétrahydroisoquinolin-7-yl)oxy)-7H-pyrrolo[2,3-d]pyrimidin-5-yl)pyridin-2-amine ;
ou leurs sels et leurs solvates pharmaceutiquement acceptables.

9. Composition pharmaceutique qui comprend un composé tel qu'il a été défini dans l'une quelconque des revendications 1 à 8, ou un de ses sels pharmaceutiquement acceptables, de manière facultative en combinaison avec au moins un ingrédient actif supplémentaire, en mélange avec au moins un support ou un excipient pharmaceutiquement acceptable.

10. Composition pharmaceutique selon la revendication 9, qui est appropriée pour être administrée par inhalation, telle que des poudres inhalables, des aérosols doseurs contenant un ou des agents propulseurs ou des formulations inhalables exemptes d'agents propulseurs.

11. Composé selon l'une quelconque des revendications 1 à 8 ou composition selon les revendications 9-10, pour son utilisation comme médicament.

12. Composé ou composition pour son utilisation selon la revendication 11, pour son utilisation dans la prévention et/ou le traitement d'une maladie pulmonaire qui est choisie parmi le groupe constitué par l'asthme, la maladie pulmonaire obstructive chronique (MPOC), la fibrose pulmonaire idiopathique (IPF), l'hypertension pulmonaire (PH) et, de manière spécifique, l'hypertension artérielle pulmonaire (PAH).

13. Combinaison d'un composé tel qu'il a été défini dans l'une quelconque des revendications 1 à 8 avec un ou plusieurs ingrédients actifs qui sont choisis parmi les classes constituées par des nitrates organiques et des donneurs de NO ; du NO inhalé ; un stimulateur de la guanylate cyclase soluble (sGC) ; un analogue de la prostacycline PGI2 et un agoniste des récepteurs de la prostacycline ; des composés qui inhibent la dégradation de la guanosine monophosphate cyclique (cGMP) et/ou de l'adénosine monophosphate cyclique (cAMP) ; des inhibiteurs de l'élastase des neutrophiles humaine ; des composés qui inhibent la cascade de transduction du signal ; des substances qui s'avèrent actives pour abaisser la pression artérielle ; un inhibiteur de l'endopeptidase neutre ; des agents osmotiques ; des inhibiteurs du canal sodique épithélial (EnaC) ; des anti-inflammatoires y compris des corticostéroïdes et des antagonistes de récepteurs des chimiokines ; des bronchodilatateurs ; des agents antihistaminiques ; des médicaments antitussifs ; des antibiotiques et des substances médicamenteuses à base de désoxyribonucléase et des agents de clivage sélectifs ; des agents qui inhibent la phosphorylation par ALK5 et/ou par ALK4 de Smad2 et de Smad3 ; des inhibiteurs de la tryptophane hydrolase 1 (TPH1) et des inhibiteurs de kinases multiples.

14. Dispositif qui comprend la composition pharmaceutique selon la revendication 9, qui est choisi parmi un inhalateur de poudre sèche du type à dose unique ou à doses multiples, un inhalateur doseur et un nébuliseur du type Soft-Mist.
